# EUROPEAN PATENT APPLICATION

(11) **EP 3 070 167 A1**
(43) Date of publication of application: **21.09.2016**
(21) Application number: 14859656.2
(22) Date of filing: 06.11.2014
(51) Int. Cl.: C12N 15/09, A61K 39/395, A61P 31/16, C07K 16/10, C12N 5/10, C12P 21/08

(54) **ANTIBODY HAVING BROAD NEUTRALIZING ACTIVITY IN GROUP 1 INFLUENZA A VIRUS**

(30) Priority: 06.11.2013 US 201361900659 P
(71) Applicant: Osaka University, Suita-shi, Osaka 565-0871 (JP); Medical & Biological Laboratories Co., Ltd., Nagoya-shi, Aichi 460-0008 (JP); Department of Medical Sciences, Nonthaburi 11000 (TH)
(72) Inventor: SASAKI, Tadahiro, Suita-shi Osaka 565-0871 (JP); ONO, Kenichiro, Nagoya-shi Aichi 460-0008 (JP); BOONSATHORN, Naphatsawan, Muang Nonthaburi 11000 (TH); WATANABE, Yohei, Suita-shi Osaka 565-0871 (JP); INOUE, Yuji, Suita-shi Osaka 565-0871 (JP); IKUTA, Kazuyoshi, Suita-shi Osaka 565-0871 (JP)
(74) Representative: Campbell, Patrick John Henry
(86) International application number: PCT/JP2014/079507
(87) International publication number: WO 2015/068781

(57) **Abstract**

From PBMCs of patients infected with H1N1pdm, three human monoclonal antibodies have been obtained which are capable of binding to an epitope present at residues 40 to 58 in an HA2 region of a hemagglutinin protein derived from H1N1pdm. Further, these antibodies have been found to also have a neutralization activity against subtype H1 and subtype H5 of group 1 influenza A viruses. On the other hand, the three antibodies have also been found to exhibit neither a binding ability nor a neutralization activity against subtype H2 which belongs to the group 1, but in the HA2 region derived from H1N1pdm of which the amino acid at residue 45 is substituted with phenylalanine and the amino acid at residue 47 is substituted with glycine.

SEQUENCE LISTING
<110>  OSAKA UNIVERSITY
       MEDICAL & BIOLOGICAL LABORATORIES CO., LTD.
       Department of Medical Sciences
<120>
\203C\203\223\203t\203\213\203G\203\223\203U\203E\203C\203\213\203X\202`\214^
\202Ì\203O\203\213\201[\203v\202P\202É\221Î\202µ\202Ä\215L\210æ\202È\222\206
\230a\212\210\220«\202ð\227L\202•\202é\215R\221Ì
<130>  IBPF13-549WO
<150>  US61/900659
<151>  2013-11-06
<160>  48
<170>  PatentIn version 3.5

<210>  1
<211>  331
<212>  DNA
<213>  Homo sapiens
<220>
<221>  CDS
<222>  (1)..(330)
<223>  Variable Region of Light Chain (1H11)
<400>  1
cag tct gcc ctg act cag cct gcc tcc gtg tct ggg tct cct gga cag          48
Gln Ser Ala Leu Thr Gln Pro Ala Ser Val Ser Gly Ser Pro Gly Gln
1               5                   10                  15
tcg atc acc atc tcc tgc act gga acc agc agt gac gtt ggt ggt tat          96
Ser Ile Thr Ile Ser Cys Thr Gly Thr Ser Ser Asp Val Gly Gly Tyr
            20                  25                  30
aac tat gtc tcc tgg tac caa caa cac ccc ggc gaa gcc ccc aaa ctc         144
Asn Tyr Val Ser Trp Tyr Gln Gln His Pro Gly Glu Ala Pro Lys Leu
        35                  40                  45
atg att tct gat gtc agt aat cgg ccc tca ggg gtt tct aat cgc ttc         192
Met Ile Ser Asp Val Ser Asn Arg Pro Ser Gly Val Ser Asn Arg Phe
    50                  55                  60
tct ggc tcc aag tct ggc aac acg gcc tcc ctg acc atc tct ggg ctc         240
Ser Gly Ser Lys Ser Gly Asn Thr Ala Ser Leu Thr Ile Ser Gly Leu
65                  70                  75                  80
cag gct gag gac gag gct gat tat tac tgc acc tca tat aca agc agc         288
Gln Ala Glu Asp Glu Ala Asp Tyr Tyr Cys Thr Ser Tyr Thr Ser Ser
                85                  90                  95
aac act ttg gtg ttc ggc gga ggg acc aag ttg acc gtc cta g               331
Asn Thr Leu Val Phe Gly Gly Gly Thr Lys Leu Thr Val Leu
            100                 105                 110

<210>  2
<211>  110
<212>  PRT
<213>  Homo sapiens
<400>  2
Gln Ser Ala Leu Thr Gln Pro Ala Ser Val Ser Gly Ser Pro Gly Gln
1               5                   10                  15
Ser Ile Thr Ile Ser Cys Thr Gly Thr Ser Ser Asp Val Gly Gly Tyr
            20                  25                  30
Asn Tyr Val Ser Trp Tyr Gln Gln His Pro Gly Glu Ala Pro Lys Leu
        35                  40                  45
Met Ile Ser Asp Val Ser Asn Arg Pro Ser Gly Val Ser Asn Arg Phe
    50                  55                  60
Ser Gly Ser Lys Ser Gly Asn Thr Ala Ser Leu Thr Ile Ser Gly Leu
65                  70                  75                  80
Gln Ala Glu Asp Glu Ala Asp Tyr Tyr Cys Thr Ser Tyr Thr Ser Ser
                85                  90                  95
Asn Thr Leu Val Phe Gly Gly Gly Thr Lys Leu Thr Val Leu

            100                 105                 110

<210> 3
<211> 9
<212> PRT
<213> Homo sapiens
<220>
<221> SITE
<222> (1)..(9)
<223> CDR1 of Light Chain (1H11)
<400> 3
Ser Ser Asp Val Gly Gly Tyr Asn Tyr
1               5

<210> 4
<211> 3
<212> PRT
<213> Homo sapiens
<220>
<221> SITE
<222> (1)..(3)
<223> CDR2 of Light Chain (1H11)
<400> 4
Asp Val Ser
1

<210> 5
<211> 8
<212> PRT
<213> Homo sapiens
<220>
<221> SITE
<222> (1)..(8)
<223> CDR3 of Light Chain (1H11)
<400> 5
Thr Ser Tyr Thr Ser Ser Asn Thr
1               5

<210> 6
<211> 364
<212> DNA
<213> Homo sapiens
<220>
<221> CDS
<222> (1)..(363)
<223> Variable Region of Heavy Chain (1H11)
<400> 6
cag gtc cag ctg gtg cag tct ggg gct gag gtg aag aag cct ggg tcc 48
Gln Val Gln Leu Val Gln Ser Gly Ala Glu Val Lys Lys Pro Gly Ser
1               5                   10                  15
tcg gtg aag gtc tcc tgc aag tct tct gga ggc aac ttc aat aac tat 96
Ser Val Lys Val Ser Cys Lys Ser Ser Gly Gly Asn Phe Asn Asn Tyr
            20                  25                  30
gct gtc agc tgg gtg cga cag gcc cct gga caa ggc ctt gag tgg atg 144
Ala Val Ser Trp Val Arg Gln Ala Pro Gly Gln Gly Leu Glu Trp Met
        35                  40                  45
gga ggg atc agc cct atc ttt ggt tca aca aac cac gca cag aag ttc 192
Gly Gly Ile Ser Pro Ile Phe Gly Ser Thr Asn His Ala Gln Lys Phe
    50                  55                  60
cag ggc aga gtc acg att agc gcg gac ata ttc acg aac aca gcc tac 240
Gln Gly Arg Val Thr Ile Ser Ala Asp Ile Phe Thr Asn Thr Ala Tyr
65                  70                  75                  80
atg gag ctg agc agc ctg aga tct gac gac acg gcc ata tat tac tgt 288

Met Glu Leu Ser Ser Leu Arg Ser Asp Asp Thr Ala Ile Tyr Tyr Cys
                85                  90                  95
gcg aga gga cgg gga tac tac ttc tac cac gct atg gac gtc tgg ggc        336
Ala Arg Gly Arg Gly Tyr Tyr Phe Tyr His Ala Met Asp Val Trp Gly
            100                 105                 110
caa gga agt acg gtc acc gtc tcc tca g                                  364
Gln Gly Ser Thr Val Thr Val Ser Ser
        115                 120

<210>  7
<211>  121
<212>  PRT
<213>  Homo sapiens
<400>  7
Gln Val Gln Leu Val Gln Ser Gly Ala Glu Val Lys Lys Pro Gly Ser
1               5                   10                  15
Ser Val Lys Val Ser Cys Lys Ser Ser Gly Gly Asn Phe Asn Asn Tyr
            20                  25                  30
Ala Val Ser Trp Val Arg Gln Ala Pro Gly Gln Gly Leu Glu Trp Met
        35                  40                  45
Gly Gly Ile Ser Pro Ile Phe Gly Ser Thr Asn His Ala Gln Lys Phe
    50                  55                  60
Gln Gly Arg Val Thr Ile Ser Ala Asp Ile Phe Thr Asn Thr Ala Tyr
65                  70                  75                  80
Met Glu Leu Ser Ser Leu Arg Ser Asp Asp Thr Ala Ile Tyr Tyr Cys
                85                  90                  95
Ala Arg Gly Arg Gly Tyr Tyr Phe Tyr His Ala Met Asp Val Trp Gly
            100                 105                 110
Gln Gly Ser Thr Val Thr Val Ser Ser
        115                 120

<210>  8
<211>  8
<212>  PRT
<213>  Homo sapiens
<220>
<221>  SITE
<222>  (1)..(8)
<223>  CDR1 of Heavy Chain (1H11)
<400>  8
Gly Gly Asn Phe Asn Asn Tyr Ala
1               5

<210>  9
<211>  8
<212>  PRT
<213>  Homo sapiens
<220>
<221>  SITE
<222>  (1)..(8)
<223>  CDR2 of Heavy Chain (1H11)
<400>  9
Ile Ser Pro Ile Phe Gly Ser Thr
1               5

<210>  10
<211>  2
<212>  PRT
<213>  Homo sapiens
<220>
<221>  SITE
<222>  (1)..(2)
<223>  CDR3 of Heavy Chain (1H11)

<400>  10
Ala Arg
1

<210>  11
<211>  331
<212>  DNA
<213>  Homo sapiens
<220>
<221>  CDS
<222>  (1)..(330)
<223>  Variable Region of Light Chain (5G2)
<220>
<221>  misc_feature
<222>  (17)..(18)
<223>  n is a, c, g, or t
<400>  11
cag tct gcc ctg act cnn cct gcc tcc gtg tct ggg tct cct gga cag      48
Gln Ser Ala Leu Thr Xaa Pro Ala Ser Val Ser Gly Ser Pro Gly Gln
1               5                   10                  15
tcg atc acc atc tcc tgc act gga acc agc agt gac gtt ggt gat tat      96
Ser Ile Thr Ile Ser Cys Thr Gly Thr Ser Ser Asp Val Gly Asp Tyr
            20                  25                  30
aac tat gtc tcc tgg tac caa caa cac ccc ggc gaa gcc ccc gaa ctc      144
Asn Tyr Val Ser Trp Tyr Gln Gln His Pro Gly Glu Ala Pro Glu Leu
        35                  40                  45
atg att tct gat gtc agt aat cgg ccc tca ggg gtt tct gat cgc ttc      192
Met Ile Ser Asp Val Ser Asn Arg Pro Ser Gly Val Ser Asp Arg Phe
    50                  55                  60
tct ggc tcc aag tct ggc aac acg gcc tcc ctg acc atc tct ggt ctc      240
Ser Gly Ser Lys Ser Gly Asn Thr Ala Ser Leu Thr Ile Ser Gly Leu
65                  70                  75                  80
cag act gag gac gag gct gat tat tac tgc agc tca tat aca agc agc      288
Gln Thr Glu Asp Glu Ala Asp Tyr Tyr Cys Ser Ser Tyr Thr Ser Ser
                85                  90                  95
aac act ttg gtg ttc ggc gga ggg acc aga ctg acc gtc cta g            331
Asn Thr Leu Val Phe Gly Gly Gly Thr Arg Leu Thr Val Leu
            100                 105                 110

<210>  12
<211>  110
<212>  PRT
<213>  Homo sapiens
<220>
<221>  misc_feature
<222>  (6)..(6)
<223>  The 'Xaa' at location 6 stands for Gln, His, Arg, Pro, or Leu.
<400>  12
Gln Ser Ala Leu Thr Xaa Pro Ala Ser Val Ser Gly Ser Pro Gly Gln
1               5                   10                  15
Ser Ile Thr Ile Ser Cys Thr Gly Thr Ser Ser Asp Val Gly Asp Tyr
            20                  25                  30
Asn Tyr Val Ser Trp Tyr Gln Gln His Pro Gly Glu Ala Pro Glu Leu
        35                  40                  45
Met Ile Ser Asp Val Ser Asn Arg Pro Ser Gly Val Ser Asp Arg Phe
    50                  55                  60
Ser Gly Ser Lys Ser Gly Asn Thr Ala Ser Leu Thr Ile Ser Gly Leu
65                  70                  75                  80
Gln Thr Glu Asp Glu Ala Asp Tyr Tyr Cys Ser Ser Tyr Thr Ser Ser
                85                  90                  95
Asn Thr Leu Val Phe Gly Gly Gly Thr Arg Leu Thr Val Leu
            100                 105                 110

<210> 13
<211> 9
<212> PRT
<213> Homo sapiens
<220>
<221> SITE
<222> (1)..(9)
<223> CDR1 of Light Chain (5G2)
<400> 13
Ser Ser Asp Val Gly Asp Tyr Asn Tyr
1               5

<210> 14
<211> 3
<212> PRT
<213> Homo sapiens
<220>
<221> SITE
<222> (1)..(3)
<223> CDR2 of Light Chain (5G2)
<400> 14
Asp Val Ser
1

<210> 15
<211> 8
<212> PRT
<213> Homo sapiens
<220>
<221> SITE
<222> (1)..(8)
<223> CDR3 of Light Chain (5G2)
<400> 15
Ser Ser Tyr Thr Ser Ser Asn Thr
1               5

<210> 16
<211> 363
<212> DNA
<213> Homo sapiens
<220>
<221> CDS
<222> (1)..(363)
<223> Variable Region of Heavy Chain (5G2)
<400> 16
cag gtg cag ctg gtg cag tct ggg gct gag gtg aag aag cct ggg tcc      48
Gln Val Gln Leu Val Gln Ser Gly Ala Glu Val Lys Lys Pro Gly Ser
1               5                   10                  15
tcg gtg aag gtc tcc tgc aag gct tct gga ggc acc ttc aac aac tac      96
Ser Val Lys Val Ser Cys Lys Ala Ser Gly Gly Thr Phe Asn Asn Tyr
            20                  25                  30
gct atc aac tgg gtg cga cag gcc cct gga caa ggg ctt gag tgg atg      144
Ala Ile Asn Trp Val Arg Gln Ala Pro Gly Gln Gly Leu Glu Trp Met
        35                  40                  45
gga ggg atc agt ccg atc ttt ggt tca gta aac tac gca cag aag ctc      192
Gly Gly Ile Ser Pro Ile Phe Gly Ser Val Asn Tyr Ala Gln Lys Leu
    50                  55                  60
cag ggc aga gtc acc att acc gcg gac gtt ttc acg agc acg gcc tac      240
Gln Gly Arg Val Thr Ile Thr Ala Asp Val Phe Thr Ser Thr Ala Tyr
65                  70                  75                  80
atg gag ctg agc agc ctg agg tct gag gac acg gcc gtc tat tat tgt      288
Met Glu Leu Ser Ser Leu Arg Ser Glu Asp Thr Ala Val Tyr Tyr Cys
                85                  90                  95

gcg aaa ggg tcg ggt tac tac tac tac tac ggt ttg gac gtc tgg ggc     336
Ala Lys Gly Ser Gly Tyr Tyr Tyr Tyr Tyr Gly Leu Asp Val Trp Gly
            100                 105                 110
cta ggg acc acg gtc acc gtc tcc tca                                 363
Leu Gly Thr Thr Val Thr Val Ser Ser
        115                 120

<210>  17
<211>  121
<212>  PRT
<213>  Homo sapiens
<400>  17
Gln Val Gln Leu Val Gln Ser Gly Ala Glu Val Lys Lys Pro Gly Ser
1               5                   10                  15
Ser Val Lys Val Ser Cys Lys Ala Ser Gly Gly Thr Phe Asn Asn Tyr
            20                  25                  30
Ala Ile Asn Trp Val Arg Gln Ala Pro Gly Gln Gly Leu Glu Trp Met
        35                  40                  45
Gly Gly Ile Ser Pro Ile Phe Gly Ser Val Asn Tyr Ala Gln Lys Leu
    50                  55                  60
Gln Gly Arg Val Thr Ile Thr Ala Asp Val Phe Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Lys Gly Ser Gly Tyr Tyr Tyr Tyr Tyr Gly Leu Asp Val Trp Gly
            100                 105                 110
Leu Gly Thr Thr Val Thr Val Ser Ser
        115                 120

<210>  18
<211>  8
<212>  PRT
<213>  Homo sapiens
<220>
<221>  SITE
<222>  (1)..(8)
<223>  CDR1 of Heavy Chain (5G2)
<400>  18
Gly Gly Thr Phe Asn Asn Tyr Ala
1               5

<210>  19
<211>  8
<212>  PRT
<213>  Homo sapiens
<220>
<221>  SITE
<222>  (1)..(8)
<223>  CDR2 of Heavy Chain (5G2)
<400>  19
Ile Ser Pro Ile Phe Gly Ser Val
1               5

<210>  20
<211>  2
<212>  PRT
<213>  Homo sapiens
<220>
<221>  SITE
<222>  (1)..(2)
<223>  CDR3 of Heavy Chain (5G2)
<400>  20
Ala Lys

1

<210>  21
<211>  331
<212>  DNA
<213>  Homo sapiens
<220>
<221>  CDS
<222>  (1)..(330)
<223>  Variable Region of Light Chain (2H5)
<400>  21
cag tct gtg ttg acg cag ccg ccc tca gtg tct gcg gcc cca gga cag      48
Gln Ser Val Leu Thr Gln Pro Pro Ser Val Ser Ala Ala Pro Gly Gln
1               5                   10                  15
aag gtc acc atc tct tgc tct gga agc agc tcc aac att gga cat aat      96
Lys Val Thr Ile Ser Cys Ser Gly Ser Ser Ser Asn Ile Gly His Asn
            20                  25                  30
ttt gtt tcc tgg tac cag cag ctc cca gca aca gcc ccc aaa ctc ctc      144
Phe Val Ser Trp Tyr Gln Gln Leu Pro Ala Thr Ala Pro Lys Leu Leu
        35                  40                  45
att tat gac act gat aag cga ccc tca ggg att cct gac cga ttc tct      192
Ile Tyr Asp Thr Asp Lys Arg Pro Ser Gly Ile Pro Asp Arg Phe Ser
    50                  55                  60
ggc tcc aag tct gga acg tca gcc acc ctg ggc atc acc gga ctc cag      240
Gly Ser Lys Ser Gly Thr Ser Ala Thr Leu Gly Ile Thr Gly Leu Gln
65                  70                  75                  80
act ggg gac gag gcc gat tat tac tgc gga aca tgg gat agc agc ctg      288
Thr Gly Asp Glu Ala Asp Tyr Tyr Cys Gly Thr Trp Asp Ser Ser Leu
                85                  90                  95
agt tcc gtg ata ttc ggc gga ggg acc aag ctg acc gtc cta g            331
Ser Ser Val Ile Phe Gly Gly Gly Thr Lys Leu Thr Val Leu
            100                 105                 110

<210>  22
<211>  110
<212>  PRT
<213>  Homo sapiens
<400>  22
Gln Ser Val Leu Thr Gln Pro Pro Ser Val Ser Ala Ala Pro Gly Gln
1               5                   10                  15
Lys Val Thr Ile Ser Cys Ser Gly Ser Ser Ser Asn Ile Gly His Asn
            20                  25                  30
Phe Val Ser Trp Tyr Gln Gln Leu Pro Ala Thr Ala Pro Lys Leu Leu
        35                  40                  45
Ile Tyr Asp Thr Asp Lys Arg Pro Ser Gly Ile Pro Asp Arg Phe Ser
    50                  55                  60
Gly Ser Lys Ser Gly Thr Ser Ala Thr Leu Gly Ile Thr Gly Leu Gln
65                  70                  75                  80
Thr Gly Asp Glu Ala Asp Tyr Tyr Cys Gly Thr Trp Asp Ser Ser Leu
                85                  90                  95
Ser Ser Val Ile Phe Gly Gly Gly Thr Lys Leu Thr Val Leu
            100                 105                 110

<210>  23
<211>  8
<212>  PRT
<213>  Homo sapiens
<220>
<221>  SITE
<222>  (1)..(8)
<223>  CDR1 of Light Chain (2H5)
<400>  23
Ser Ser Asn Ile Gly His Asn Phe

1               5

<210>  24
<211>  3
<212>  PRT
<213>  Homo sapiens
<220>
<221>  SITE
<222>  (1)..(3)
<223>  CDR2 of Light Chain (2H5)
<400>  24
Asp Thr Asp
1

<210>  25
<211>  8
<212>  PRT
<213>  Homo sapiens
<220>
<221>  SITE
<222>  (1)..(8)
<223>  CDR3 of Light Chain (2H5)
<400>  25
Gly Thr Trp Asp Ser Ser Leu Ser
1               5

<210>  26
<211>  364
<212>  DNA
<213>  Homo sapiens
<220>
<221>  CDS
<222>  (1)..(363)
<400>  26
cag gtg cag ctg gtg cag tct ggg gct gag gtg aag aag cct ggg tcc      48
Gln Val Gln Leu Val Gln Ser Gly Ala Glu Val Lys Lys Pro Gly Ser
1               5                   10                  15
tcg gtg aag gtc tcc tgc aag tct tct gga ggc aac ttc aat aac tat      96
Ser Val Lys Val Ser Cys Lys Ser Ser Gly Gly Asn Phe Asn Asn Tyr
            20                  25                  30
gct gtc agc tgg gtg cga cag gcc cct gga caa ggc ctt gag tgg atg      144
Ala Val Ser Trp Val Arg Gln Ala Pro Gly Gln Gly Leu Glu Trp Met
        35                  40                  45
gga ggg atc agc cct atc ttt ggt tca aca aac cac gca cag aag ttc      192
Gly Gly Ile Ser Pro Ile Phe Gly Ser Thr Asn His Ala Gln Lys Phe
    50                  55                  60
cag ggc aga gtc acg att agc gcg gac ata ttc acg aac aca gcc tac      240
Gln Gly Arg Val Thr Ile Ser Ala Asp Ile Phe Thr Asn Thr Ala Tyr
65                  70                  75                  80
atg gag ctg agc agc ctg aga tct gac gac acg gcc ata tat tac tgt      288
Met Glu Leu Ser Ser Leu Arg Ser Asp Asp Thr Ala Ile Tyr Tyr Cys
                85                  90                  95
gcg aga gga cgg gga tac tac ttc tac cac gct atg gac gtc tgg ggc      336
Ala Arg Gly Arg Gly Tyr Tyr Phe Tyr His Ala Met Asp Val Trp Gly
            100                 105                 110
caa gga agt acg gtc acc gtc tcc tca g                                364
Gln Gly Ser Thr Val Thr Val Ser Ser
        115                 120

<210>  27
<211>  121
<212>  PRT
<213>  Homo sapiens

<400>  27
Gln Val Gln Leu Val Gln Ser Gly Ala Glu Val Lys Lys Pro Gly Ser
1               5                   10                  15
Ser Val Lys Val Ser Cys Lys Ser Ser Gly Gly Asn Phe Asn Asn Tyr
            20                  25                  30
Ala Val Ser Trp Val Arg Gln Ala Pro Gly Gln Gly Leu Glu Trp Met
        35                  40                  45
Gly Gly Ile Ser Pro Ile Phe Gly Ser Thr Asn His Ala Gln Lys Phe
    50                  55                  60
Gln Gly Arg Val Thr Ile Ser Ala Asp Ile Phe Thr Asn Thr Ala Tyr
65                  70                  75                  80
Met Glu Leu Ser Ser Leu Arg Ser Asp Asp Thr Ala Ile Tyr Tyr Cys
                85                  90                  95
Ala Arg Gly Arg Gly Tyr Tyr Phe Tyr His Ala Met Asp Val Trp Gly
            100                 105                 110
Gln Gly Ser Thr Val Thr Val Ser Ser
        115                 120

<210>  28
<211>  8
<212>  PRT
<213>  Homo sapiens
<220>
<221>  SITE
<222>  (1)..(8)
<223>  CDR1 of Heavy Chain (2H5)
<400>  28
Gly Gly Asn Phe Asn Asn Tyr Ala
1               5

<210>  29
<211>  8
<212>  PRT
<213>  Homo sapiens
<220>
<221>  SITE
<222>  (1)..(8)
<223>  CDR2 of Heavy Chain (2H5)
<400>  29
Ile Ser Pro Ile Phe Gly Ser Thr
1               5

<210>  30
<211>  2
<212>  PRT
<213>  Homo sapiens
<220>
<221>  SITE
<222>  (1)..(2)
<223>  CDR3 of Heavy Chain (2H5)
<400>  30
Ala Arg
1

<210>  31
<211>  1596
<212>  DNA
<213>  Influenza A virus
<220>
<221>  CDS
<222>  (3)..(1595)
<223>  A/Suita/1/200/2009 HA
<400>  31

ac aca tta tgt ata ggt tat cat gcg aac aat tca aca gac act gta 47
Thr Leu Cys Ile Gly Tyr His Ala Asn Asn Ser Thr Asp Thr Val
1               5                   10                  15
gac aca gta cta gaa aag aat gta aca gta aca cac tct gtt aac ctt 95
Asp Thr Val Leu Glu Lys Asn Val Thr Val Thr His Ser Val Asn Leu
                20                  25                  30
cta gaa gac aag cat aac ggg aaa cta tgc aaa cta aga ggg gta gcc 143
Leu Glu Asp Lys His Asn Gly Lys Leu Cys Lys Leu Arg Gly Val Ala
            35                  40                  45
cca ttg cat ttg ggt aaa tgt aac att gct ggc tgg atc ctg gga aat 191
Pro Leu His Leu Gly Lys Cys Asn Ile Ala Gly Trp Ile Leu Gly Asn
        50                  55                  60
cca gag tgt gaa tca ctc tcc aca gca agc tca tgg tcc tac att gtg 239
Pro Glu Cys Glu Ser Leu Ser Thr Ala Ser Ser Trp Ser Tyr Ile Val
    65                  70                  75
gaa aca tct agt tca gac aat gga acg tgt tac cca gga gat ttc atc 287
Glu Thr Ser Ser Ser Asp Asn Gly Thr Cys Tyr Pro Gly Asp Phe Ile
80                  85                  90                  95
gat tat gag gag cta aga gag caa ttg agc tca gtg tca tca ttt gaa 335
Asp Tyr Glu Glu Leu Arg Glu Gln Leu Ser Ser Val Ser Ser Phe Glu
                100                 105                 110
agg ttt gag ata ttc ccc aag aca agt tca tgg ccc aat cat gac tcg 383
Arg Phe Glu Ile Phe Pro Lys Thr Ser Ser Trp Pro Asn His Asp Ser
            115                 120                 125
aac aaa ggt gta acg gca gca tgt cct cat gct gga gca aaa agc ttc 431
Asn Lys Gly Val Thr Ala Ala Cys Pro His Ala Gly Ala Lys Ser Phe
        130                 135                 140
tac aaa aat tta ata tgg cta gtt aaa aaa gga aat tca tac cca aag 479
Tyr Lys Asn Leu Ile Trp Leu Val Lys Lys Gly Asn Ser Tyr Pro Lys
    145                 150                 155
ctc agc aaa tcc tac att aat gat aaa ggg aaa gaa gtc ctc gtg cta 527
Leu Ser Lys Ser Tyr Ile Asn Asp Lys Gly Lys Glu Val Leu Val Leu
160                 165                 170                 175
tgg ggc att cac cat cca tct act agt gct gac caa caa agt ctc tat 575
Trp Gly Ile His His Pro Ser Thr Ser Ala Asp Gln Gln Ser Leu Tyr
                180                 185                 190
cag aat gca gat gca tat gtt ttt gtg ggg aca tca aga tac agc aag 623
Gln Asn Ala Asp Ala Tyr Val Phe Val Gly Thr Ser Arg Tyr Ser Lys
            195                 200                 205
aag ttc aag ccg gaa ata gca ata aga ccc aaa gtg agg gat caa gaa 671
Lys Phe Lys Pro Glu Ile Ala Ile Arg Pro Lys Val Arg Asp Gln Glu
        210                 215                 220
ggg aga atg aac tat tac tgg aca cta gta gag ccg gga gac aaa ata 719
Gly Arg Met Asn Tyr Tyr Trp Thr Leu Val Glu Pro Gly Asp Lys Ile
    225                 230                 235
aca ttc gaa gca act gga aat cta gtg gta ccg aga tat gca ttc gca 767
Thr Phe Glu Ala Thr Gly Asn Leu Val Val Pro Arg Tyr Ala Phe Ala
240                 245                 250                 255
atg gaa aga gat gct gga tct ggt att atc att tca gat aca cca gtc 815
Met Glu Arg Asp Ala Gly Ser Gly Ile Ile Ile Ser Asp Thr Pro Val
                260                 265                 270
cac gat tgc aat aca act tgt cag aca ccc aag ggt gct ata aac acc 863
His Asp Cys Asn Thr Thr Cys Gln Thr Pro Lys Gly Ala Ile Asn Thr
            275                 280                 285
agc ctc cca ttt cag aat ata cat ccg atc aca att gga aaa tgt cca 911
Ser Leu Pro Phe Gln Asn Ile His Pro Ile Thr Ile Gly Lys Cys Pro
        290                 295                 300
aaa tat gta aaa agc aca aaa ttg aga ctg gcc aca gga ttg agg aat 959
Lys Tyr Val Lys Ser Thr Lys Leu Arg Leu Ala Thr Gly Leu Arg Asn
    305                 310                 315
gtc ccg tct att caa tct aga ggc cta ttt ggg gcc att gcc ggt ttc 1007
Val Pro Ser Ile Gln Ser Arg Gly Leu Phe Gly Ala Ile Ala Gly Phe
320                 325                 330                 335

att gaa ggg ggg tgg aca ggg atg gta gat gga tgg tac ggt tat cac 1055
Ile Glu Gly Gly Trp Thr Gly Met Val Asp Gly Trp Tyr Gly Tyr His
                340                 345                 350
cat caa aat gag cag ggg tca gga tat gca gcc gac ctg aag agc aca 1103
His Gln Asn Glu Gln Gly Ser Gly Tyr Ala Ala Asp Leu Lys Ser Thr
            355                 360                 365
cag aat gcc att gac gag att act aac aaa gta aat tct gtt att gaa 1151
Gln Asn Ala Ile Asp Glu Ile Thr Asn Lys Val Asn Ser Val Ile Glu
        370                 375                 380
aag atg aat aca cag ttc aca gca gta ggt aaa gag ttc aac cac ctg 1199
Lys Met Asn Thr Gln Phe Thr Ala Val Gly Lys Glu Phe Asn His Leu
    385                 390                 395
gaa aaa aga ata gag aat tta aat aaa aaa att gat gat ggt ttc ctg 1247
Glu Lys Arg Ile Glu Asn Leu Asn Lys Lys Ile Asp Asp Gly Phe Leu
400                 405                 410                 415
gac att tgg act tac aat gcc gaa ctg ttg gtt cta ttg gaa aat gaa 1295
Asp Ile Trp Thr Tyr Asn Ala Glu Leu Leu Val Leu Leu Glu Asn Glu
                420                 425                 430
aga act ttg gac tac cac gat tca aat gtg aag aac tta tat gaa aag 1343
Arg Thr Leu Asp Tyr His Asp Ser Asn Val Lys Asn Leu Tyr Glu Lys
            435                 440                 445
gta aga agc cag tta aaa aac aat gcc aag gaa att gga aac ggc tgc 1391
Val Arg Ser Gln Leu Lys Asn Asn Ala Lys Glu Ile Gly Asn Gly Cys
        450                 455                 460
ttt gaa ttt tac cac aaa tgc gat aac acg tgc atg gaa agt gtc aaa 1439
Phe Glu Phe Tyr His Lys Cys Asp Asn Thr Cys Met Glu Ser Val Lys
    465                 470                 475
aat ggg act tat gac tac cca aaa tac tca gag gaa gca aaa tta aac 1487
Asn Gly Thr Tyr Asp Tyr Pro Lys Tyr Ser Glu Glu Ala Lys Leu Asn
480                 485                 490                 495
aga gaa gaa ata gat ggg gta aag ctg gaa tca aca agg att tac cag 1535
Arg Glu Glu Ile Asp Gly Val Lys Leu Glu Ser Thr Arg Ile Tyr Gln
                500                 505                 510
att ttg gcg atc tat tca act gtc gcc agt tca ttg gta ctg gta gtc 1583
Ile Leu Ala Ile Tyr Ser Thr Val Ala Ser Ser Leu Val Leu Val Val
            515                 520                 525
tcc ctg ggg gca a 1596
Ser Leu Gly Ala
        530

<210> 32
<211> 531
<212> PRT
<213> Influenza A virus
<400> 32
Thr Leu Cys Ile Gly Tyr His Ala Asn Asn Ser Thr Asp Thr Val Asp
1               5                   10                  15
Thr Val Leu Glu Lys Asn Val Thr Val Thr His Ser Val Asn Leu Leu
            20                  25                  30
Glu Asp Lys His Asn Gly Lys Leu Cys Lys Leu Arg Gly Val Ala Pro
        35                  40                  45
Leu His Leu Gly Lys Cys Asn Ile Ala Gly Trp Ile Leu Gly Asn Pro
    50                  55                  60
Glu Cys Glu Ser Leu Ser Thr Ala Ser Ser Trp Ser Tyr Ile Val Glu
65                  70                  75                  80
Thr Ser Ser Ser Asp Asn Gly Thr Cys Tyr Pro Gly Asp Phe Ile Asp
                85                  90                  95
Tyr Glu Glu Leu Arg Glu Gln Leu Ser Ser Val Ser Ser Phe Glu Arg
            100                 105                 110
Phe Glu Ile Phe Pro Lys Thr Ser Ser Trp Pro Asn His Asp Ser Asn
        115                 120                 125
Lys Gly Val Thr Ala Ala Cys Pro His Ala Gly Ala Lys Ser Phe Tyr
    130                 135                 140

Lys Asn Leu Ile Trp Leu Val Lys Lys Gly Asn Ser Tyr Pro Lys Leu
145                 150                 155                 160
Ser Lys Ser Tyr Ile Asn Asp Lys Gly Lys Glu Val Leu Val Leu Trp
                165                 170                 175
Gly Ile His His Pro Ser Thr Ser Ala Asp Gln Gln Ser Leu Tyr Gln
            180                 185                 190
Asn Ala Asp Ala Tyr Val Phe Val Gly Thr Ser Arg Tyr Ser Lys Lys
        195                 200                 205
Phe Lys Pro Glu Ile Ala Ile Arg Pro Lys Val Arg Asp Gln Glu Gly
    210                 215                 220
Arg Met Asn Tyr Tyr Trp Thr Leu Val Glu Pro Gly Asp Lys Ile Thr
225                 230                 235                 240
Phe Glu Ala Thr Gly Asn Leu Val Val Pro Arg Tyr Ala Phe Ala Met
                245                 250                 255
Glu Arg Asp Ala Gly Ser Gly Ile Ile Ile Ser Asp Thr Pro Val His
            260                 265                 270
Asp Cys Asn Thr Thr Cys Gln Thr Pro Lys Gly Ala Ile Asn Thr Ser
        275                 280                 285
Leu Pro Phe Gln Asn Ile His Pro Ile Thr Ile Gly Lys Cys Pro Lys
    290                 295                 300
Tyr Val Lys Ser Thr Lys Leu Arg Leu Ala Thr Gly Leu Arg Asn Val
305                 310                 315                 320
Pro Ser Ile Gln Ser Arg Gly Leu Phe Gly Ala Ile Ala Gly Phe Ile
                325                 330                 335
Glu Gly Gly Trp Thr Gly Met Val Asp Gly Trp Tyr Gly Tyr His His
            340                 345                 350
Gln Asn Glu Gln Gly Ser Gly Tyr Ala Ala Asp Leu Lys Ser Thr Gln
        355                 360                 365
Asn Ala Ile Asp Glu Ile Thr Asn Lys Val Asn Ser Val Ile Glu Lys
    370                 375                 380
Met Asn Thr Gln Phe Thr Ala Val Gly Lys Glu Phe Asn His Leu Glu
385                 390                 395                 400
Lys Arg Ile Glu Asn Leu Asn Lys Lys Ile Asp Asp Gly Phe Leu Asp
                405                 410                 415
Ile Trp Thr Tyr Asn Ala Glu Leu Leu Val Leu Leu Glu Asn Glu Arg
            420                 425                 430
Thr Leu Asp Tyr His Asp Ser Asn Val Lys Asn Leu Tyr Glu Lys Val
        435                 440                 445
Arg Ser Gln Leu Lys Asn Asn Ala Lys Glu Ile Gly Asn Gly Cys Phe
    450                 455                 460
Glu Phe Tyr His Lys Cys Asp Asn Thr Cys Met Glu Ser Val Lys Asn
465                 470                 475                 480
Gly Thr Tyr Asp Tyr Pro Lys Tyr Ser Glu Glu Ala Lys Leu Asn Arg
                485                 490                 495
Glu Glu Ile Asp Gly Val Lys Leu Glu Ser Thr Arg Ile Tyr Gln Ile
            500                 505                 510
Leu Ala Ile Tyr Ser Thr Val Ala Ser Ser Leu Val Leu Val Val Ser
        515                 520                 525
Leu Gly Ala
    530

<210>  33
<211>  326
<212>  PRT
<213>  Influenza A virus
<220>
<221>  PEPTIDE
<222>  (1)..(326)
<223>  A/Suita/1/200/2009 HA1
<400>  33
Thr Leu Cys Ile Gly Tyr His Ala Asn Asn Ser Thr Asp Thr Val Asp
1               5                   10                  15
Thr Val Leu Glu Lys Asn Val Thr Val Thr His Ser Val Asn Leu Leu

20                  25                  30
Glu Asp Lys His Asn Gly Lys Leu Cys Lys Leu Arg Gly Val Ala Pro
        35                  40                  45
Leu His Leu Gly Lys Cys Asn Ile Ala Gly Trp Ile Leu Gly Asn Pro
    50                  55                  60
Glu Cys Glu Ser Leu Ser Thr Ala Ser Ser Trp Ser Tyr Ile Val Glu
65                  70                  75                  80
Thr Ser Ser Ser Asp Asn Gly Thr Cys Tyr Pro Gly Asp Phe Ile Asp
                85                  90                  95
Tyr Glu Glu Leu Arg Glu Gln Leu Ser Ser Val Ser Ser Phe Glu Arg
            100                 105                 110
Phe Glu Ile Phe Pro Lys Thr Ser Ser Trp Pro Asn His Asp Ser Asn
        115                 120                 125
Lys Gly Val Thr Ala Ala Cys Pro His Ala Gly Ala Lys Ser Phe Tyr
    130                 135                 140
Lys Asn Leu Ile Trp Leu Val Lys Lys Gly Asn Ser Tyr Pro Lys Leu
145                 150                 155                 160
Ser Lys Ser Tyr Ile Asn Asp Lys Gly Lys Glu Val Leu Val Leu Trp
                165                 170                 175
Gly Ile His His Pro Ser Thr Ser Ala Asp Gln Gln Ser Leu Tyr Gln
            180                 185                 190
Asn Ala Asp Ala Tyr Val Phe Val Gly Thr Ser Arg Tyr Ser Lys Lys
        195                 200                 205
Phe Lys Pro Glu Ile Ala Ile Arg Pro Lys Val Arg Asp Gln Glu Gly
    210                 215                 220
Arg Met Asn Tyr Tyr Trp Thr Leu Val Glu Pro Gly Asp Lys Ile Thr
225                 230                 235                 240
Phe Glu Ala Thr Gly Asn Leu Val Val Pro Arg Tyr Ala Phe Ala Met
                245                 250                 255
Glu Arg Asp Ala Gly Ser Gly Ile Ile Ile Ser Asp Thr Pro Val His
            260                 265                 270
Asp Cys Asn Thr Thr Cys Gln Thr Pro Lys Gly Ala Ile Asn Thr Ser
        275                 280                 285
Leu Pro Phe Gln Asn Ile His Pro Ile Thr Ile Gly Lys Cys Pro Lys
    290                 295                 300
Tyr Val Lys Ser Thr Lys Leu Arg Leu Ala Thr Gly Leu Arg Asn Val
305                 310                 315                 320
Pro Ser Ile Gln Ser Arg
                325

<210>  34
<211>  205
<212>  PRT
<213>  Influenza A virus
<220>
<221>  PEPTIDE
<222>  (1)..(205)
<223>  A/Suita/1/200/2009 HA2
<400>  34
Gly Leu Phe Gly Ala Ile Ala Gly Phe Ile Glu Gly Gly Trp Thr Gly
1               5                   10                  15
Met Val Asp Gly Trp Tyr Gly Tyr His His Gln Asn Glu Gln Gly Ser
            20                  25                  30
Gly Tyr Ala Ala Asp Leu Lys Ser Thr Gln Asn Ala Ile Asp Glu Ile
        35                  40                  45
Thr Asn Lys Val Asn Ser Val Ile Glu Lys Met Asn Thr Gln Phe Thr
    50                  55                  60
Ala Val Gly Lys Glu Phe Asn His Leu Glu Lys Arg Ile Glu Asn Leu
65                  70                  75                  80
Asn Lys Lys Ile Asp Asp Gly Phe Leu Asp Ile Trp Thr Tyr Asn Ala
                85                  90                  95
Glu Leu Leu Val Leu Leu Glu Asn Glu Arg Thr Leu Asp Tyr His Asp
            100                 105                 110

Ser Asn Val Lys Asn Leu Tyr Glu Lys Val Arg Ser Gln Leu Lys Asn
        115                 120                 125
Asn Ala Lys Glu Ile Gly Asn Gly Cys Phe Glu Phe Tyr His Lys Cys
    130                 135                 140
Asp Asn Thr Cys Met Glu Ser Val Lys Asn Gly Thr Tyr Asp Tyr Pro
145                 150                 155                 160
Lys Tyr Ser Glu Glu Ala Lys Leu Asn Arg Glu Glu Ile Asp Gly Val
                165                 170                 175
Lys Leu Glu Ser Thr Arg Ile Tyr Gln Ile Leu Ala Ile Tyr Ser Thr
            180                 185                 190
Val Ala Ser Ser Leu Val Leu Val Val Ser Leu Gly Ala
        195                 200                 205

<210>  35
<211>  531
<212>  PRT
<213>  Influenza A virus
<220>
<221>  PEPTIDE
<222>  (1)..(531)
<223>  5G2_1_Esc.gpt
<400>  35
Thr Leu Cys Ile Gly Tyr His Ala Asn Asn Ser Thr Asp Thr Val Asp
1               5                   10                  15
Thr Val Leu Glu Lys Asn Val Thr Val Thr His Ser Val Asn Leu Leu
            20                  25                  30
Glu Asp Lys His Asn Gly Lys Leu Cys Lys Leu Arg Gly Val Ala Pro
        35                  40                  45
Leu His Leu Gly Lys Cys Asn Ile Ala Gly Trp Ile Leu Gly Asn Pro
    50                  55                  60
Glu Cys Glu Ser Leu Ser Thr Ala Ser Ser Trp Ser Cys Ile Val Glu
65                  70                  75                  80
Thr Ser Ser Ser Asp Asn Gly Thr Cys Tyr Pro Gly Asp Phe Ile Asp
                85                  90                  95
Tyr Glu Glu Leu Arg Glu Gln Leu Ser Ser Val Ser Ser Phe Glu Arg
            100                 105                 110
Phe Glu Ile Phe Pro Lys Thr Ser Ser Trp Pro Asn His Asp Ser Asn
        115                 120                 125
Lys Gly Val Thr Ala Ala Cys Pro His Ala Gly Ala Lys Ser Phe Tyr
    130                 135                 140
Lys Asn Leu Ile Trp Leu Val Lys Lys Gly Asn Ser Tyr Pro Lys Leu
145                 150                 155                 160
Ser Lys Ser Tyr Ile Asn Asp Lys Gly Lys Glu Val Leu Val Leu Trp
                165                 170                 175
Gly Ile His His Pro Ser Thr Ser Ala Asp Gln Gln Ser Leu Tyr Gln
            180                 185                 190
Asn Ala Asp Ala Tyr Val Phe Val Gly Thr Ser Arg Tyr Ser Lys Lys
        195                 200                 205
Phe Lys Pro Glu Ile Ala Ile Arg Pro Lys Val Arg Asp Gln Glu Gly
    210                 215                 220
Arg Met Asn Tyr Tyr Trp Thr Leu Val Glu Pro Gly Asp Lys Ile Thr
225                 230                 235                 240
Phe Glu Ala Thr Gly Asn Leu Val Val Pro Arg Tyr Ala Phe Ala Met
                245                 250                 255
Glu Arg Asp Ala Gly Ser Gly Ile Ile Ile Ser Asp Thr Pro Val His
            260                 265                 270
Asp Cys Asn Thr Thr Cys Gln Thr Pro Lys Gly Ala Ile Asn Thr Ser
        275                 280                 285
Leu Pro Phe Gln Asn Ile His Pro Ile Thr Ile Gly Lys Cys Pro Lys
    290                 295                 300
Tyr Val Lys Ser Thr Lys Leu Arg Leu Ala Thr Gly Leu Arg Asn Val
305                 310                 315                 320
Pro Ser Ile Gln Ser Arg Gly Leu Phe Gly Ala Ile Ala Gly Phe Ile

325                 330                 335
Glu Gly Gly Trp Thr Gly Met Val Asp Gly Trp Tyr Gly Tyr His His
            340                 345                 350
Gln Asn Glu Gln Gly Ser Gly Tyr Ala Ala Asp Leu Lys Ser Thr Gln
        355                 360                 365
Asn Ala Ile Asp Glu Ile Thr Asn Lys Val Asn Ser Val Ile Glu Lys
    370                 375                 380
Met Asn Thr Gln Phe Thr Ala Val Gly Lys Glu Phe Asn His Leu Glu
385                 390                 395                 400
Lys Arg Ile Glu Asn Leu Asn Lys Lys Ile Asp Asp Gly Phe Leu Asp
                405                 410                 415
Ile Trp Thr Tyr Asn Ala Glu Leu Leu Val Leu Leu Glu Asn Glu Arg
            420                 425                 430
Thr Leu Asp Tyr His Asp Ser Asn Val Lys Asn Leu Tyr Glu Lys Val
        435                 440                 445
Arg Ser Gln Leu Lys Asn Asn Ala Lys Glu Ile Gly Asn Gly Cys Phe
    450                 455                 460
Glu Phe Tyr His Lys Cys Asp Asn Thr Cys Met Glu Ser Val Lys Asn
465                 470                 475                 480
Gly Thr Tyr Asp Tyr Pro Lys Tyr Ser Glu Glu Ala Lys Leu Asn Arg
                485                 490                 495
Glu Glu Ile Asp Gly Val Lys Leu Glu Ser Thr Arg Ile Tyr Gln Ile
            500                 505                 510
Leu Ala Ile Tyr Ser Thr Val Ala Ser Ser Leu Val Leu Val Val Ser
        515                 520                 525
Leu Gly Ala
    530

<210>  36
<211>  531
<212>  PRT
<213>  Influenza A virus
<220>
<221>  PEPTIDE
<222>  (1)..(531)
<223>  5E4_1_Esc.gpt
<400>  36
Thr Leu Cys Ile Gly Tyr His Ala Asn Asn Ser Thr Asp Thr Val Asp
1               5                   10                  15
Thr Val Leu Glu Lys Asn Val Thr Val Thr His Ser Val Asn Leu Leu
            20                  25                  30
Glu Asp Lys His Asn Gly Lys Leu Cys Lys Leu Arg Gly Val Ala Pro
        35                  40                  45
Leu His Leu Gly Lys Cys Asn Ile Ala Gly Trp Ile Leu Gly Asn Pro
    50                  55                  60
Glu Cys Glu Ser Leu Ser Thr Ala Ser Ser Trp Ser Tyr Ile Val Glu
65                  70                  75                  80
Thr Ser Ser Ser Asp Asn Gly Thr Cys Tyr Pro Gly Asp Phe Ile Asp
                85                  90                  95
Tyr Glu Glu Leu Arg Glu Gln Leu Ser Ser Val Ser Ser Phe Glu Arg
            100                 105                 110
Phe Glu Ile Phe Pro Asn Thr Ser Ser Trp Pro Asn His Asp Ser Asn
        115                 120                 125
Lys Gly Val Thr Ala Ala Cys Pro His Ala Gly Ala Lys Ser Phe Tyr
    130                 135                 140
Lys Asn Leu Ile Trp Leu Val Lys Lys Gly Asn Ser Tyr Pro Lys Leu
145                 150                 155                 160
Ser Lys Ser Tyr Ile Asn Asp Lys Gly Lys Glu Val Leu Val Leu Trp
                165                 170                 175
Gly Ile His His Pro Ser Thr Ser Ala Asp Gln Gln Ser Leu Tyr Gln
            180                 185                 190
Asn Ala Asp Ala Tyr Val Phe Val Gly Thr Ser Arg Tyr Ser Lys Lys
        195                 200                 205

Phe Lys Pro Glu Ile Ala Ile Arg Pro Lys Val Arg Asp Gln Glu Gly
    210                 215                 220
Arg Met Asn Tyr Tyr Trp Thr Leu Val Glu Pro Gly Asp Lys Ile Thr
225                 230                 235                 240
Phe Glu Ala Thr Gly Asn Leu Val Val Pro Arg Tyr Ala Phe Ala Met
                245                 250                 255
Glu Arg Asp Ala Gly Ser Gly Ile Ile Ile Ser Asp Thr Pro Val His
            260                 265                 270
Asp Cys Asn Thr Thr Cys Gln Thr Pro Lys Gly Ala Ile Asn Thr Ser
        275                 280                 285
Leu Pro Phe Gln Asn Ile His Pro Ile Thr Ile Gly Lys Cys Pro Lys
    290                 295                 300
Tyr Val Lys Ser Thr Lys Leu Arg Leu Ala Thr Gly Leu Arg Asn Val
305                 310                 315                 320
Pro Ser Ile Gln Ser Arg Gly Leu Phe Gly Ala Ile Ala Gly Phe Ile
                325                 330                 335
Glu Gly Gly Trp Thr Gly Met Val Asp Gly Trp Tyr Gly Tyr His His
            340                 345                 350
Gln Asn Glu Gln Gly Ser Gly Tyr Ala Ala Asp Leu Lys Ser Thr Gln
        355                 360                 365
Asn Ala Ile Asp Glu Ile Thr Asn Lys Val Asn Ser Val Ile Glu Lys
    370                 375                 380
Met Asn Thr Gln Phe Thr Ala Val Gly Lys Glu Phe Asn His Leu Glu
385                 390                 395                 400
Lys Arg Ile Glu Asn Leu Asn Lys Lys Ile Asp Asp Gly Phe Leu Asp
                405                 410                 415
Ile Trp Thr Tyr Asn Ala Glu Leu Leu Val Leu Leu Glu Asn Glu Arg
            420                 425                 430
Thr Leu Asp Tyr His Asp Ser Asn Val Lys Asn Leu Tyr Glu Lys Val
        435                 440                 445
Arg Ser Gln Leu Lys Asn Asn Ala Lys Glu Ile Gly Asn Gly Cys Phe
    450                 455                 460
Glu Phe Tyr His Lys Cys Asp Asn Thr Cys Met Glu Ser Val Lys Asn
465                 470                 475                 480
Gly Thr Tyr Asp Tyr Pro Lys Tyr Ser Glu Glu Ala Lys Leu Asn Arg
                485                 490                 495
Glu Glu Ile Asp Gly Val Lys Leu Glu Ser Thr Arg Ile Tyr Gln Ile
            500                 505                 510
Leu Ala Ile Tyr Ser Thr Val Ala Ser Ser Leu Val Leu Val Val Ser
        515                 520                 525
Leu Gly Ala
    530

<210>  37
<211>  531
<212>  PRT
<213>  Influenza A virus
<220>
<221>  PEPTIDE
<222>  (1)..(531)
<223>  5E4_2_Esc.gpt
<400>  37
Thr Leu Cys Ile Gly Tyr His Ala Asn Asn Ser Thr Asp Thr Val Asp
1               5                   10                  15
Thr Val Leu Glu Lys Asn Val Thr Val Thr His Ser Val Asn Leu Leu
            20                  25                  30
Glu Asp Lys His Asn Gly Lys Leu Cys Lys Leu Arg Gly Val Ala Pro
        35                  40                  45
Leu His Leu Gly Lys Cys Asn Ile Ala Gly Trp Ile Leu Gly Asn Pro
    50                  55                  60
Glu Cys Glu Ser Leu Ser Thr Ala Ser Ser Trp Ser Tyr Ile Val Glu
65                  70                  75                  80
Thr Ser Ser Ser Asp Asn Gly Thr Cys Tyr Pro Gly Asp Phe Ile Asp

85                  90                  95
Tyr Glu Glu Leu Arg Glu Gln Leu Ser Ser Val Ser Ser Phe Glu Arg
            100                 105                 110
Phe Glu Ile Phe Pro Lys Thr Ser Ser Trp Pro Asn His Asp Ser Asn
        115                 120                 125
Lys Gly Val Thr Ala Ala Cys Pro His Ala Gly Ala Lys Ser Phe Tyr
    130                 135                 140
Lys Asn Leu Ile Trp Leu Val Lys Lys Gly Asn Ser Tyr Pro Lys Leu
145                 150                 155                 160
Ser Lys Ser Tyr Ile Asn Asp Lys Gly Lys Glu Val Leu Val Leu Trp
                165                 170                 175
Gly Ile His His Pro Ser Thr Ser Ala Asp Gln Gln Ser Leu Tyr Gln
            180                 185                 190
Asn Ala Asp Ala Tyr Val Phe Val Gly Thr Ser Arg Tyr Ser Lys Lys
        195                 200                 205
Phe Gly Pro Glu Ile Ala Ile Arg Pro Lys Val Arg Asp Gln Glu Gly
    210                 215                 220
Arg Met Asn Tyr Tyr Trp Thr Leu Val Glu Pro Gly Asp Lys Ile Thr
225                 230                 235                 240
Phe Glu Ala Thr Gly Asn Leu Val Val Pro Arg Tyr Ala Phe Ala Met
                245                 250                 255
Glu Arg Asp Ala Gly Ser Gly Ile Ile Ile Ser Asp Thr Pro Val His
            260                 265                 270
Asp Cys Asn Thr Thr Cys Gln Thr Pro Lys Gly Ala Ile Asn Thr Ser
        275                 280                 285
Leu Pro Phe Gln Asn Ile His Pro Ile Thr Ile Gly Lys Cys Pro Lys
    290                 295                 300
Tyr Val Lys Ser Thr Lys Leu Arg Leu Ala Thr Gly Leu Arg Asn Val
305                 310                 315                 320
Pro Ser Ile Gln Ser Arg Gly Leu Phe Gly Ala Ile Ala Gly Phe Ile
                325                 330                 335
Glu Gly Gly Trp Thr Gly Met Val Asp Gly Trp Tyr Gly Tyr His His
            340                 345                 350
Gln Asn Glu Gln Gly Ser Gly Tyr Ala Ala Asp Leu Lys Ser Thr Gln
        355                 360                 365
Asn Ala Ile Asp Glu Ile Thr Asn Lys Val Asn Ser Val Ile Glu Lys
    370                 375                 380
Met Asn Thr Gln Phe Thr Ala Val Gly Lys Glu Phe Asn His Leu Glu
385                 390                 395                 400
Lys Arg Ile Glu Asn Leu Asn Lys Lys Ile Asp Asp Gly Phe Leu Asp
                405                 410                 415
Ile Trp Thr Tyr Asn Ala Glu Leu Leu Val Leu Leu Glu Asn Glu Arg
            420                 425                 430
Thr Leu Asp Tyr His Asp Ser Asn Val Lys Asn Leu Tyr Glu Lys Val
        435                 440                 445
Arg Ser Gln Leu Lys Asn Asn Ala Lys Glu Ile Gly Asn Gly Cys Phe
    450                 455                 460
Glu Phe Tyr His Lys Cys Asp Asn Thr Cys Met Glu Ser Val Lys Asn
465                 470                 475                 480
Gly Thr Tyr Asp Tyr Pro Lys Tyr Ser Glu Glu Ala Lys Leu Asn Arg
                485                 490                 495
Glu Glu Ile Asp Gly Val Lys Leu Glu Ser Thr Arg Ile Tyr Gln Ile
            500                 505                 510
Leu Ala Ile Tyr Ser Thr Val Ala Ser Ser Leu Val Leu Val Val Ser
        515                 520                 525
Leu Gly Ala
    530

<210>  38
<211>  566
<212>  PRT
<213>  Influenza A virus
<220>

<221>  PEPTIDE
<222>  (1)..(566)
<223>  H1N1pdm-OS68/09
<400>  38
Met Lys Ala Ile Leu Val Val Leu Leu Tyr Thr Phe Ala Thr Ala Asn
1               5                   10                  15
Ala Asp Thr Leu Cys Ile Gly Tyr His Ala Asn Asn Ser Thr Asp Thr
            20                  25                  30
Val Asp Thr Val Leu Glu Lys Asn Val Thr Val Thr His Ser Val Asn
        35                  40                  45
Leu Leu Glu Asp Lys His Asn Gly Lys Leu Cys Lys Leu Arg Gly Val
    50                  55                  60
Ala Pro Leu His Leu Gly Lys Cys Asn Ile Ala Gly Trp Ile Leu Gly
65                  70                  75                  80
Asn Pro Glu Cys Glu Ser Leu Ser Thr Ala Ser Ser Trp Ser Tyr Ile
                85                  90                  95
Val Glu Thr Ser Ser Ser Asp Asn Gly Thr Cys Tyr Pro Gly Asp Phe
            100                 105                 110
Ile Asp Tyr Glu Glu Leu Arg Glu Gln Leu Ser Ser Val Ser Ser Phe
        115                 120                 125
Glu Arg Phe Glu Ile Phe Pro Lys Thr Ser Ser Trp Pro Asn His Asp
    130                 135                 140
Ser Asn Lys Gly Val Thr Ala Ala Cys Pro His Ala Gly Ala Lys Ser
145                 150                 155                 160
Phe Tyr Lys Asn Leu Ile Trp Leu Val Lys Lys Gly Asn Ser Tyr Pro
                165                 170                 175
Lys Leu Ser Lys Ser Tyr Ile Asn Asp Lys Gly Lys Glu Val Leu Val
            180                 185                 190
Leu Trp Gly Ile His His Pro Ser Thr Ser Ala Asp Gln Gln Ser Leu
        195                 200                 205
Tyr Gln Asn Ala Asp Ala Tyr Val Phe Val Gly Thr Ser Arg Tyr Ser
    210                 215                 220
Lys Lys Phe Lys Pro Glu Ile Ala Ile Arg Pro Lys Val Arg Asp Gln
225                 230                 235                 240
Glu Gly Arg Met Asn Tyr Tyr Trp Thr Leu Val Glu Pro Gly Asp Lys
                245                 250                 255
Ile Thr Phe Glu Ala Thr Gly Asn Leu Val Val Pro Arg Tyr Ala Phe
            260                 265                 270
Ala Met Glu Arg Asn Ala Gly Ser Gly Ile Ile Ile Ser Asp Thr Pro
        275                 280                 285
Val His Asp Cys Asn Thr Thr Cys Gln Thr Pro Lys Gly Ala Ile Asn
    290                 295                 300
Thr Ser Leu Pro Phe Gln Asn Ile His Pro Ile Thr Ile Gly Lys Cys
305                 310                 315                 320
Pro Lys Tyr Val Lys Ser Thr Lys Leu Arg Leu Ala Thr Gly Leu Arg
                325                 330                 335
Asn Val Pro Ser Ile Gln Ser Arg Gly Leu Phe Gly Ala Ile Ala Gly
            340                 345                 350
Phe Ile Glu Gly Gly Trp Thr Gly Met Val Asp Gly Trp Tyr Gly Tyr
        355                 360                 365
His His Gln Asn Glu Gln Gly Ser Gly Tyr Ala Ala Asp Leu Lys Ser
    370                 375                 380
Thr Gln Asn Ala Ile Asp Glu Ile Thr Asn Lys Val Asn Ser Val Ile
385                 390                 395                 400
Glu Lys Met Asn Thr Gln Phe Thr Ala Val Gly Lys Glu Phe Asn His
                405                 410                 415
Leu Glu Lys Arg Ile Glu Asn Leu Asn Lys Lys Val Asp Asp Gly Phe
            420                 425                 430
Leu Asp Ile Trp Thr Tyr Asn Ala Glu Leu Leu Val Leu Leu Glu Asn
        435                 440                 445
Glu Arg Thr Leu Asp Tyr His Asp Ser Asn Val Lys Asn Leu Tyr Glu
    450                 455                 460
Lys Val Arg Ser Gln Leu Lys Asn Asn Ala Lys Glu Ile Gly Asn Gly

465                 470                 475                 480
Cys Phe Glu Phe Tyr His Lys Cys Asp Asn Thr Cys Met Glu Ser Val
                485                 490                 495
Lys Asn Gly Thr Tyr Asp Tyr Pro Lys Tyr Ser Glu Glu Ala Lys Leu
            500                 505                 510
Asn Arg Glu Glu Ile Asp Gly Val Lys Leu Glu Ser Thr Arg Ile Tyr
        515                 520                 525
Gln Ile Leu Ala Ile Tyr Ser Thr Val Ala Ser Ser Leu Val Leu Val
    530                 535                 540
Val Ser Leu Gly Ala Ile Ser Phe Trp Met Cys Ser Asn Gly Ser Leu
545                 550                 555                 560
Gln Cys Arg Ile Cys Ile
                565

<210>  39
<211>  566
<212>  PRT
<213>  Influenza A virus
<220>
<221>  PEPTIDE
<222>  (1)..(566)
<223>  H1N1pdm-CA07/09
<400>  39
Met Lys Ala Ile Leu Val Val Leu Leu Tyr Thr Phe Ala Thr Ala Asn
1               5                   10                  15
Ala Asp Thr Leu Cys Ile Gly Tyr His Ala Asn Asn Ser Thr Asp Thr
            20                  25                  30
Val Asp Thr Val Leu Glu Lys Asn Val Thr Val Thr His Ser Val Asn
        35                  40                  45
Leu Leu Glu Asp Lys His Asn Gly Lys Leu Cys Lys Leu Arg Gly Val
    50                  55                  60
Ala Pro Leu His Leu Gly Lys Cys Asn Ile Ala Gly Trp Ile Leu Gly
65                  70                  75                  80
Asn Pro Glu Cys Glu Ser Leu Ser Thr Ala Ser Ser Trp Ser Tyr Ile
                85                  90                  95
Val Glu Thr Ser Ser Ser Asp Asn Gly Thr Cys Tyr Pro Gly Asp Phe
            100                 105                 110
Ile Asp Tyr Glu Glu Leu Arg Glu Gln Leu Ser Ser Val Ser Ser Phe
        115                 120                 125
Glu Arg Phe Glu Ile Phe Pro Lys Thr Ser Ser Trp Pro Asn His Asp
    130                 135                 140
Ser Asn Lys Gly Val Thr Ala Ala Cys Pro His Ala Gly Ala Lys Ser
145                 150                 155                 160
Phe Tyr Lys Asn Leu Ile Trp Leu Val Lys Lys Gly Asn Ser Tyr Pro
                165                 170                 175
Lys Leu Ser Lys Ser Tyr Ile Asn Asp Lys Gly Lys Glu Val Leu Val
            180                 185                 190
Leu Trp Gly Ile His His Pro Pro Thr Ser Ala Asp Gln Gln Ser Leu
        195                 200                 205
Tyr Gln Asn Ala Asp Ala Tyr Val Phe Val Gly Ser Ser Arg Tyr Ser
    210                 215                 220
Lys Lys Phe Lys Pro Glu Ile Ala Ile Arg Pro Lys Val Arg Asp Gln
225                 230                 235                 240
Glu Gly Arg Met Asn Tyr Tyr Trp Thr Leu Val Glu Pro Gly Asp Lys
                245                 250                 255
Ile Thr Phe Glu Ala Thr Gly Asn Leu Val Val Pro Arg Tyr Ala Phe
            260                 265                 270
Ala Met Glu Arg Asn Ala Gly Ser Gly Ile Ile Ile Ser Asp Thr Pro
        275                 280                 285
Val His Asp Cys Asn Thr Thr Cys Gln Thr Pro Lys Gly Ala Ile Asn
    290                 295                 300
Thr Ser Leu Pro Phe Gln Asn Ile His Pro Ile Thr Ile Gly Lys Cys
305                 310                 315                 320

Pro Lys Tyr Val Lys Ser Thr Lys Leu Arg Leu Ala Thr Gly Leu Arg
                325                 330                 335
Asn Ile Pro Ser Ile Gln Ser Arg Gly Leu Phe Gly Ala Ile Ala Gly
            340                 345                 350
Phe Ile Glu Gly Gly Trp Thr Gly Met Val Asp Gly Trp Tyr Gly Tyr
        355                 360                 365
His His Gln Asn Glu Gln Gly Ser Gly Tyr Ala Ala Asp Leu Lys Ser
    370                 375                 380
Thr Gln Asn Ala Ile Asp Glu Ile Thr Asn Lys Val Asn Ser Val Ile
385                 390                 395                 400
Glu Lys Met Asn Thr Gln Phe Thr Ala Val Gly Lys Glu Phe Asn His
                405                 410                 415
Leu Glu Lys Arg Ile Glu Asn Leu Asn Lys Lys Val Asp Asp Gly Phe
            420                 425                 430
Leu Asp Ile Trp Thr Tyr Asn Ala Glu Leu Leu Val Leu Leu Glu Asn
        435                 440                 445
Glu Arg Thr Leu Asp Tyr His Asp Ser Asn Val Lys Asn Leu Tyr Glu
    450                 455                 460
Lys Val Arg Ser Gln Leu Lys Asn Asn Ala Lys Glu Ile Gly Asn Gly
465                 470                 475                 480
Cys Phe Glu Phe Tyr His Lys Cys Asp Asn Thr Cys Met Glu Ser Val
                485                 490                 495
Lys Asn Gly Thr Tyr Asp Tyr Pro Lys Tyr Ser Glu Glu Ala Lys Leu
            500                 505                 510
Asn Arg Glu Glu Ile Asp Gly Val Lys Leu Glu Ser Thr Arg Ile Tyr
        515                 520                 525
Gln Ile Leu Ala Ile Tyr Ser Thr Val Ala Ser Ser Leu Val Leu Val
    530                 535                 540
Val Ser Leu Gly Ala Ile Ser Phe Trp Met Cys Ser Asn Gly Ser Leu
545                 550                 555                 560
Gln Cys Arg Ile Cys Ile
                565

<210>  40
<211>  535
<212>  PRT
<213>  Influenza A virus
<220>
<221>  PEPTIDE
<222>  (1)..(535)
<223>  H1N1pdm-SU17/11
<400>  40
Ala Asn Ala Asp Thr Leu Cys Ile Gly Tyr His Ala Asn Asn Ser Thr
1               5                   10                  15
Asp Thr Val Asp Thr Val Leu Glu Lys Asn Val Thr Val Thr His Ser
            20                  25                  30
Val Asn Leu Leu Glu Asp Lys His Asn Gly Lys Leu Cys Lys Leu Arg
        35                  40                  45
Gly Val Ala Pro Leu His Leu Gly Lys Cys Asn Ile Ala Gly Trp Ile
    50                  55                  60
Leu Gly Asn Pro Glu Cys Glu Ser Leu Ser Thr Ala Ser Ser Trp Ser
65                  70                  75                  80
Tyr Ile Val Glu Thr Ser Ser Ser Asp Asn Gly Thr Cys Tyr Pro Gly
                85                  90                  95
Asp Phe Ile Asp Tyr Glu Glu Leu Arg Glu Gln Leu Ser Ser Val Ser
            100                 105                 110
Ser Phe Glu Arg Phe Glu Ile Phe Pro Lys Thr Ser Ser Trp Pro Asp
        115                 120                 125
His Asp Ser Asn Lys Gly Val Thr Ala Ala Cys Pro His Ala Gly Ala
    130                 135                 140
Lys Ser Phe Tyr Lys Asn Leu Ile Trp Leu Val Lys Lys Gly Asn Ser
145                 150                 155                 160
Tyr Pro Lys Leu Ser Lys Ser Tyr Ile Asn Asp Lys Gly Lys Glu Val

165                 170                 175
Leu Val Leu Trp Gly Ile His His Pro Ser Thr Ser Ala Asp Gln Gln
            180                 185                 190
Ser Leu Tyr Gln Asn Ala Asp Ala Tyr Val Phe Val Gly Thr Ser Arg
        195                 200                 205
Tyr Ser Lys Lys Phe Lys Pro Glu Ile Ala Ile Arg Pro Lys Val Arg
    210                 215                 220
Asp Gln Glu Gly Arg Met Asn Tyr Tyr Trp Thr Leu Val Glu Pro Gly
225                 230                 235                 240
Asp Lys Ile Thr Phe Glu Ala Thr Gly Asn Leu Val Val Pro Arg Tyr
                245                 250                 255
Ala Phe Ala Met Glu Arg Asn Ala Gly Ser Gly Ile Ile Ile Ser Asp
            260                 265                 270
Thr Pro Val His Asp Cys Asn Thr Thr Cys Gln Thr Pro Lys Gly Ala
        275                 280                 285
Ile Asn Thr Ser Leu Pro Phe Gln Asn Ile His Pro Ile Thr Ile Gly
    290                 295                 300
Lys Cys Pro Lys Tyr Val Lys Ser Thr Lys Leu Arg Leu Ala Thr Gly
305                 310                 315                 320
Leu Arg Asn Val Pro Ser Ile Gln Ser Arg Gly Leu Phe Gly Ala Ile
                325                 330                 335
Ala Gly Phe Ile Glu Gly Gly Trp Thr Gly Met Val Asp Gly Trp Tyr
            340                 345                 350
Gly Tyr His His Gln Asn Glu Gln Gly Ser Gly Tyr Ala Ala Asp Leu
        355                 360                 365
Lys Ser Thr Gln Asn Ala Ile Asp Lys Ile Thr Asn Lys Val Asn Ser
    370                 375                 380
Val Ile Glu Lys Met Asn Thr Gln Phe Thr Ala Val Gly Lys Glu Phe
385                 390                 395                 400
Asn His Leu Glu Lys Arg Ile Glu Asn Leu Asn Lys Lys Val Asp Asp
                405                 410                 415
Gly Phe Leu Asp Ile Trp Thr Tyr Asn Ala Glu Leu Leu Val Leu Leu
            420                 425                 430
Glu Asn Glu Arg Thr Leu Asp Tyr His Asp Ser Asn Val Lys Asn Leu
        435                 440                 445
Tyr Glu Lys Val Arg Ser Gln Leu Lys Asn Asn Ala Lys Glu Ile Gly
    450                 455                 460
Asn Gly Cys Phe Glu Phe Tyr His Lys Cys Asp Asn Thr Cys Met Glu
465                 470                 475                 480
Ser Val Lys Asn Gly Thr Tyr Asp Tyr Pro Lys Tyr Ser Glu Glu Ala
                485                 490                 495
Lys Leu Asn Arg Glu Glu Ile Asp Gly Val Lys Leu Glu Ser Thr Arg
            500                 505                 510
Ile Tyr Gln Ile Leu Ala Ile Tyr Ser Thr Val Ala Ser Ser Leu Val
        515                 520                 525
Leu Val Val Ser Leu Gly Ala
    530                 535

<210>  41
<211>  535
<212>  PRT
<213>  Influenza A virus
<220>
<221>  PEPTIDE
<222>  (1)..(535)
<223>  H1N1pdm-SU04/11
<400>  41
Ala Asn Ala Asp Thr Leu Cys Ile Gly Tyr His Ala Asn Asn Ser Thr
1               5                   10                  15
Asp Thr Val Asp Thr Val Leu Glu Lys Asn Val Thr Val Thr His Ser
            20                  25                  30
Val Asn Leu Leu Glu Asp Lys His Asn Gly Lys Leu Cys Lys Leu Arg
        35                  40                  45

Gly Val Ala Pro Leu His Leu Gly Lys Cys Asn Ile Ala Gly Trp Ile
    50                  55                  60
Leu Gly Asn Pro Glu Cys Glu Ser Leu Ser Thr Ala Ser Ser Trp Ser
65                  70                  75                  80
Tyr Ile Val Glu Thr Ser Ser Ser Asp Asn Gly Thr Cys Tyr Pro Gly
                85                  90                  95
Asp Phe Ile Asp Tyr Glu Glu Leu Arg Glu Gln Leu Ser Ser Val Ser
            100                 105                 110
Ser Phe Glu Arg Phe Glu Ile Phe Pro Lys Thr Ser Ser Trp Pro Asn
        115                 120                 125
His Asp Ser Asn Lys Gly Val Thr Ala Ala Cys Pro Arg Ala Gly Ala
    130                 135                 140
Lys Gly Phe Tyr Lys Asn Leu Ile Trp Leu Val Lys Lys Gly Asn Ser
145                 150                 155                 160
Tyr Pro Lys Leu Asn Lys Ser Tyr Ile Asn Asp Lys Gly Lys Glu Val
                165                 170                 175
Leu Val Leu Trp Gly Ile His His Pro Ser Thr Thr Ala Asp Gln Gln
            180                 185                 190
Ser Leu Tyr Gln Asn Ala Asp Thr Tyr Val Phe Val Gly Thr Ser Arg
        195                 200                 205
Tyr Ser Lys Lys Phe Lys Pro Glu Ile Glu Ile Arg Pro Lys Val Arg
    210                 215                 220
Asp Gln Glu Gly Arg Met Asn Tyr Tyr Trp Thr Leu Val Glu Pro Gly
225                 230                 235                 240
Asp Lys Ile Thr Phe Glu Ala Thr Gly Asn Leu Val Val Pro Arg Tyr
                245                 250                 255
Ala Phe Ala Met Glu Arg Asn Ala Gly Ser Gly Ile Ile Ile Ser Asp
            260                 265                 270
Thr Pro Val His Asp Cys Asn Thr Thr Cys Gln Thr Pro Lys Gly Ala
        275                 280                 285
Ile Asn Thr Ser Leu Pro Phe Gln Asn Ile His Pro Ile Thr Ile Gly
    290                 295                 300
Lys Cys Pro Lys Tyr Val Lys Ser Thr Lys Leu Arg Leu Ala Thr Gly
305                 310                 315                 320
Leu Arg Asn Val Pro Ser Ile Gln Ser Arg Gly Leu Phe Gly Ala Ile
                325                 330                 335
Ala Gly Phe Ile Glu Gly Gly Trp Thr Gly Met Val Asp Gly Trp Tyr
            340                 345                 350
Gly Tyr His His Gln Asn Glu Gln Gly Ser Gly Tyr Ala Ala Asp Leu
        355                 360                 365
Lys Ser Thr Gln Asn Ala Ile Asp Lys Ile Thr Asn Lys Val Asn Ser
    370                 375                 380
Val Ile Glu Lys Met Asn Thr Gln Phe Thr Ala Val Asp Lys Glu Phe
385                 390                 395                 400
Asn His Leu Glu Lys Arg Ile Glu Asn Leu Asn Lys Lys Val Asp Asp
                405                 410                 415
Gly Phe Leu Asp Ile Trp Thr Tyr Asn Ala Glu Leu Leu Val Leu Leu
            420                 425                 430
Glu Asn Glu Arg Thr Leu Asp Tyr His Asp Ser Asn Val Lys Asn Leu
        435                 440                 445
Tyr Glu Lys Val Arg Asn Gln Leu Lys Asn Asn Ala Lys Glu Ile Gly
    450                 455                 460
Asn Gly Cys Phe Glu Phe Tyr His Lys Cys Asp Asn Thr Cys Met Glu
465                 470                 475                 480
Ser Val Lys Asn Gly Thr Tyr Asp Tyr Pro Lys Tyr Ser Glu Glu Ala
                485                 490                 495
Lys Leu Asn Arg Glu Glu Ile Asp Gly Val Lys Leu Glu Ser Thr Arg
            500                 505                 510
Ile Tyr Gln Ile Leu Ala Ile Tyr Ser Thr Val Ala Ser Ser Leu Val
        515                 520                 525
Leu Val Val Ser Leu Gly Ala
    530                 535

<210>  42
<211>  535
<212>  PRT
<213>  Influenza A virus
<220>
<221>  PEPTIDE
<222>  (1)..(535)
<223>  H1N1pdm-SU05/11
<400>  42
Ala Asn Ala Asp Thr Leu Cys Ile Gly Tyr His Ala Asn Asn Ser Thr
1               5                   10                  15
Asp Thr Val Asp Thr Val Leu Glu Lys Asn Val Thr Val Thr His Ser
            20                  25                  30
Val Asn Leu Leu Glu Asp Lys His Asn Gly Lys Leu Cys Lys Leu Arg
        35                  40                  45
Gly Val Ala Pro Leu His Leu Gly Lys Cys Asn Ile Ala Gly Trp Ile
    50                  55                  60
Leu Gly Asn Pro Glu Cys Glu Ser Leu Ser Thr Ala Ser Ser Trp Ser
65                  70                  75                  80
Tyr Ile Val Glu Thr Ser Ser Ser Asp Asn Gly Thr Cys Tyr Pro Gly
                85                  90                  95
Asp Phe Ile Asp Tyr Glu Glu Leu Arg Glu Gln Leu Ser Ser Val Ser
            100                 105                 110
Ser Phe Glu Arg Phe Glu Ile Phe Pro Lys Thr Ser Ser Trp Pro Asn
        115                 120                 125
His Asp Ser Asn Lys Gly Val Thr Ala Ala Cys Pro Arg Ala Gly Ala
    130                 135                 140
Lys Gly Phe Tyr Lys Asn Leu Ile Trp Leu Val Lys Lys Gly Asn Ser
145                 150                 155                 160
Tyr Pro Lys Leu Asn Lys Ser Tyr Ile Asn Asp Lys Gly Lys Glu Val
                165                 170                 175
Leu Val Leu Trp Gly Ile His His Pro Ser Thr Thr Ala Asp Gln Gln
            180                 185                 190
Ser Leu Tyr Gln Asn Ala Asp Thr Tyr Val Phe Val Gly Thr Ser Arg
        195                 200                 205
Tyr Ser Lys Lys Phe Lys Pro Glu Ile Glu Ile Arg Pro Lys Val Arg
    210                 215                 220
Asp Gln Glu Gly Arg Met Asn Tyr Tyr Trp Thr Leu Val Glu Pro Gly
225                 230                 235                 240
Asp Lys Ile Thr Phe Glu Ala Thr Gly Asn Leu Val Val Pro Arg Tyr
                245                 250                 255
Ala Phe Ala Met Glu Arg Asn Ala Gly Ser Gly Ile Ile Ile Ser Asp
            260                 265                 270
Thr Pro Val His Asp Cys Asn Thr Thr Cys Gln Thr Pro Lys Gly Ala
        275                 280                 285
Ile Asn Thr Ser Leu Pro Phe Gln Asn Ile His Pro Ile Thr Ile Gly
    290                 295                 300
Lys Cys Pro Lys Tyr Val Lys Ser Thr Lys Leu Arg Leu Ala Thr Gly
305                 310                 315                 320
Leu Arg Asn Val Pro Ser Ile Gln Ser Arg Gly Leu Phe Gly Ala Ile
                325                 330                 335
Ala Gly Phe Ile Glu Gly Gly Trp Thr Gly Met Val Asp Gly Trp Tyr
            340                 345                 350
Gly Tyr His His Gln Asn Glu Gln Gly Ser Gly Tyr Ala Ala Asp Leu
        355                 360                 365
Lys Ser Thr Gln Asn Ala Ile Asp Lys Ile Thr Asn Lys Val Asn Ser
    370                 375                 380
Val Ile Glu Lys Met Asn Thr Gln Phe Thr Ala Val Asp Lys Glu Phe
385                 390                 395                 400
Asn His Leu Glu Lys Arg Ile Glu Asn Leu Asn Lys Lys Val Asp Asp
                405                 410                 415
Gly Phe Leu Asp Ile Trp Thr Tyr Asn Ala Glu Leu Leu Val Leu Leu
            420                 425                 430

Glu Asn Glu Arg Thr Leu Asp Tyr His Asp Ser Asn Val Lys Asn Leu
        435                 440                 445
Tyr Glu Lys Val Arg Asn Gln Leu Lys Asn Asn Ala Lys Glu Ile Gly
    450                 455                 460
Asn Gly Cys Phe Glu Phe Tyr His Lys Cys Asp Asn Thr Cys Met Glu
465                 470                 475                 480
Ser Val Lys Asn Gly Thr Tyr Asp Tyr Pro Lys Tyr Ser Glu Glu Ala
                485                 490                 495
Lys Leu Asn Arg Glu Glu Ile Asp Gly Val Lys Leu Glu Ser Thr Arg
            500                 505                 510
Ile Tyr Gln Ile Leu Ala Ile Tyr Ser Thr Val Ala Ser Ser Leu Val
        515                 520                 525
Leu Val Val Ser Leu Gly Ala
    530                 535

<210>  43
<211>  28
<212>  DNA
<213>  Artificial Sequence
<220>
<223>  Artificially synthesized primer sequence (HA Forward)
<400>  43
tattcgtctc agggagcaaa agcagggg                                          28

<210>  44
<211>  35
<212>  DNA
<213>  Artificial Sequence
<220>
<223>  Artificially synthesized primer sequence (HA Reverse)
<400>  44
atatcgtctc gtattagtag aaacaagggt gtttt                                  35

<210>  45
<211>  24
<212>  DNA
<213>  Artificial Sequence
<220>
<223>  Artificially synthesized primer sequence (Heavy Chain Forward)
<400>  45
atggagtttg ggctgagctg ggtt                                              24

<210>  46
<211>  25
<212>  DNA
<213>  Artificial Sequence
<220>
<223>  Artificially synthesized primer sequence (Heavy Chain Reverse)
<400>  46
ctcccgcggc tttgtcttgg catta                                             25

<210>  47
<211>  24
<212>  DNA
<213>  Artificial Sequence
<220>
<223>  Artificially synthesized primer sequence (Light Chain Forward)
<400>  47
atggcctggr ycycmytcyw cctm                                              24

<210>  48
<211>  25
<212>  DNA

<213>  Artificial Sequence
<220>
<223>  Artificially synthesized primer sequence (Light Chain Reverse)
<400>  48
tggcagctgt agcttctgtg ggact                                          25

## Description

### [Technical Field]

The present invention relates to an antibody having a broad neutralization activity against group 1 influenza A viruses. More specifically, the present invention relates to an antibody having a neutralization activity against subtype H1 and subtype H5 of group 1 influenza A viruses.

### [Background Art]

Influenza is a viral disease that spreads around the world as seasonal epidemics. In addition, influenza results in three to five million yearly cases of severe illness and about 500, 000 yearly deaths. Moreover, in some pandemic years, the number of deaths due to influenza rises to millions worldwide. In the 20th century, three influenza pandemics have occurred, each caused by the emergence of new strains that had become likely to infect human, leading to the killing of ten million people.

Sometimes, new influenza strains appear when an existing influenza virus spreads to human from another animal species, or when an existing human-infecting influenza virus strain acquires a new gene from a virus that usually infects birds or pigs. In fact, a new influenza virus strain, swine-origin pandemic H1N1 (2009) (H1N1pdm) evolved in April 2009, had a combination of genes from human, pig, and bird infectious influenza viruses. Further, since H1N1pdm acquired the ability to infect human due to the gene combination, it quickly spread all over the world and induced many severe cases. Moreover, according to the experience of the H1N1pdm pandemic at that time, a highly pathogenic avian influenza virus strain, named H5N1, which emerged in Asia in the 1990s, also raised a concern of evolving a new influenza pandemic, thus drawing attention for the possible emergence of H5N1-derived pandemic ever since.

Influenza virus is classified into three genera (type A, type B, type C), and any of H1N1pdm, H5N1, and the like belongs to type A. The difference among type A, type B, and type C is based on the difference in antigenicities of the M1 protein and the NP protein among proteins constituting virus particles. Moreover, type A influenza virus are classified into two groups based on the difference in antigenicities of hemagglutinin (hereinafter also simply referred to as "HA") and neuraminidase (NA) which are molecules on the surface of an envelope, and further classified into multiple subtypes , multiple sub-types , and furthermore multiple strains (isolates). To be more specific, influenza A viruses are classified into group 1 including subtypes H1, H2, H5, H6, H8, H9, H11, H12, H13, and H16; and group 2 including subtypes H3, H4, H7, H10, H14, and H15. These subtypes are further classified into the above-described sub-types such as H1N1 and H5N1.

HA of influenza A viruses is known as follows. HA is constituted of regions of head regions (globular head regions) and the stem region or stalk region having structures different from each other. The regions contain a receptor binding site so that the virus can bind to target cells, and are involved in the blood agglutination activity of HA. The stalk region contains a fusion peptide necessary for the membrane fusion between the viral envelope and the endosome membrane of cells, and is involved in the fusion activity.

Moreover, there are several options for the treatment and prevention against such influenza virus infectious disease. For example, several small molecules are used for the treatment against influenza: neuraminidase inhibitors zanamivir (Relenza) and oseltamivir (Tamiflu) which inhibit release of nascent virus particles (virions) ; and amantadine which inhibits the M2 channel proton conducting activity.

However, the efficacy of small molecule administration in the influenza treatment and prevention is limited to 48 hours or shorter after the onset of symptoms. Further, the excessive use of small molecules leads to resistant viruses that surprisingly reduce the efficacy of the small molecules by the escape mutations, thereby contributing to rapid worldwide spread.

Vaccination is another option for the influenza prevention. However, this approach provides important limitations due to the accumulation of antigenic mutations in the virus, known as antigenic drift. Meanwhile, current seasonal influenza vaccines predominantly induce anti-HA antibodies and specifically target HA head regions to block the receptor-binding function. These responses are usually virus strain-specific neutralization, and therefore need to be annually reformulated based on the forecasting of viral antigens that will circulate in the coming influenza season. Furthermore, under a pandemic situation, a formulation targeting a current pandemic virus is necessary. On the other hand, the efficacy of vaccine strategy in the treatment and prevention against pandemic is very limited by the time required for the vaccine development and production.

As described above, earnest studies have been conducted against influenza viruses, particularly H1N1pdm, H1N1sea, H5N1, and the like, and various treatment and prevention methods have been tried. Nevertheless, sufficiently effective methods have not been established yet, and researches and developments for these have been earnestly in progress.

Under such situations, human monoclonal antibodies (HuMAbs) capable of exhibiting a specificity to broad virus strains have very desirable properties such as long-term stability in serum, high specificity, and low immunogenicity. Accordingly, HuMAbs have attracted attention as prophylactic drugs and therapeutic drugs against influenza and so forth. Particularly, in the treatment using such a drug as small molecules described above, when the molecules are administered 48 hours after the onset of symptoms, the efficacy is hardly observed. In contrast, in the treatment with such an antibody 48 hours after the onset of symptoms, the efficacy can be expected. Further, the broad protection range thereof can cope with: the concern of the escape mutations which occur in the treatment with the small molecules; and viral antigens which change every year in such a manner that the vaccine strategy is made ineffective.

Hence, antibodies capable of exhibiting a global neutralization activity against influenza viruses have attracted attention, and a wide variety of anti-influenza virus antibodies have been researched and developed. For example, human- and mouse-derived antibodies against group 1 influenza A viruses have been reported (NPLs 1 to 10). Moreover, antibodies against group 2 influenza A viruses have been reported in NPLs 11 to 14. Further, antibodies against both group 1 and 2 inf luenza A viruses and antibodies against influenza B viruses of both Yamagata and Victoria lineages have also been reported respectively in NPLs 11 and 15 to 18 and NPLs 19 and 20. In addition, while some of the neutralizing HuMAbs with broad cross-reactivity are antibodies capable of recognizing head portions of HA (NPLs 7, 8, 14, 21 to 24, and 26), most of them are antibodies capable of recognizing the stalk region of HA (NPLs 1 to 6, 8, 19, and 25).

As described above, the research and development of various neutralizing antibodies with broad cross-reactivity have been in progress. However, under current situations, no antibody has been developed yet which is capable of exhibiting a global neutralization activity and effective in the treatment and the like against influenza viruses, particularly group 1 influenza A viruses including H1N1pdm having induced pandemic recently and H5N1 having a risk of inducing pandemic in the future.

### [Citation List]

### [Non Patent Literatures]

[NPL 1] Wrammert J et al., J Exp Med, 2011, vol. 208, pp. 181-193
[NPL 2] Ekiert DC et al., Science, 2009, vol. 324, pp. 246-251
[NPL 3] Sui J et al., Nat Struct Mol Biol, 2009, vol. 16, pp. 265-273
[NPL4] Okuno Yetal., JVirol, 1993, vol. 67, pp. 2552-2558
[NPL 5] Tan GS et al., JVirol, 2012, vol. 86, pp. 6179-6188
[NPL 6] Throsby M et al., Heterosubtypic neutralizing monoclonal antibodies cross-protective against H5N1 and H1N1 recovered from human IgM+ memory B cells., PLoS One, 2008, 3: e3942.
[NPL 7] Ekiert DC et al., Nature, 2012, vol. 489, pp. 526-532
[NPL 8] De Marco D et al., A non-VH1-69 heterosubtypic neutralizing human monoclonal antibody protects mice against H1N1 and H5N1 viruses. , PLoS One, 2012, 7: e34415.
[NPL 9] Kashyap AK et al., Proc Natl Acad Sci USA, 2008, vol. 105, pp. 5986-5991
[NPL 10] Kashyap AK et al., Protection from the 2009 H1N1 pandemic influenza by an antibody from combinatorial survivor-basedlibraries., PLoS Pathog, 2010, 6: e1000990.
[NPL 11] Corti D et al., Science, 2011, vol. 333, pp. 850-856
[NPL 12] Ekiert DC et al., Science, 2011, vol. 333, pp. 843-850
[NPL 13] Wang TT et al., Broadly protective monoclonal antibodies against H3 influenza viruses following sequential immunization with different hemagglutinins., PLoS Pathog, 2010, 6: e1000796.
[NPL 14] Margine I et al., J Virol, 2013, vol. 87, pp. 4728-4737
[NPL 15] Clementi N et al., A human monoclonal antibody with neutralizing activity against highly divergent influenza subtypes., PLoS One, 2011, 6: e28001.
[NPL 16] Ohshima N et al., J Virol, 2011, vol. 85, pp. 11048-11057
[NPL 17] Lee PS et al., Proc Natl Acad Sci USA, 2012, vol. 109, pp. 17040-17045
[NPL 18] Yoshida R et al., Cross-protective potential of a novel monoclonal antibody directed against antigenic site B of the hemagglutinin of influenza A viruses. , PLoS Pahtog, 2009, 5: e1000350.
[NPL 19] Yasugi M et al., Human monoclonal antibodies broadly neutralizing against influenza B virus., PLoS Pathog, 2013, 9: e1003150.
[NPL 20] Dreyfus C et al., Science, 2012, vol. 337, pp. 1343-1348
[NPL 21] Ohshima N et al. , J Virol, 2011, vol. 85, pp. 11048-11057
[NPL 22] Whittle JR et al., Proc Natl Acad Sci USA, 2011, vol. 108, pp. 14216-14221
[NPL 23] Krause JG et al., J Virol, 2011, vol. 85, pp. 10905-10908
[NPL 24] Krause JG et al., J Virol, 2012, vol. 86, pp. 6334-6340
[NPL 25] Kashyap AK et al., Proc Natl Acad Sci USA, 2008, vol. 105, pp. 5986-5991
[NPL 26] Ekiert DC et al. , Nature, 2012, vol. 489, pp. 526-532

### [Summary of Invention]

### [Technical Problem]

The present invention has been made in consideration of the above-described problems of the conventional techniques. An object of the present invention is to provide an antibody having a neutralization activity against subtype H1 and subtype H5 of group 1 influenza A viruses.

### [Solution to Problem]

In order to achieve the object, the present inventors have focused on the efficacy of human antibodies against infectious diseases. To be more specific, in consideration of the case where the transfusion of patients infected with SARS-CoV and patients infected with avian influenza virus H5N1 with the sera drawn from other patients who had already recovered from these viral infections demonstrated significant treatment effects (Marasco WA, Sui J. (2007) The growth and potential of human antiviral monoclonal antibody therapeutics. Nat Biotechnol 25: 1421-1434), the inventors have made efforts to prepare human antibodies derived from patients infected with group 1 influenza A viruses. To be more specific, peripheral blood mononuclear cells (PBMCs) were prepared from the blood of patients infected with HIN1pdm, and further fused with fusion partner cells, SPYMEG, to prepare hybridomas. Then, from the prepared hybridomas, cells were selected which produced antibodies specific to influenza virus. As a result, three human-derived monoclonal antibodies (HuMAbs), H9N2, 1H11, 2H5, and 5G2 were successfully obtained. Subsequently, these antibodies were analyzed for the binding ability. The analysis revealed that the antibodies exhibited a binding ability to not only HINlpdm but also other sub-types (HIN1sea, H5N1, H9N2) belonging to group 1 influenza A viruses. On the other hand, none of the three antibodies exhibited any binding ability to H2N1 belonging to the group 1. Moreover, none of these antibodies exhibited any binding ability to sub-types (H3N2 and H7N7) belonging to group 2 influenza A viruses.

Next, in order to verify the efficacy of the obtained three HuMAbs in the treatment and prevention against influenza virus infectious disease, these antibodies were analyzed for the neutralization activity. The result revealed that all of the three HuMAbs exhibited a neutralization activity against not only HINlpdm but also HINlsea and H5N1. Particularly, it was revealed that 1H11 and 5G2 further exhibited a neutralization activity against H9N2. In addition, the three HuMAbs exhibited a neutralization activity against any of H1N1pdm strains isolated in 2009 and 2011, but the neutralization activity against the 2011 isolates was lower than that against the 2009 isolates.

Next, in order to identify the epitope for these antibodies, epitope mapping assay was conducted by protease treatment followed by mass spectrometry. The result revealed that the epitope common to all the three HuMAbs was located at a shorter α-helix (region having amino acids at residues 40 to 58) in the stalk region of hemagglutinin 2 (HA2) subunit. Moreover, importantly, it was also revealed that all the HuMAbs had activities to inhibit the cleavage of trypsin-treated HA precursor (HA0), the cleavage into mature HA1/HA2, and conformation change of HA0 by a low pH treatment.

Further, existing antibodies having a broad neutralization activity against influenza viruses cannot recognize some amino-acid substituted HA proteins, known examples of which include: an amino-acid substituted HA protein with Q42G, D46T, T49N, and N52D in the HA2 region; an amino-acid substituted HA protein with D19N, W21F, and I45V in the HA2 region; an amino-acid substituted HA protein with G189R, G225D, and T318K in the HA1 region; and the like. Hence, the three HuMAbs were analyzed for the binding ability to these amino-acid substituted HA proteins. As a result, it was found out that all of these three HuMAbs were capable of binding to any of the amino-acid substituted HA proteins. This revealed that the HuMAbs obtained this time were antibodies capable of recognizing a different epitope from those for existing antibodies having a broad neutralization activity against influenza viruses. Furthermore, the result of the genetic analysis on >6000 sequences from the NCBI influenza database revealed that the number of H1N1pdm having lysine as the amino acid at residue 47 in the HA2 region of the HA protein was increasing year after year. It was found out that the aforementioned low sensitivity to the H1N1pdm 2011 isolates was attributable to the substitution of the amino acid at residue 47 from lysine to glutamic acid in the 2009 isolates. Note that, as described above, all of the three HuMAbs exhibit an effective neutralization activity against the predominant H1N1pdm population of mutants having lysine as the amino acid at residue 47, even though the activity is lower than that against the 2009 isolates. Thus, it can also be said that these three HuMAbs are still effective against recent H1N1pdm, too.

Furthermore, regarding H2N2 which belongs to the same group 1 as H1N1pdm but to which the three HuMAbs cannot bind, in addition to the substitution of the amino acid at residue 47 from lysine to glutamic acid in the HA2 region of the HAprotein, the amino acidat residue 45 is substituted from isoleucine to phenylalanine. Hence, it was found out that these amino acid substitutions also influenced the binding ability of the three HuMAbs.

In order to confirm the influence of the amino acid substitutions on the binding ability, mutants were actually prepared by substituting the amino acids in the HA2 region of the HA protein of H1N1pdm: the amino acid at residue 45 was substituted from isoleucine to phenylalanine; the amino acid at residue 47 was substituted from glutamic acid to lysine or glycine; or the amino acid at residue 45 and the amino acid at residue 47 were substituted from isoleucine to phenylalanine and from glutamic acid to glycine, respectively. Then, the result of evaluating the three HuMAbs for the binding ability to these mutants verified that the three HuMAbs reacted with the mutants having the amino acid substitution introduced at residue 45 or 47 individually, but did not react in the case where the mutations were introduced to residues 45 and 47 at the same time. Thus, the present invention has been completed. To be more specific, the present invention relates to the following.
<1> An antibody having a neutralization activity against subtype H1 and subtype H5 of group 1 influenza A viruses and having the following features (a) and (b):
   (a) capable of binding to an epitope present at residues 40 to 58 in an HA2 region of a hemagglutinin protein derived from an A/Suita/1/200/2009 strain; and
   (b) not binding to the hemagglutinin protein derived from the A/Suita/1/200/2009 strain in the HA2 region of which an amino acid at residue 45 is substituted with phenylalanine and an amino acid at residue 47 is substituted with glycine.
<2> The antibody according to <1>, which further has any one of the following features (c) to (e):
   (c) capable of binding to the hemagglutinin protein derived from the A/Suita/1/200/2009 strain in the HA2 region of which an amino acid at residue 42 is substituted with glycine, an amino acid at residue 46 is substituted with threonine, an amino acid at residue 49 is substituted with asparagine, and an amino acid at residue 52 is substituted with aspartic acid;
   (d) capable of binding to the hemagglutinin protein derived from the A/Suita/1/200/2009 strain in the HA2 region of which an amino acid at residue 19 is substituted with asparagine, an amino acid at residue 21 is substituted with phenylalanine, and the amino acid at residue 45 is substituted with valine; and
   (e) capable of binding to the hemagglutinin protein derived from the A/Suita/1/200/2009 strain in an HA1 region of which an amino acid at residue 189 is substituted with arginine, an amino acid at residue 225 is substituted with aspartic acid, and an amino acid at residue 318 is substituted with lysine.
<3> The antibody according to claim 1 or 2, which has any one of the following features (i) to (iii):
   (i) comprising
      a light chain variable region including amino acid sequences of SEQ ID NOs: 3 to 5 or the amino acid sequences in at least any one of which one or more amino acids are substituted, deleted, added, and/or inserted, and
      a heavy chain variable region including amino acid sequences of SEQ ID NOs: 8 to 10 or the amino acid sequences in at least any one of which one or more amino acids are substituted, deleted, added, and/or inserted;
   (ii) comprising
      a light chain variable region including amino acid sequences of SEQ ID NOs: 13 to 15 or the amino acid sequences in at least any one of which one or more amino acids are substituted, deleted, added, and/or inserted, and
      a heavy chain variable region including amino acid sequences of SEQ ID NOs: 18 to 20 or the amino acid sequences in at least any one of which one or more amino acids are substituted, deleted, added, and/or inserted; and
   (iii) comprising
      a light chain variable region including amino acid sequences of SEQ ID NOs: 23 to 25 or the amino acid sequences in at least any one of which one or more amino acids are substituted, deleted, added, and/or inserted, and
      a heavy chain variable region including amino acid sequences of SEQ ID NOs: 28 to 30 or the amino acid sequences in at least any one of which one or more amino acids are substituted, deleted, added, and/or inserted.
<4> The antibody according to claim 1 or 2, which has any one of the following features (i) to (iii):
   (i) comprising
      a light chain variable region including the amino acid sequence of SEQ ID NO: 2 or the amino acid sequence in which one or more amino acids are substituted, deleted, added, and/or inserted, and
      a heavy chain variable region including the amino acid sequence of SEQ ID NO: 7 or the amino acid sequence in which one or more amino acids are substituted, deleted, added, and/or inserted;
   (ii) comprising
      a light chain variable region including the amino acid sequence of SEQ ID NO: 12 or the amino acid sequence in which one or more amino acids are substituted, deleted, added, and/or inserted, and
      a heavy chain variable region including the amino acid sequence of SEQ ID NO: 17 or the amino acid sequence in which one or more amino acids are substituted, deleted, added, and/or inserted; and
   (iii) comprising
      a light chain variable region including the amino acid sequence of SEQ ID NO: 22 or the amino acid sequence in which one or more amino acids are substituted, deleted, added, and/or inserted, and
      a heavy chain variable region including the amino acid sequence of SEQ ID NO: 27 or the amino acid sequence in which one or more amino acids are substituted, deleted, added, and/or inserted.
<5> The antibody according to any one of <1> to <4>, which is an antibody further having a neutralization activity against subtype H9 of group 1 influenza A viruses.
<6> The antibody according to anyone of <1> to <5>, wherein a concentration of the antibody required to neutralize the group 1 influenza A viruses to 50% is 30 µg/ml or less.
<7> The antibody according to <1> to <6>, which further has the following feature (f):
   (f) capable of binding to the hemagglutinin protein derived from the A/Suita/1/200/2009 strain in the HA2 region of which the amino acid at residue 47 is substituted with lysine.
<8> The antibody according to <1> to <7>, which further has the following feature (g):
   (g) not binding to the hemagglutinin protein derived from the A/Suita/1/200/2009 strain in the HA1 region of which an amino acid at residue 86 is substituted with cysteine.
<9> The antibody according to any one of <1> to <8>, which is a human antibody.
<10> A DNA encoding the antibody according to any one of <1> to <9>.
<11> A cell which produces the antibody according to any one of <1> to <9>, or comprises the DNA according to <10>.
<12> A method for producing an antibody, comprising the steps of:
   culturing the cell according to <11>; and
   collecting the antibody according to any one of <1> to <9> produced in the cell or from a culture fluid thus obtained.
<13> A pharmaceutical composition comprising the antibody according to any one of <1> to <9> as an active ingredient.

### [Advantageous Effects of Invention]

The present invention makes it possible to provide an antibody having a neutralization activity against subtype H1 and subtype H5 of group 1 influenza A viruses. To be more specific, the present invention makes it possible to provide an antibody capable of exhibiting a strong neutralization activity against not only seasonal influenza H1N1sea but also H1N1pdm having induced pandemic and H5N1 expected to induce pandemic in the future. Further, the present invention makes it possible to provide an antibody capable of exhibiting a neutralization activity also against a recent predominant H1N1pdm population of mutants having lysine as the amino acid at residue 47 of the HA2 protein.

### [Brief Description of Drawings]

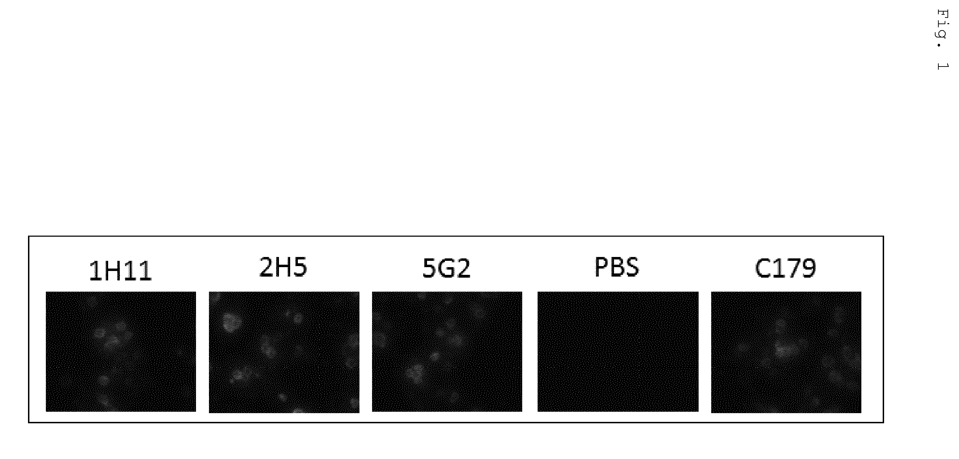

[Fig. 1] Fig. 1 shows micrographs for illustrating the result of immunofluorescence assay (IFA) of 293T cells transfected with an HA coding plasmid and three antibodies against influenza A viruses. To be more specific, the 293T cells, which were transfected with the plasmid encoding wild-type (A/Suita/1/2009-derived) hemagglutinin (HA) protein, were reacted with the three antibodies of the present invention (1H11, 2H5, and 5G2). The result is shown in the micrographs. Note that, in place of the antibodies, only PBS was added to and C179 MAb were reacted with the same cells for use as a negative control and a positive control, respectively.

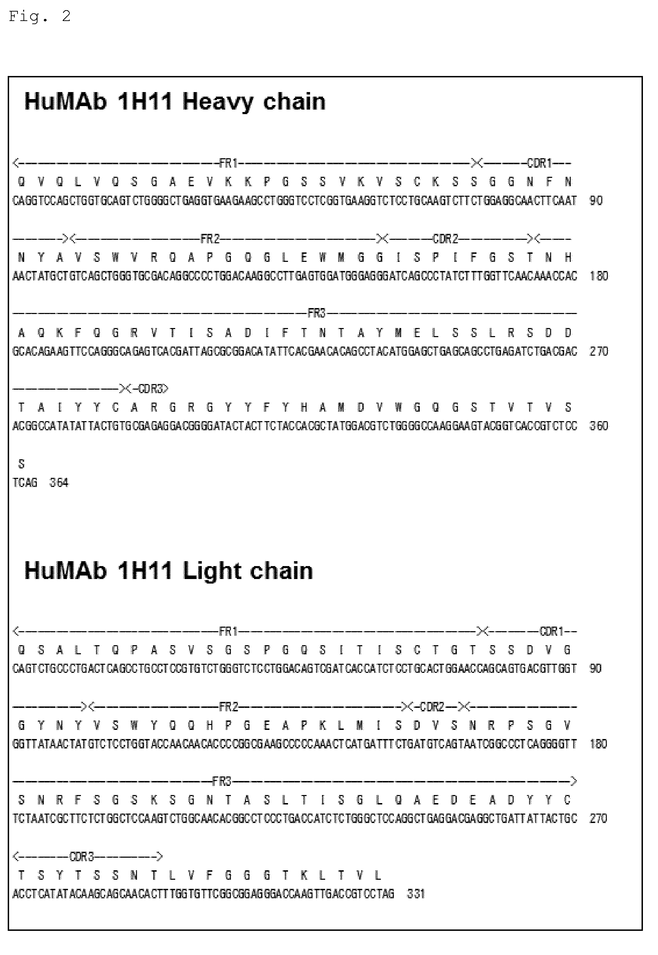

[Fig. 2] Fig. 2 is a figure showing the deduced amino acid sequences of the heavy chain variable region (VH) and the light chain variable region (VL) of the human monoclonal antibody (HuMAb) 1H11 of the present invention, and the deduced amino acid sequences of complementarity determining regions (CDRs) in each of the regions.

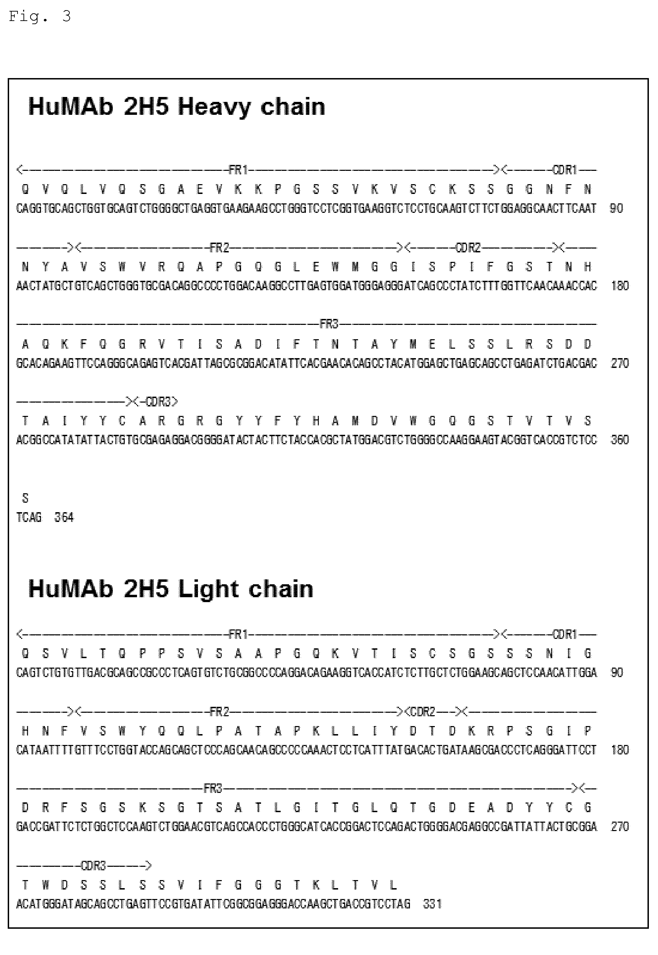

[Fig. 3] Fig. 3 is a figure showing the deduced amino acid sequences of the heavy chain variable region (VH) and the light chain variable region (VL) of the human monoclonal antibody (HuMAb) 2H5 of the present invention, and the deduced amino acid sequences of complementarity determining regions (CDRs) in each of the regions.

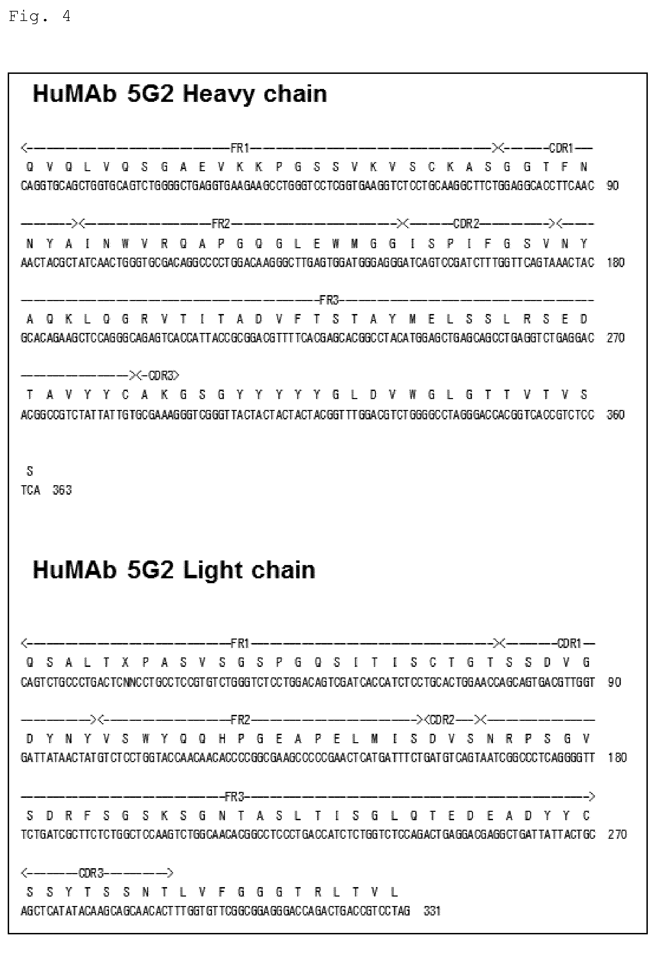

[Fig. 4] Fig. 4 is a figure showing the deduced amino acid sequences of the heavy chain variable region (VH) and the light chain variable region (VL) of the human monoclonal antibody (HuMAb) 5G2 of the present invention, and the deduced amino acid sequences of complementarity determining regions (CDRs) in each of the regions.

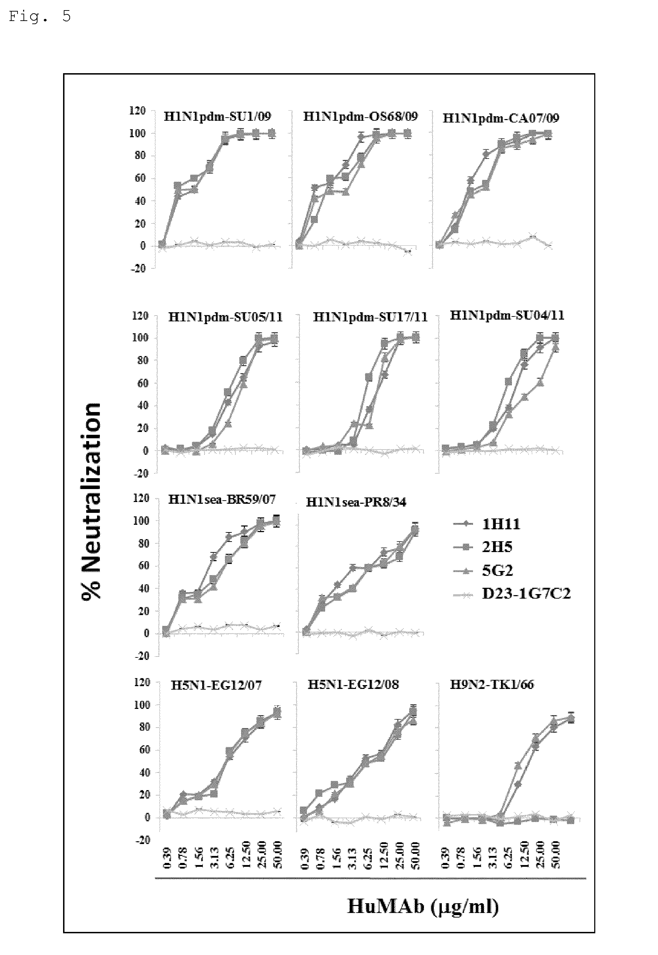

[Fig. 5] Fig. 5 shows graphs for illustrating the virus neutralization activities of the three HuMAbs of the present invention against H1N1pdm, H1N1sea, H5N1, and H9N2. To be more specific, H1N1pdm (2009 isolates and 2011 isolates), two isolates of H1N1sea, two isolates of H5N1, and one isolate of H9N2 were subjected to neutralization with serial dilutions of the HuMAbs. The result is shown in the graphs. Note that, as a negative control, an anti-dengue virus HuMAb D23-1G7C2 was used.

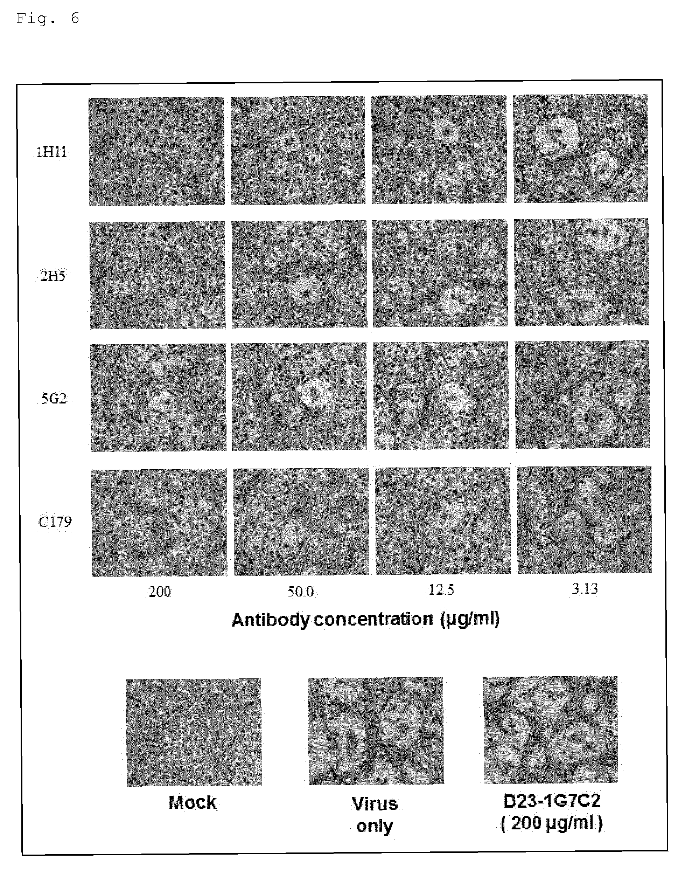

[Fig. 6] Fig. 6 shows micrographs for illustrating the result of fusion inhibition assay using CV-1 cells. To be more specific, the CV-1 cells were infected with A/Suita/1/2009 and incubated for 24 hours. The cells were further incubated at 37°C for 1 hour together with various concentrations of the three antibodies (1H11, 2H5, and 5G2) of the present invention shown in the figure. Then, the medium was replaced with PBS (pH 5.5) followed by incubation for 5 minutes. After washing, the cells were further incubated for 3 hours, and then fixed with methanol, followed by staining with Giemsa. The observation result is shown in the micrographs. As a control, C179 MAb was used in place of the anti-influenza virus antibodies HuMAbs. In addition, mock-infected CV-1 cells but not treated with the HuMAbs was used (Mock/-). Moreover, the CV-1 cells infected A/Suita/1/2009 but not treated with the HuMAbs (virus only), or CV-1 cells infected with A/Suita/1/2009 and treated with 200 µg/ml of a control HuMAb against dengue virus (D23-1G7C2 (200 µg/ml)) were also used.

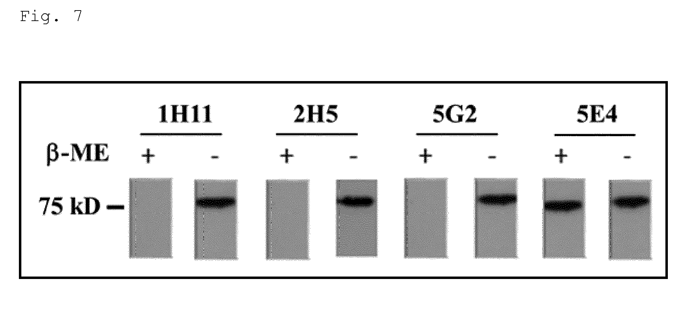

[Fig. 7] Fig. 7 shows photographs for illustrating the result of western blotting using the three HuMAbs of the present invention against an HA protein in a reducing (-ME+) or non-reducing state (β-ME-). To be more specific, MDCK cells infected with A/Suita/1/2009 were subjected to SDS-PAGE in the presence or absence of β-mercaptoethanol (β-ME). Then, the gels after the SDS-PAGE were subjected to western blotting using 1H11, 2H5, 5G2, or control 5E4. The result is shown in the photographs.

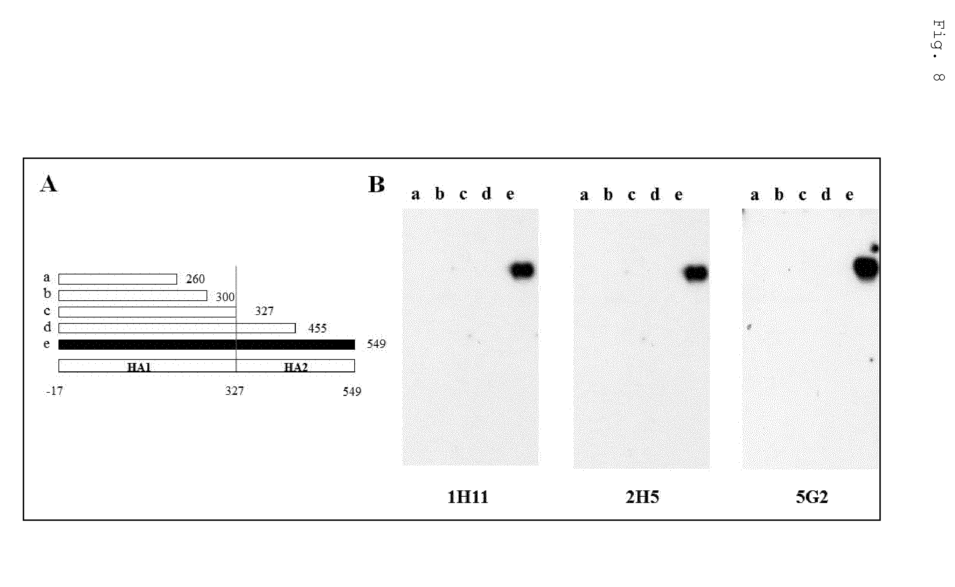

[Fig. 8] Fig. 8 shows photographs (B in the figure) for illustrating the result of western blotting to analyze the reactivity of the present invention three HuMAbs with constructions (A in the figure) of deletion variants (a to e) of H1N1pdm-derived HA protein.

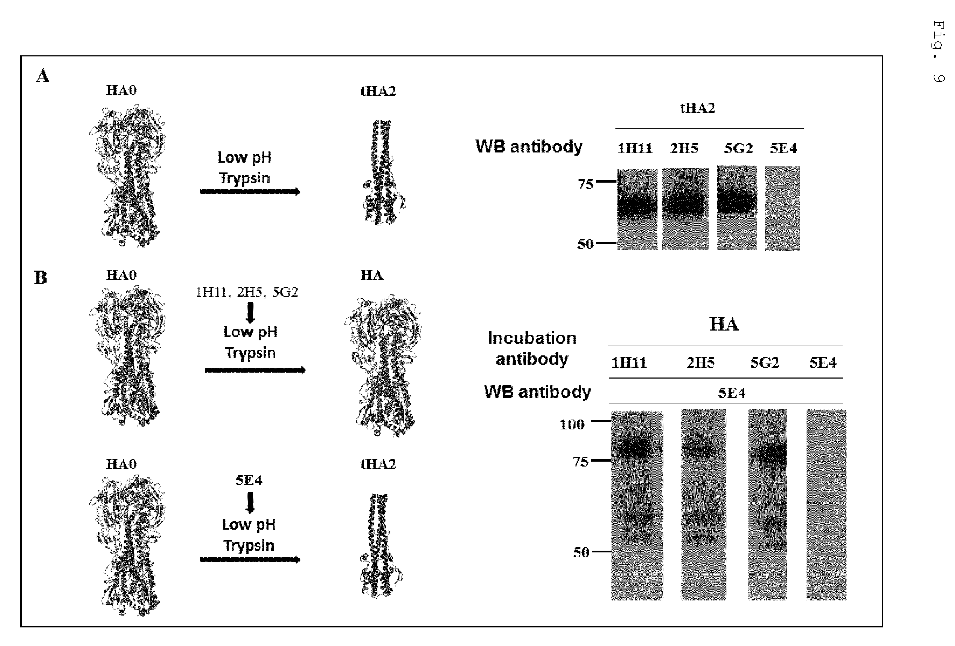

[Fig. 9] Fig. 9 is a figure for illustrating the result of western blotting of H1N1pdm HA0 protein treated with trypsin at low pH. In the figure, A shows schematic drawings and photographs for illustrating the result of treating the HA0 protein with trypsin at low pH, followed by western blotting with the three HuMAbs (1H11, 2H5, and 5G2). Note that 5E4, anti-H1N1pdm-derived HA0-protein globular head recognizing antibody, was used as a negative control. In the figure, B shows schematic drawings and photographs for illustrating the result of treating the HA0 protein first with 5E4 (upper) or 1H11, 2H5, or 5G2 (lower), then with trypsin at low pH, followed by western blotting using 5E4.

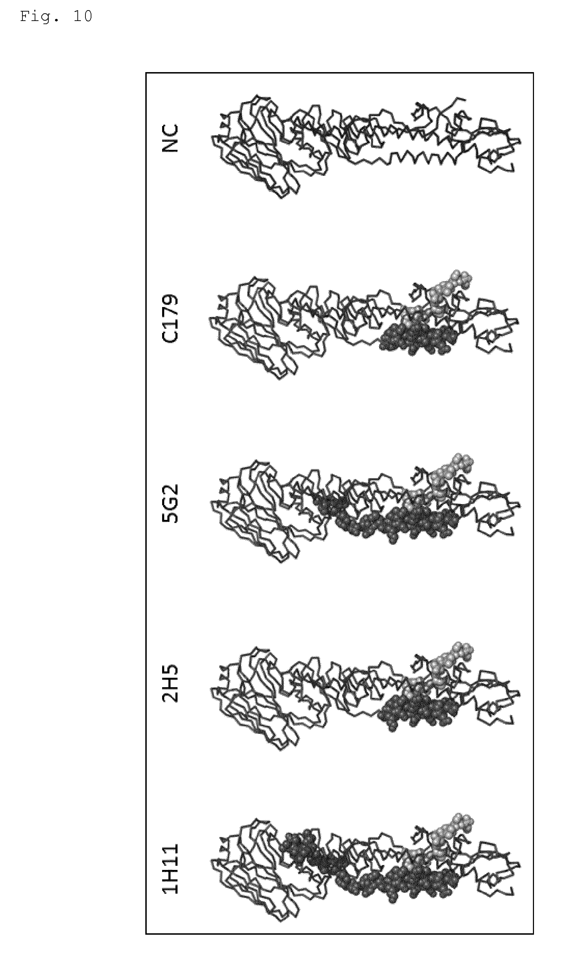

[Fig. 10] Fig. 10 shows drawings each for illustrating the result of mapping, on a HA-monomer structural model, the results of: trypsin-digesting HA proteins bound on columns, to which the three HuMAbs (1H11, 2H5, and 5G2) had been respectively immobilized; and analyzing, by mass spectrometry, peptide fragments having interacting with the antibody. In the drawing, two regions with high scores are indicated by balls. Note that the murine monoclonal antibody C179 known to interact with the stalk region of HA protein was used as a control.

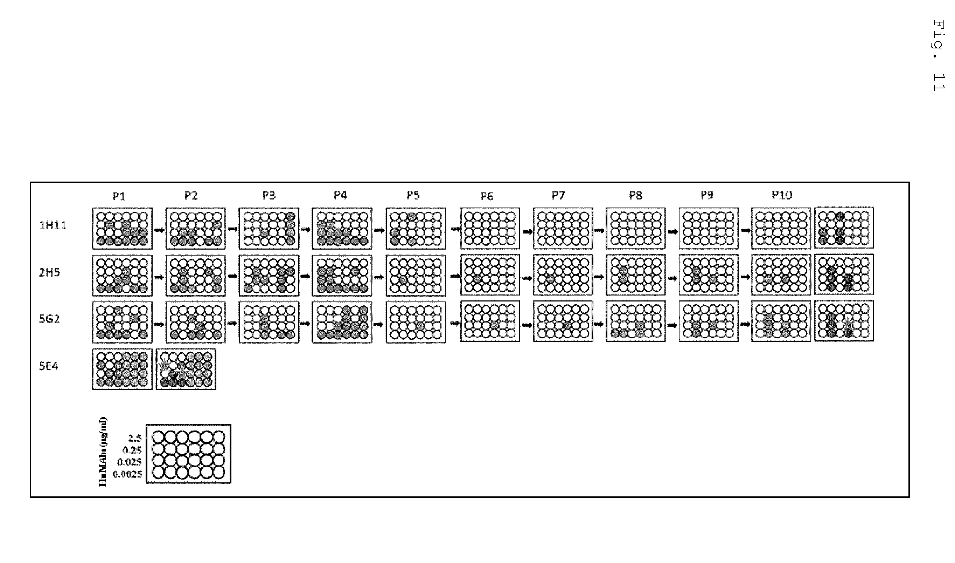

[Fig. 11] Fig. 11 is a schematic drawing showing the presence or absence of escape mutant variant appearing by serial ten passages of MDCK cells in the presence of anti-influenzaAvirus. To be more specif ic, the schematic drawing shows serial ten passages of A/Suita/1/2009 inMDCK cells in the presence of 1H11, 2H5, or 5G2 in various concentrations (0.0025, 0.025, 0.25, and 2.5 µg/ml). Moreover, as a control, A/Suita/1/2009 was incubated once together with the MDCK cells in the presence of 5E4. Then, in the plates P1 to P10, wells showing cytopathic effects were highlighted. In addition, in the rightmost plates P10 (in the case of 5E4, the plate P1), the highlighted wells were subjected to genome sequencing analysis. As a result of the sequencing, wells from which a mutant was identified were denoted by stars (mutants were identified from one well in the 5G2 plate and two wells in the 5E4 plate, and accordingly denoted by stars, respectively, in the figure).

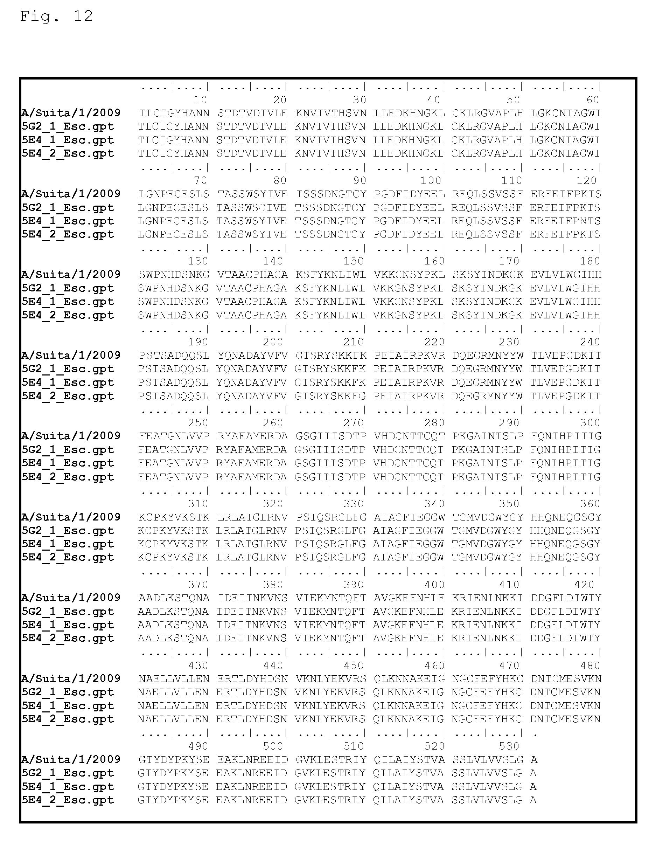

[Fig. 12] Fig. 12 is a figure showing the amino acid sequences of HA protein mutants of A/Suita/1/2009 detected in the MDCK-cell passages in the presence of the anti-influenza A virus shown in Fig. 11. In the figure, "5G2_1_Esc.gpt" shows the result of the escape mutant detected in the presence of 5G2, while "5E4_1_Esc.gpt" and "5E4_2_Esc.gpt" show the results of two escape mutants detected in the presence of 5E4.

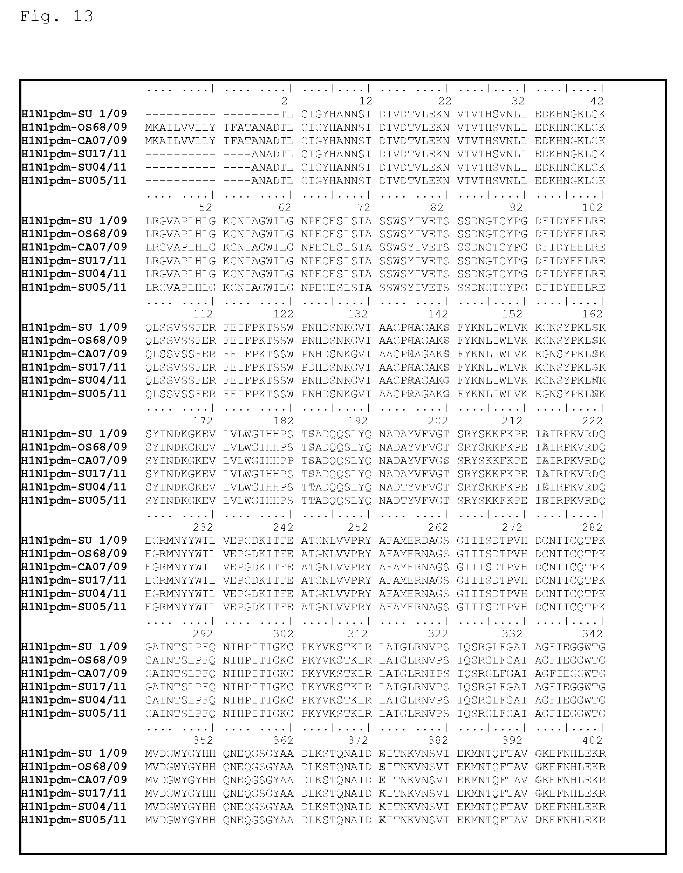

[Fig. 13] Fig. 13 is a figure for illustrating the result of comparing the amino acid sequences of the HA protein until residue 402 between the H1N1pdm 2009 isolates and the H1N1pdm 2011 isolates.

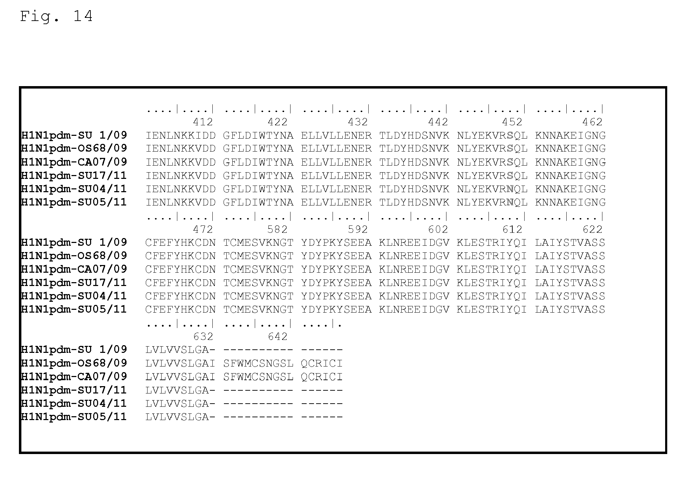

[Fig. 14] Fig. 14 is a figure for illustrating the result of comparing the amino acid sequences of the HA protein from residue 403 between the H1N1pdm 2009 isolates and the H1N1pdm 2011 isolates.

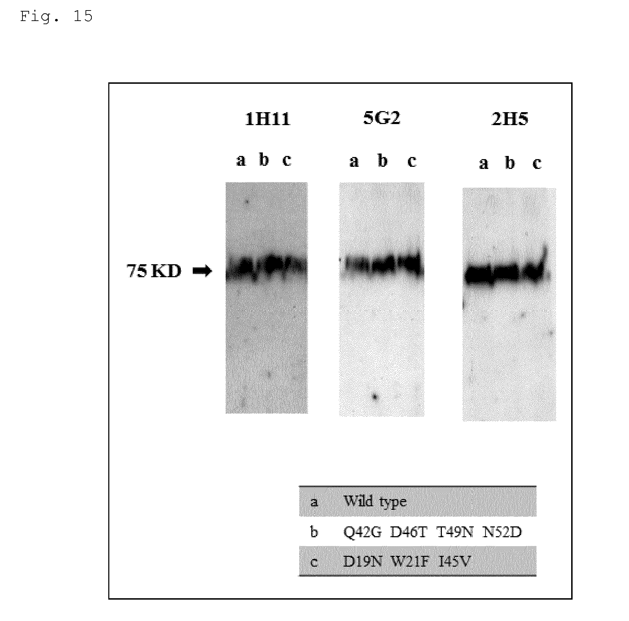

[Fig. 15] Fig. 15 shows photographs for illustrating the result of western blotting to analyze the reactivity between the three HuMAbs of the present invention and amino-acid substituted HA proteins (an amino-acid substituted HA protein with Q42G, D46T, T49N, and N52D in the HA2 region, an amino-acid substituted HA protein with D19N, W21F, and I45V in the HA2 region).

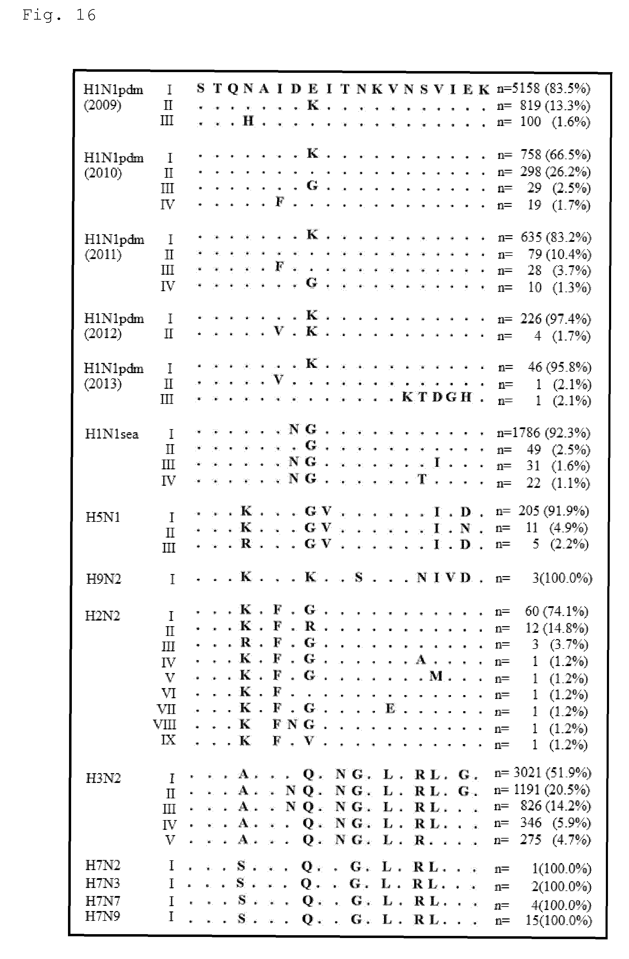

[Fig. 16] Fig. 16 is a figure for illustrating variation at the amino acid residues 40 to 58 in the HA2 region of the HA protein among sub-types of influenza A viruses.

### [Description of Embodiments]

### <Antibody Having Broad Neutralization Activity against Group 1 Influenza A Viruses>

As described later in Examples, the present inventors have obtained three human monoclonal antibodies (1H11, 2H5, and 5G2) capable of binding to an epitope present at residues 40 to 58 in an HA2 region of a hemagglutinin protein derived from H1N1pdm (A/Suita/1/200/2009 strain). Further, these antibodies have been found to also have a neutralization activity against subtype H1 and subtype H5 of group 1 influenza A viruses. On the other hand, the three antibodies have also been found to exhibit neither a binding ability nor a neutralization activity against subtype H2 which belongs to the group 1, but in the HA2 region of the hemagglutinin protein derived from H1N1pdm (A/Suita/1/200/2009 strain) of which the amino acid at residue 45 is substituted with phenylalanine and the amino acid at residue 47 is substituted with glycine.

Thus, the present invention provides the following antibody having a broad neutralization activity against group 1 influenza A viruses.

An antibody having a neutralization activity against subtype H1 and subtype H5 of group 1 influenza A viruses and having the following features (a) and (b):
(a) capable of binding to an epitope present at residues 40 to 58 in an HA2 region of a hemagglutinin protein derived from an A/Suita/1/200/2009 strain; and
(b) not binding to the hemagglutinin protein derived from the A/Suita/1/200/2009 strain in the HA2 region of which an amino acid at residue 45 is substituted with phenylalanine and an amino acid at residue 47 is substituted with glycine.

Meanwhile, existing antibodies having a broad neutralization activity against influenza viruses cannot recognize some amino-acid substituted hemagglutinin (HA) proteins, known examples of which include: an amino-acid substituted HA protein with Q42G, D46T, T49N, and N52D in the HA2 region; an amino-acid substituted HA protein with D19N, W21F, and I45V in the HA2 region; an amino-acid substituted HA protein with G189R, G225D, and T318K in the HA1 region; and the like. With respect to these amino-acid substituted HA proteins, the antibody of the present invention is capable of binding to any of the amino-acid substituted HA proteins as described later in Examples. Thus, a more preferable embodiment of the antibody of the present invention includes the following antibody.

The antibody of the present invention, which further has any one of the following features (c) to (e):
(c) capable of binding to the hemagglutinin protein derived from the A/Suita/1/200/2009 strain in the HA2 region of which an amino acid at residue 42 is substituted with glycine, an amino acid at residue 46 is substituted with threonine, an amino acid at residue 49 is substituted with asparagine, and an amino acid at residue 52 is substituted with aspartic acid;
(d) capable of binding to the hemagglutinin protein derived from the A/Suita/1/200/2009 strain in the HA2 region of which an amino acid at residue 19 is substituted with asparagine, an amino acid at residue 21 is substituted with phenylalanine, and the amino acid at residue 45 is substituted with valine; and
(e) capable of binding to a protein in an HA1 region of the hemagglutinin protein derived from the A/Suita/1/200/2009 strain in which an amino acid at residue 189 is substituted with arginine, an amino acid at residue 225 is substituted with aspartic acid, and an amino acid at residue 318 is substituted with lysine.

Note that, regarding (e), the antibody of the present invention is capable of binding to a hemagglutinin protein derived from an A/Suita/1/89(R) (H1-SU1/89R) strain as a further preferable embodiment (regarding the hemagglutinin protein derived from the A/Suita/1/89(R) strain, see Examples to be described later).

Moreover, as described later in Examples, the antibody having the feature (e) is capable of binding also to the H1-SU1/89R strain, which an existing broad neutralizing antibody C179 cannot suppress, and consequently exhibiting a neutralization activity against the strain.

Further, the antibody of the present invention more preferably further has all the features (c) to (e).

In addition, as described later in Examples, the antibody of the present invention exhibits an effective neutralization activity also against a recent predominant H1N1pdm population of virus strains having lysine as the amino acid at residue 47 in the HA2 region of the hemagglutinin protein as a result of the genetic analysis on >6000 sequences from the NCBI influenza database. Thus, a more preferable embodiment of the antibody of the present invention includes the following antibody.

The antibody of the present invention, which further has the following feature (f):
(f) capable of binding to the hemagglutinin protein derived from the A/Suita/1/200/2009 strain in the HA2 region of which the amino acid at residue 47 is substituted with lysine.

Moreover, as described later in Examples, an escape mutation, in which the amino acid at residue 86 in the HA1 region of the hemagglutinin protein is substituted with cysteine, has been identified from the influenza virus in a well of the tenth passage cultured in the presence of 5G2. This suggests that the antibody of the present invention, at least 5G2 to be described later, is incapable of binding to such a mutant. Thus, the following embodiment is also possible.

The antibody of the present invention, which further has the following feature (g):
(g) not binding to the hemagglutinin protein derived from the A/Suita/1/200/2009 strain in the HA1 region of which an amino acid at residue 86 is substituted with cysteine.

The "group 1 influenza A viruses" against which the antibody of the present invention exhibits a neutralization activity means one group (group 1) among viruses classified into *Influenza A virus* of orthomyxoviridae. In addition, the influenza virus targeted by the antibody of the present invention includes not only seasonal (sea) influenza viruses, but also pandemic (pdm) influenza viruses whose scales are worldwide and pandemic regardless of the season.

Moreover, the group 1 is further classified into subtypes based on the difference in the hemagglutinin protein structure to be described later. The subtypes belonging to the group 1 are: H1, H2, H5, H6, H8, H9, H11, H12, H13, and H16. The antibody of the present invention targets at least H1 and H5 as described above. Additionally, as described later in Examples, antibodies capable of exhibiting a neutralization activity also against H9 by exhibiting the above-described binding ability are also obtained in the present invention. Thus, a more preferable embodiment of the antibody of the present invention includes an antibody further having a neutralization activity against subtype H9 of group 1 influenza A viruses.

Moreover, these subtypes were further classified into sub-types depending on the type of neuraminidase produced by the virus. Examples of such sub-types include H1N1, H1N2, H1N3, H1N4, H1N5, H1N6, H1N7, H1N8, H1N9, H5N1, H5N2, H5N3, H5N4, H5N5, H5N6, H5N7, H5N8, H5N9, H9N1, H9N2, H9N3, H9N4, H9N5, H9N6, H9N7, H9N8, and H9N9. Among these, H1N1, H5N1, and H9N2 can be suitable targets of the antibody of the present invention.

In addition, those subtypes are further classified into strains (isolates) depending on the site of isolation, the order of isolation, and the year of isolation. Such isolates are not particularly limited, as long as they belong to H1, H5, or H9. Suitable targets of the antibody of the present invention may be A/Suita/1/2009 (H1N1pdm-SU1/09), A/Osaka/168/2009 (H1N1pdm-OS68/09), A/California/07/2009 (H1N1pdm-07/09), A/Suita/117/2011 (H1N1pdm-SU17/11), A/Suita/104/2011 (H1N1pdm-SU04/11), A/Suita/105/2011 (H1N1pdm-SU05/11), A/Brisbane/59/2007 (HlNlsea-BR59/07), A/PR/8/34 (H1N1sea-PR8/34), A/Duck/Egypt/DIBr12/2007 (H5N1-CE12/07), A/Chicken/Egypt/PIMD12-3/2008 (H5N1-CE12/08), and A/Turkey/Wisconsin/1/1966 (H9N2-TW1/66).

In the present invention, the term "neutralization activity" means an activity to suppress infection (invasion) of the influenza virus into host cells and/or propagation of the influenza virus in the cells. Moreover, the "suppress" and related terms include not only complete suppression (inhibition) but also partial suppression. Further, the "suppress" and related terms also include not only reduction, but also prevention, inhibition, and delay in influenza virus propagation and the like. Meanwhile, whether or not an antibody has a neutralization activity against an influenza virus can be determined by known methods. An example of such methods includes VN assay as described later in Examples. Additionally, in this assay, if the antibody concentration required to neutralize an influenza virus to 50% (minimum concentration of the antibody required, for example, to inhibit influenza virus propagation to 50%), that is, VN50, is normally less than 100 µg/ml, it is possible to determine that the antibody has a neutralization activity. In the present invention, the antibody concentration is preferably 30 µg/ml or less, more preferably 15 µg/ml or less, furthermore preferably 7 µg/ml or less, still furthermore preferably 3 µg/ml or less, and particularly preferably 1 µg/ml or less.

Moreover, in the present invention, the VN50 against 2009 isolates of H1N1pdm is preferably 3 µg/ml or less, more preferably 2 µg/ml or less, and particularly preferably 1 µg/ml or less. In addition, in the present invention, the VN50 against H5N1 is preferably 15 µg/ml or less, more preferably 7 µg/ml or less.

In the present invention, the term "antibody" includes all classes and subclasses of immunoglobulins. An"antibody"includesa polyclonalantibody and monoclonal antibody, and is also meant to include the form of a functional fragment of an antibody. A "polyclonal antibody" is an antibody preparation including different antibodies against different epitopes. A "monoclonal antibody" means an antibody (including an antibody fragment) obtained from a substantially uniform antibody population, and capable of recognizing a single determinant on an antigen. The antibody of the present invention is preferably a monoclonal antibody. Moreover, the antibody of the present invention is an antibody separated and/or collected (i.e., isolated) from components in a natural environment.

The "HA protein derived from an A/Suita/1/200/2009 strain" to which the antibody of the present invention binds means a hemagglutinin (HA) protein derived from a strain of H1N1pdm belonging to group 1 influenza A viruses targeting human as a host, the strain being the 200th strain isolated in 2009 in Suita City, Osaka, Japan. The hemagglutinin is a protein for influenza viruses to infect host cells by binding to sialic acid on the cell surface thereof. Three identical monomers constituting HA have an α-helix at the central portion, and head regions thereof contain sialic acid binding sites. Moreover, the HA monomers are first synthesized as precursors (HA0) containing an HA1 region and an HA2 region. Then, the precursors are glycosylated and cleaved into two subunits of an HA1 subunit and an HA2 subunit.

Note that, in the present invention, the amino acid numbers of the HA1 region or the HA2 region of the hemagglutinin protein are numbered, unless otherwise specifically stated, based on the description of Nobusawa E, Aoyama T, Kato H, Suzuki Y, Tateno Y, et al. (1991) Comparison of complete amino acid sequences and receptor-binding properties among 13 serotypes of hemagglutinins of influenza A viruses. Virology 182: 475-485. To be more specific, amino acids are numbered based on the HA1 region or the HA2 region of a hemagglutinin protein derived from influenza virus subtype H3 (A/Aichi/2/68 strain).

The hemagglutinin protein derived from the A/Suita/1/200/2009 strain according to the present invention is typically a protein having an amino acid sequence of SEQ ID NO: 32 (protein having an amino acid sequence encoded by a nucleotide sequence of SEQ ID NO: 31). In the hemagglutinin protein (the amino acid sequence of SEQ ID NO: 32), the HA1 region is a region having an amino acid sequence of residues 1 to 326 (region having an amino acid sequence of SEQ ID NO: 33), and the HA2 region is a region having an amino acid sequence of residues 327 to 531 (region having an amino acid sequence of SEQ ID NO: 34).

Thus, the residues 86, 189, 225, and 318 in the HA1 region according to the present invention correspond respectively to residues 77, 185, 221, and 315 in the amino acid sequence of SEQ ID NO: 33. Moreover, the residues 19, 21, 40, 42, 45, 46, 47, 49, 52, and 58 in the HA2 region according to the present invention correspond respectively to residues 19, 21, 40, 42, 45, 46, 47, 49, 52, and 58 in the amino acid sequence of SEQ ID NO: 34.

Note that those skilled in the art can evaluate whether or not an antibody is the above-described antibody capable of binding to the epitope present at residues 40 to 58 in the HA2 region, or the antibody capable of binding or not binding to the amino-acid substituted HA proteins described in (b) to (f) above by utilizing, as described later in Examples, protease treatment followed by mass spectrometry, western blotting in a reducing condition or non-reducing condition, immunofluorescence assay (IFA), or trypsin cleavage inhibition assay. Moreover, in addition to these methods described later in Examples, the evaluation is also possible by utilizing a known immunological analysis method (such as flow cytometry, ELISA, immunoprecipitation). Further, those skilled in the art can prepare the hemagglutinin protein derived from the A/Suita/1/200/2009 strain used in the assay, without obtaining the strain as described later in Examples, by constructing a vector encoding the protein based on known amino acid sequence information on the HA protein derived from the A/Suita/1/200/2009 strain, introducing the vector into cells, and expressing the protein as appropriate. Furthermore, those skilled in the art can prepare the amino-acid substituted HA proteins described in (b) to (f) by utilizingsite-directed mutagenesisbased on known amino acid sequence information on the HA protein derived from the A/Suita/1/200/2009 strain as described later in Examples.

Moreover, a site containing the amino acids to which the antibody of the present invention binds, that is, "epitope", means an antigen determinant present at residues 40 to 58 in the HA2 region (a site on an antigen where an antigen-binding domain in the antibody binds). Thus, in the present invention, the epitope may be a polypeptide (linear epitope) having several consecutive amino acids in a primary sequence of amino acids, or may be a polypeptide (discontinuous epitope, conformational epitope) formed of amino acids which are not next to each other in a primary sequence of amino acids, but which come near each other in a three-dimensional conformation by folding or the like of a peptide or protein.

Further, as described later in Examples, the antibody of the present invention is capable of recognizing an α-helix structure having 40 to 58 amino acids in the HA2 region and constituting the stalk region of the hemagglutinin protein. Thus, the antibody of the present invention is preferably an antibody capable of binding in a manner dependent on the higher-order structure of the epitope (α-helix structure having 40 to 58 amino acids in the HA2 region), more preferably an antibody capable of binding to the epitope in the hemagglutinin protein in a non-reducing state , and furthermore preferably an antibody capable of binding to the epitope in the hemagglutinin protein in the absence of β-mercaptoethanol.

Note that amino acids are herein referred to by one-letter code or three-letter code or both as, for example, Ala/A, Leu/L, Arg/R, Lys/K, Asn/N, Met/M, Asp/D, Phe/F, Cys/C, Pro/P, Gln/Q, Ser/S, Glu/E, Thr/T, Gly/G, Trp/W, His/H, Tyr/Y, Ile/I, or Val/V. Note that amino acids contained in the amino acid sequence described in the present invention may be subjected to a modification (for example, glycosylation, phosphorylation, ubiquitination, SUMOylation, palmitoylation, prenylation, methylation, acetylation, hydroxylation, amidation) after the translation. Even when the amino acids are modified after the translation in this manner, the amino acid sequence described in the present invention comprises these modified amino acids as a matter of course.

Another preferable embodiment of the antibody of the present invention includes an antibody, which has any one of the following features (i) to (iii):
(i) comprising
   a light chain variable region including amino acid sequences of SEQ ID NOs : 3 to 5 (CDRs 1 to 3 of a light chain variable region of 1H11 to be described later) or the amino acid sequences in at least any one of which one or more amino acids are substituted, deleted, added, and/or inserted, and
   a heavy chain variable region including amino acid sequences of SEQ ID NOs: 8 to 10 (CDRs 1 to 3 of a heavy chain variable region of 1H11 to be described later) or the amino acid sequences in at least any one of which one or more amino acids are substituted, deleted, added, and/or inserted;
(ii) comprising
   a light chain variable region including amino acid sequences of SEQ ID NOs: 13 to 15 (CDRs 1 to 3 of a light chain variable region of 5G2 to be described later) or the amino acid sequences in at least any one of which one or more amino acids are substituted, deleted, added, and/or inserted, and
   a heavy chain variable region including amino acid sequences of SEQ ID NOs: 18 to 20 (CDRs 1 to 3 of a heavy chain variable region of 5G2 to be described later) or the amino acid sequences in at least any one of which one or more amino acids are substituted, deleted, added, and/or inserted; and
(iii) comprising
   a light chain variable region including amino acid sequences of SEQ ID NOs: 23 to 25 (CDRs 1 to 3 of a light chain variable region of 2H5 to be described later) or the amino acid sequences in at least any one of which one or more amino acids are substituted, deleted, added, and/or inserted, and
   a heavy chain variable region including amino acid sequences of SEQ ID NOs: 28 to 30 (CDRs 1 to 3 of a heavy chain variable region of 2H5 to be described later) or the amino acid sequences in at least any one of which one or more amino acids are substituted, deleted, added, and/or inserted.

Moreover, a more preferable embodiment of the antibody of the present invention includes an antibody, which has any one of the following features (i) to (iii) :
(i) comprising
   a light chain variable region including the amino acid sequence of SEQ ID NO: 2 (the light chain variable region of 1H11 to be described later) or the amino acid sequence in which one or more amino acids are substituted, deleted, added, and/or inserted, and
   a heavy chain variable region including the amino acid sequence of SEQ ID NO: 7 (the heavy chain variable region of 1H11 to be described later) or the amino acid sequence in which one or more amino acids are substituted, deleted, added, and/or inserted;
(ii) comprising
   a light chain variable region including the amino acid sequence of SEQ ID NO: 12 (the light chain variable region of 5G2 to be described later) or the amino acid sequence in which one or more amino acids are substituted, deleted, added, and/or inserted, and
   a heavy chain variable region including the amino acid sequence of SEQ ID NO : 17 (the heavy chain variable region of 5G2 to be described later) or the amino acid sequence in which one or more amino acids are substituted, deleted, added, and/or inserted; and
(iii) comprising
   a light chain variable region including the amino acid sequence of SEQ IDNO: 22 (the light chain variable region of 2H5 to be described later) or the amino acid sequence in which one or more amino acids are substituted, deleted, added, and/or inserted, and
   a heavy chain variable region including the amino acid sequence of SEQ ID NO: 27 (the heavy chain variable region of 2H5 to be described later) or the amino acid sequence in which one or more amino acids are substituted, deleted, added, and/or inserted.

In addition, among the above-described antibodies comprising the particular amino acid sequences, the antibody having the feature (i) and the antibody having the feature (ii) are more preferable from the viewpoint of further having a neutralization activity against subtype H9 of group 1 influenza A viruses as described later in Examples. Further, the antibody having the feature (i) and the antibody having the feature (iii) are more preferable from the viewpoint that the antibodies at low concentration are capable of inhibiting fusion between cells infected with group 1 influenza A viruses. Furthermore, in addition to these viewpoints, from the viewpoint of strongly suppressing the occurrence of escape mutation in group 1 influenza A viruses, the antibody having the feature (i) is particularly preferable.

The antibody of the present invention includes a humanized antibody, a human antibody, a murine antibody, a chimeric antibody, and a functional fragment of these antibodies. For administration as a pharmaceutical agent to human, the antibody of the present invention is desirably a chimeric antibody, a humanized antibody, or a human antibody from the viewpoint of side effect reduction. Furthermore, a human antibody is particularly desirable from the viewpoints of long-term stability in serum, high specificity, and low immunogenicity.

In the present invention, a "human antibody" is an antibody all regions of which are derived from human. As described later in Examples, in preparing a human antibody, it is possible to utilize a method in which peripheral blood mononuclear cells (PBMCs) derived from human, for example, a human having an infectious disease of group 1 influenza A virus (for example, a patient and/or a vaccinated person), are fused with fusion partner cells capable of efficient cell fusion to thereby prepare a hybridoma, and an anti-human influenza virus monoclonal antibody is obtained from the hybridoma (see, for example, WO2007/119808) It is also possible to utilize a screening method for a production of an antibody having a higher activity than human B cells, a phage display method, a transgenic animal (for example, a mouse) capable of producing a repertoire of the human antibody by immunization, or other means. Preparation methods for a human antibody are known (see, for example, Nature, 362: 255-258 (1993), Intern. Rev. Immunol, 13: 65-93 (1995), J. Mol. Biol, 222: 581-597 (1991), Nature Genetics, 15: 146-156 (1997), Proc. Natl. Acad. Sci. USA, 97: 722-727 (2000), Japanese Unexamined Patent Application Publication Nos. Hei 10-146194 and Hei 10-155492, Japanese Patent No. 2938569, Japanese Unexamined Patent Application Publication No. Hei 11-206387, International Application Japanese-Phase Publication Nos. Hei 8-509612 and Hei 11-505107).

In the present invention, a "chimeric antibody" is an antibody obtained by linking a variable region of an antibody of one species to a constant region of an antibody of another species. A chimeric antibody can be obtained as follows, for example. Concretely, a mouse is immunized with an antigen. A portion corresponding to an antibody variable part (variable region) which binds to the antigen is cut out from a gene of a monoclonal antibody of the mouse. The portion is linked to a gene of a constant part (constant region) of an antibody derived from human bone marrow. This is incorporated into an expression vector, which is then introduced into a host for the production of a chimeric antibody (see, for example, Japanese Unexamined Patent Application Publication No. Hei 8-280387, U.S. Pat. Nos. 4,816,397, 4,816,567, and 5,807,715).

Moreover, in the present invention, a "humanized antibody" is an antibody obtained by grafting (CDR grafting) a gene sequence of an antigen-binding site (CDR) of a non-human-derived antibody onto a human antibody gene. The preparation methods are known (see, for example, EP239400, EP125023, WO90/07861, WO96/02576).

In the present invention, a "functional fragment" of anantibodymeans apart (partial fragment) of anantibody, which binds to the antigen. Examples of the form of the "functional fragment" of the antibody according to the present invention include Fab, Fab', F(ab')2, a variable region fragment (Fv), a disulfide bonded Fv, a single chain Fv (scFv), a sc (Fv) 2, a diabody, a polyspecific antibody, and polymers thereof.

Here, "Fab" means a monovalent antigen-binding fragment, of an immunoglobulin, composed of a part of one light chain and a part of one heavy chain. Fab can be obtained by papain digestion of an antibody or by a recombination method. "Fab' " is different from Fab in that a small number of residues are added to the carboxy terminal of a heavy chain CH1 domain including one or more cysteines from an antibody hinge region. "F(ab')2" means a bivalent antigen-binding fragment, of an immunoglobulin, composed of parts of both light chains and parts of both heavy chains.

A "variable region fragment (Fv)" is a smallest antibody fragment having complete antigen recognition and binding sites. An Fv is a dimer in which a heavy chain variable region and a light chain variable region are strongly linked by non-covalent bonding. A "single chain Fv (scFv)" includes a heavy chain variable region and a light chain variable region of an antibody, and these regions exist in a single polypeptide chain. A "sc (Fv) 2" is a single chain obtained by linking two heavy chain variable regions and two light chain variable regions with a linker or the like. A "diabody" is a small antibody fragment having two antigen-binding sites. This fragment includes a heavy chain variable region linked to a light chain variable region in a single polypeptide chain. Each region forms a pair with a complementary region in another chain. A "polyspecific antibody" is a monoclonal antibody having a binding specificity to at least two different antigens. For example, a polyspecific antibody can be prepared by coexpression of two immunoglobulin heavy chain/light chain pairs in which two heavy chains have different specificities.

The antibody of the present invention includes antibodies whose amino acid sequences are modified without impairing desirable activities (antigen binding activity, the neutralization activity, and other biological properties). An amino acid sequence mutant of the antibody of the present invention can be prepared by introduction of a mutation into a DNA encoding an antibody chain of the present invention or by peptide synthesis. Examples of such a modification include substitution, deletion, addition, and/or insertion of a residue in the amino acid sequence of the antibody of the present invention. A site where the amino acid sequence of the antibody is modified may be a constant region of the heavy chain or the light chain of the antibody or a variable region (framework region and CDR) thereof, as long as the resulting antibody has activities equivalent to those before the modification. It is conceivable that modification on an amino acid other than CDR has a relatively small influence on binding affinity for an antigen. As of now, there are known screening methods for an antibody whose affinity for an antigen has been enhanced by modifying an amino acid of CDR (PNAS, 102: 8466-8471 (2005), Protein Engineering, Design & Selection, 21: 485-493 (2008), International Publication No. WO2002/051870, J. Biol. Chem., 280: 24880-24887 (2005), Protein Engineering, Design & Selection, 21: 345-351 (2008)).

The number of amino acids modified is preferably 10 amino acids or less, more preferably 6 amino acids or less, furthermore preferably 5 amino acids or less, and most preferably 3 amino acids or less (for example, 2 amino acids or less, 1 amino acid).

In fact, 1H11, 2H5, and 5G2 obtained in Examples to be described later exhibit similar antigen binding abilities and neutralization activities to each other. In comparing their CDR sequences, regarding CDR1 of the light chain variable region, there is such a common sequence that the amino acids at residues 1 and 2 from the N-terminal side are both serine, the amino acid at residue 5 from the N-terminal side is glycine, and the amino acid at residue 2 from the C-terminal side is asparagine; however, the remaining 4four or five amino acids are different. Moreover, regarding CDR2 of the light chain variable region, the amino acid at residue 1 from the N-terminal side is asparagine in common; however, the remaining two amino acids are different. Further, regarding CDR3 of the light chain variable region, the amino acids at residues 3 and 4 from the N-terminal side are both serine in common; however, the remaining five or six amino acids are different. Further, regarding CDR1 of the heavy chain variable region, only the amino acid at residue 3 from the N-terminal side is different. Furthermore, regarding CDR2 of the heavy chain variable region, only the amino acid at residue 1 from the C-terminal side is different. Additionally, regarding CDR3 of the heavy chain variable region, only the amino acid at residue 1 from the C-terminal side is different. As described above, even though the CDRs vary in the amino acids, the antibodies can exert similar antigen binding abilities and neutralization activities. Thus, the antibody of the present invention also includes antibodies whose amino acid sequences of CDR or the like are modified.

The modification of amino acids is preferably conservative substitution. In the present invention, the term "conservative substitution" means substitution with a different amino acid residue having a chemically similar side chain. Groups of amino acid residues having chemically similar amino acid side chains are well known in the technical field to which the present invention pertains. For example, amino acids can be grouped into acidic amino acids (aspartic acid and glutamic acid), basic amino acids (lysine, arginine, histidine), and neutral amino acids such as amino acids having a hydrocarbon chain (glycine, alanine, valine, leucine, isoleucine, proline), amino acids having a hydroxy group (serine, threonine), amino acids containing sulfur (cysteine, methionine), amino acids having an amide group (asparagine, glutamine), an amino acid having an imino group (proline), and amino acids having an aromatic group (phenylalanine, tyrosine, tryptophan).

Meanwhile, "having equivalent activities" and similar phrases mean that the antigen binding activity and the neutralization activity are equivalent (for example, 70% or more, preferably 80% or more, more preferably 90% or more) to those of a subject antibody (typically, 1H11, 2H5, or 5G2 described later in Examples). The antigen binding activity can be evaluated by utilizing an immunological analysis method as described above.

Further, the modification on the antibody of the present invention may be a modification on post-translational process of the antibody such as, for example, alternation of the number or position of the glycosylation sites. Thereby, for example, the ADCC activity of the antibody can be improved. Glycosylation of the antibody is typically N-linked or O-linked glycosylation. The glycosylation of the antibody largely depends on cells used for expression of the antibody. The glycosylation pattern can be modified by known methods such as introduction or deletion of a certain enzyme involved in carbohydrate production (Japanese Unexamined Patent Application Publication No. 2008-113663, U.S. Pat. Nos. 5,047,335, 5,510,261, and 5,278,299, International Publication No. WO99/54342). Furthermore, in the present invention, for the purpose of increasing the stability of the antibody or other purposes, an amino acid subjected to deamidation or an amino acid next to the amino acid subjected to the deamidation may be substituted with a different amino acid to suppress the deamidation. Alternatively, the stability of the antibody can also be increased by substituting glutamic acid with a different amino acid. The present invention also provides an antibody thus stabilized.

In the case where the antibody of the present invention is a polyclonal antibody, the polyclonal antibody can be obtained as follows. Concretely, an animal is immunized with an antigen (the HA protein derived from the A/Suita/1/200/2009 strain, a partial peptide thereof, cells expressing these, or the like). An antiserum from the animal is purified by conventional means (for example, salting-out, centrifugation, dialysis, column chromatography, or the like) to obtain the polyclonal antibody.

Meanwhile, a monoclonal antibody can be prepared by a hybridoma method or a recombinant DNA method.

A typical example of the hybridoma method includes a method by Kohler and Milstein (Kohler & Milstein, Nature, 256: 495 (1975)). Antibody-producing cells used in the cell fusion process of this method are spleen cells, lymph node cells, peripheral blood leukocytes, or the like of an animal (for example, mouse, rat, hamster, rabbit, monkey, goat, chicken, camel) immunized with the antigen. It is also possible to use antibody-producing cells obtained by causing the antigen to act, in a medium, on the above-described types of cells, lymphocytes, or the like, which are isolated from non-immunized animals in advance. As myeloma cells, various known cell lines can be used. The antibody-producing cells and the myeloma cells may be originated from different animal species, as long as they can be fused. However, the antibody-producing cells and the myeloma cells are preferably originated from the same animal species. Hybridomas can be produced, for example, by cell fusion between mouse myeloma cells and spleen cells obtained from a mouse immunized with the antigen. By the subsequent screening, a hybridoma can be obtained which produces an antibody capable of binding, such as capable of binding to the epitope present at residues 40 to 58 in the HA2 region of the hemagglutinin protein derived from the A/Suita/1/200/2009 strain. The monoclonal antibody of the present invention can be obtained by culturing the hybridoma, or from the ascitic fluid of a mammal to which the hybridoma is administered.

The recombinant DNA method is a method by which the antibody of the present invention is produced as a recombinant antibody as follows. Concretely, a DNA encoding the antibody of the present invention is cloned from a hybridoma, B cells, or the like. The cloned DNA is incorporated into an appropriate vector, which is then introduced into cells (for example, a mammalian cell line, *Escherichia coli,* yeast cells, insect cells, plant cells, or the like) for the production (for example, P. J. Delves, Antibody Production: Essential Techniques, 1997 WILEY, P. Shepherd and C. Dean Monoclonal Antibodies, 2000 OXFORD UNIVERSITY PRESS, Vandamme A. M. et al., Eur. J. Biochem. 192: 767-775 (1990)). For the expression of the DNA encoding the antibody of the present invention, DNAs encoding the heavy chain or the light chain may be incorporated separately into expression vectors to transform the cells. Alternatively, the DNAs encoding the heavy chain and the light chain may be incorporated into a single expression vector to transform the cells (see International Publication No. WO94/11523).

The antibody of the present invention can be obtained in a substantially pure and homogeneous form by culturing the transformed cells or the hybridoma, followed by collection (separation and purification) in the cells or from the culture fluid. For the separation and purification of the antibody, an ordinary method used for polypeptide purification can be employed.

Once a transgenic animal (cattle, goat, sheep, pig, or the like) incorporating the antibody gene is prepared using a transgenic animal production technique, a large amount of monoclonal antibodies derived from the antibody gene can also be obtained frommilk of the transgenic animal.

Thus, the present invention can also provide: a DNA encoding the antibody of the present invention; and a cell which produces the antibody of the present invention, or comprises the DNA encoding the antibody of the present invention. Moreover, the present invention can also provide a method for producing an antibody, comprising the steps of:
culturing the above-described cell; and
collecting the antibody of the present invention produced in the cell or from a culture fluid thus obtained.

Further, the antibody of the present invention can form an immunoconjugate by forming a conjugate with a functional agent. The functional agent can be a cytotoxic agent such as a chemotherapeutic agent, a toxin (for example, an enzymatically active toxin originated from bacteria, fungi, plants, or animals, or a fragment thereof), or a radioisotope (i.e., radioconjugate), an antibiotic, a nuclease, or any combination thereof. For the preparation of the immunoconjugate, it is possible to use chemotherapeutic agents, for example, methotrexate, adriamycin, vinca alkaloids (vincristine, vinblastine, etoposide), doxorubicin, melphalan, mitomycin C, chlorambucil, daunorubicin, or other intercalating agents; enzymes and/or fragments thereof, such as nucleases; antibiotics; toxins, including for example fragments and/or variants thereof, small molecule toxins, or enzymatically active toxins originated from bacteria, fungi, plants, or animals; and various antitumor agents or anticancer agents disclosed below. Examples of the usable enzymatically active toxins and fragments thereof include diphtheria toxin A chain, non-binding active fragments of diphtheria toxin, exotoxin A chain (derived from *Pseudomonas aeruginosa*), ricin A chain, abrin A chain, modeccin A chain, α-sarcin, *Aleurites fordii* proteins, dianthin proteins, *Phytolacca americana* proteins (PAPI, PAPII, and PAP-S), *Momordica* charantia inhibitor, curcin, crotin, *Saponaria officinalis* inhibitor, gelonin, mitogellin, restrictocin, phenomycin, enomycin, and tricothenes. Any appropriate radionucleotide or radioactive agent known or available in the technical field can be used to produce a radioconjugated antibody.

The conjugate of the antibody and the cytotoxic agent can be prepared using various bifunctional protein coupling agents, for example, N-succinimidyl-3-(2-pyridyldithiol)propionate (SPDP); iminothiolane (IT); bifunctional derivatives of imidoesters (such as dimethyl adipimidate HCL); active esters (such as disuccinimidyl suberate); aldehydes (such as glutaraldehyde); bis-azido compounds (such as bis(p-azidobenzoyl)hexanediamine); bis-diazonium derivatives (such as bis-(p-diazoniumbenzoyl)-ethylenediamine); diisocyanates (such as tolyene 2,6-diisocyanate); bis-active fluorine compounds (such as 1,5-difluoro-2,4-dinitrobenzene); maleimidocaproyl (MC); valine-citrulline, dipeptide site in protease cleavable linker (VC); 2-amino-5-ureido pentanoic acid PAB=p-aminobenzylcarbamoyl ("self immolative" portion of linker) (Citrulene); N-methyl-valine citrulline (here, the linker peptide bond is modified to prevent its cleavage by cathepsin B) (Me); maleimidocaproyl-polyethylene glycol attached to antibody cysteines; N-succinimidyl 4-(2-pyridylthio)pentanoate (SPP); and N-succinimidyl 4-(N-maleimidomethyl)cyclohexane-1 carboxylate (SMCC). For example, a ricin immunotoxin can be prepared as described in Vitetta et al., Science, 238: 1098 (1987). ¹⁴C (Carbon-14)-labeled 1-isothiocyanatobenzyl-3-methyldiethylene triaminepentaacetic acid (MX-DTPA) is an exemplary chelating agent for conjugate formation of a radionucleotide to an antibody. See International Publication No. WO94/1102 The antibody can form a conjugate with a "receptor" (such as streptavidin) used in tumor pre-targeting. After the antibody-receptor conjugate is administered to a subject, followed by removal of unbound conjugate from the blood circulation using a clearing agent, and then administration of a "ligand" (for example, avidin) that forms a conjugate with a cytotoxic agent (for example, a radionucleotide).

The antibody of the present invention can be directly or indirectly coupled to a detectable marker by a technique known in the technical field. The detectable marker is an agent detectable, for example, by spectroscopic, photochemical, biochemical, immunochemical, or chemical means. Examples of useful detectable markers include, but are not limited to, fluorescent dyes, chemiluminescent compounds, radioisotopes, electron-dense reagents, enzymes, colored particles, biotin, or dioxigenin. A detectable marker often generates a measurable signal such as radioactivity, fluorescence, color, or enzyme activity. An antibody forming a conjugate with a detectable agent can be used for diagnostic or treatment purposes. Examples of the detectable agent include various enzymes, prosthetic groups, fluorescent materials, luminescent materials, bioluminescentmaterials,radioactive materials,positron emitting metals used in various positron emission tomographies, and nonradioactive paramagnetic metal ions. The detectable substance can be coupled or conjugated either directly or indirectly to the antibody through an intermediate such as, for example, a linker known in the technical field by using a technique known in the technical field. See, for example, U.S. Pat. No. 4,741,900 describing a conjugate formation of metal ions to an antibody for diagnosticuse. Examples of suitable enzymes include horseradish peroxidase, alkaline phosphatase, β-galactosidase, and acetylcholinesterase. Examples of suitable prosthetic group complexes include streptavidin/biotin and avidin/biotin. Examples of suitable fluorescent materials include umbelliferone, fluorescein, fluorescein isothiocyanate, rhodamine, dichlorotriazinylamine fluorescein, dansyl chloride, and phycoerythrin. An example of the luminescent materials includes luminal. Examples of the bioluminescent materials include luciferin and aequorin.

The antibody can be bispecific. A bispecific antibody capable of specifically binding to one protein and also specifically binding to another antigen relevant to a disease condition and/or treatment is produced, isolated, and tested using standard methods described in the literature (see, for example, Pluckthun & Pack, Immunotechnology, 3: 83-105 (1997), Carter, et al., J. Hematotherapy, 4: 463-470 (1995), Renner & Pfreundschuh, Immunological Reviews, 1995, No. 145, pp. 179-209, U.S. Pat. No. 5,643,759 of Pfreundschuh, Segal, et al., J. Hematotherapy, 4: 377-382 (1995), Segal, et al., Immunobiology, 185: 390-402 (1992), and Bolhuis, et al., Cancer Immunol. Immunother., 34: 1-8 (1991)).

Moreover, the antibody of the present invention can be formulated as an immunoliposome. A liposome comprising the antibody is prepared by methods known in the technical field as described in Epstein et al., Proc. Natl. Acad. Sci. USA, 82: 3688 (1985), Hwang et al., Proc. Natl. Acad. Sci. USA 77: 4030 (1980), and U.S. Pat. Nos. 4,485,045 and 4,544,545. U.S. Pat. No. 5,013,556 discloses liposomes with enhanced circulation time. Particularly useful liposomes can be prepared by the reverse-phase evaporation method using a lipid composition containing phosphatidylcholine, cholesterol, and PEG-derivatized phosphatidylethanolamine (PEG-PE). A liposome having a desired diameter can be obtained by extruding a liposome through a filter of predetermined pore diameter. Fab' fragments of the antibody of the present invention can form a conjugate with a liposome by a disulfide exchange reaction as described in Martin et al. , J. Biol. Chem. , 257 : 286-288 (1982). The liposome optionally comprises a chemotherapeutic agent (such as doxorubicin). See Gabizon et al, J. National Cancer Inst., 81 (19): 1484 (1989).

### <Method for Preventing or Treating Infectious Disease of Group 1 Influenza A Virus>

The present invention provides a method for preventing or treating an infectious disease of group 1 influenza A virus in a human subject, the method comprising administering a therapeutically effective amount of the antibody of the present invention to the human subject. The method can further comprise diagnosing the patient as an infectious disease of group 1 influenza A virus. The antibody of the present invention can be administered before the patient is diagnosed as the infectious disease of influenza, during the diagnosis, and/or after the diagnosis.

The method can further comprise monitoring a decrease in at least one symptom of an infectious disease of group 1 influenza A virus. Example of the at least one symptom may include fever, headache, fatigue, chills, malaise, muscle sore, joint pain, nasal congestion, sore throat, cough, respiratory distress, stomachache, or any combination thereof. The antibody of the present invention is administered together with one or more additional therapeutic drugs against influenza A and/or other influenzas such as influenza B and/or influenza C. The combination can act synergistically to inhibit or treat group 1 influenza A viruses. Examples of the one or more additional therapeutic drugs can include a neuraminidase inhibitor, a hemagglutinin inhibitor, an anti-inflammatory agent, or any combination thereof. Examples of the neuraminidase inhibitor include zanamivir, oseltamivir,peramivir,laninamivir,any pharmaceutically acceptable salts thereof, or any combination thereof.

According to the present invention, two or more influenza antagonists can be administered. At least one of the influenza antagonists can include an influenza A virus antagonist. At least one influenza B virus antagonist can be combined with one or more influenza A virus antagonists and/or one or more influenza C virus antagonists. At least one influenza antagonist can be administered in combination with one or more additional therapeutic drugs against infectious diseases of influenza viruses. The administration of two or more therapeutic drugs including one or more influenza antagonists may be simultaneous, sequential, or in combination. Thus, when two or more therapeutic drugs are administered, it is not necessary to administer the therapeutic drugs simultaneously or in the same way or in the same dose. When administered simultaneously, the two or more therapeutic drugs may be administered in the same composition or in different compositions. The two or more therapeutic drugs may be administered using the same route of administration or different routes of administration. When administered at different times, the therapeutic drugs may be administered before or after each other. Administration order of the two or more therapeutic drugs can be changed. Each dose of the one or more therapeutic drugs can be changed over time. The type of the one or more therapeutic drugs can be changed overtime. In the administration at separate times, the separation of the two or more administrations can be any time period. In multiple administrations, the length of the time period can be changed. The separation between the administrations of the two or more therapeutic drugs can be 0 seconds, 1 second, 5 seconds, 10 seconds, 30 seconds, 1 minute, 5 minutes, 10 minutes, 15 minutes, 20 minutes, 30 minutes, 45 minutes, 1 hour, 1.5 hours, 2 hours, 2.5 hours, 3 hours, 4 hours, 5 hours, 7.5 hours, 10 hours, 12 hours, 15 hours, 18 hours, 21 hours, 24 hours, 1.5 days, 2 days, 3 days, 4 days, 5 days, 6 days, 7 days, 10 days, 2 weeks, 3 weeks, 4 weeks, 1 month, 6 weeks, 8 weeks, 3 months, 6 months, 1 year or longer.

Two or more influenza virus antagonists can act synergistically to treat or reduce an infectious disease of influenza or a symptom thereof, for example, fever. An influenza virus antagonist can be one or more anti-influenza virus antibodies alone, or in combination with one or more other influenza virus antagonists, for example, a small pharmaceutical drug(s), or other anti-influenza virus therapeutic drugs. Two or more anti-influenza virus antibodies, or at least one anti-influenza virus antibody, and one or more additional therapeutic drugs can act synergistically to treat or reduce an infectious disease of group 1 influenza A virus. Two or more therapeutic drugs including one or more anti-influenza virus antibodies can be administered in synergistic amounts. Thus, the administration of two or more therapeutic drugs can have a synergistic effect on the decrease in one or more symptoms of an infectious disease of influenza, whether administered simultaneously, sequentially, or in any combination. A first therapeutic drug can increase the efficacy of a second therapeutic drug much greater than a case where the second therapeutic drug is used alone, or the second therapeutic drug can increase the efficacy of the first therapeutic drug, or both. The effect of administering two or more therapeutic drugs can be such that the effect on the decrease in one or more symptoms of an infectious disease of influenza is greater than the additive effect of administering each therapeutic drug alone. When given in synergistic amounts, one therapeutic drug can enhance the efficacy of one or more other therapeutic drugs on the decrease in one or more symptoms of an infectious disease of influenza, even if the amount of one or more therapeutic drugs alone does not have a substantial effect on one or more symptoms of the infectious disease of influenza. The measurement and calculation of synergism can be performed as described in Teicher, "Assays for In Vitro and In Vivo Synergy," in Methods in Molecular Medicine, vol. 85: Novel Anticancer Drug Protocols, pp. 297-321 (2003), and/or by calculating the combination index (CI) using CalcuSyn software.

### <Use of Antibody of Present Invention to Produce Drug for Preventing or Treating Infectious Disease of Group 1 Influenza A Virus>

The present invention provides use of the antibody of the present invention to produce a drug for preventing or treating an infectious disease of group 1 influenza A virus in a human subject. The present invention also provides a method for detecting a group 1 influenza A virus in a human subject. The method can comprise bringing a sample derived from the human subject into contact with the antibody of the present invention. The method can further comprise detecting the presence or absence of a group 1 influenza A virus in the human subject based on whether the antibody binds to an HA2 protein of the group 1 influenza A virus.

### <Pharmaceutical Composition Comprising Antibody of Present Invention as Active Ingredient>

The present invention provides a pharmaceutical composition comprising the antibody of the present invention as an active ingredient (a pharmaceutical composition comprising the antibody of the present invention and a pharmacologically acceptable carrier). The present invention further provides a kit for at least one of the prevention, treatment, and detection of a group 1 influenza A virus, the kit comprising the antibody of the present invention. The kit can comprise the pharmaceutical composition and/or one or more additional influenza virus antagonists or other antagonists.

Exact formulation, route of administration, and dosage can be selected by the individual physician in view of the patient's condition (see, for example, Fingl et. al., in The Pharmacological Basis of Therapeutics, 1975, Ch. 1 p. 1). The attending physician can determine when to terminate, interrupt, or adjust administration in accordance with toxicity or organ dysfunctions. In contrast, the attending physician can also adjust the treatment to higher levels if the clinical response were not adequate, precluding toxicity. The magnitude of an administrated dose in the management of disorder of interest will vary with the severity of the disorder to be treated and the route of administration. The severity of the disorder can, for example, be evaluated in part by standard prognostic evaluation methods. The dose and dose frequency can vary according to the age, body weight, and response of the individual patient. A program comparable to that described above can be used in veterinary medicine.

The use of pharmaceutically acceptable carriers to formulate the compounds in the present description disclosed for the practice of the present invention into dosages suitable for systemic administration is within the scope of the present invention. With proper choice of carrier and suitable manufacturing practice, the compositions relevant to the present invention, particularly those formulated as solutions, can be administered parenterally by intravenous injection or the like. The compounds can be formulated readily using pharmaceutically acceptable carriers known in the technical field, into dosages suitable for oral administration. Such carriers enable the compounds relevant to the present invention to be formulated as tablets, pills, capsules, liquids, gels, syrups, slurries, tablets, dragees, solutions, suspensions, and the like for oral ingestion by a patient to be treated.

The therapeutic agent can be prepared in a depot form to allow for release into the body to which it is administered and to be controlled in accordance with time and location within the body (see, for example, U.S. Pat. No. 4,450,150). The depot form of the therapeutic agent can be, for example, an implantable composition containing the therapeutic agent and a porous or non-porous material, such as a polymer, where the therapeutic agent is encapsulated by the material or diffused throughout the material and/or degradation of the non-porous material. The depot is then implanted into a desired location within the body, and the therapeutic agent is released from the implant at a predetermined rate.

The therapeutic agent used in the present invention can be formed as a composition such as a pharmaceutical composition containing a carrier and a therapeutic compound. A pharmaceutical composition containing the therapeutic agent may contain two or more therapeutic agents. Alternatively, the pharmaceutical composition may contain the therapeutic agent together with other pharmaceutically active agents or drugs.

The carrier can be any suitable carrier. For example, the carrier can be a pharmaceutically acceptable carrier. With respect to pharmaceutical compositions, the carrier may be any of those conventionally used with consideration of the route of administration and chemico-physical considerations such as solubility and lack of reactivity with the active compound(s). In addition to, or in the alternative to, the following pharmaceutical compositions, the therapeutic compounds in the methods of the present invention can be formulated as liposomes or inclusion complexes such as cyclodextrin inclusion complexes.

The pharmaceutically acceptable carriers described in the present description, for example, vehicles, adjuvants, excipients, and diluents, are known to those skilled in the art and are readily available to the public. The pharmaceutically acceptable carrier can be chemically inert to the active agent (s) and have no detrimental side effects or toxicity under use conditions. The choice of carrier can be determined in part by the particular therapeutic agent, and by the particular method used to administer the therapeutic compound. There are a variety of suitable formulations of the pharmaceutical composition of the present invention. The following formulations for oral administration, aerosol administration, parenteral administration, subcutaneous administration, transdermal administration, transmucosal administration, intestinal administration, intramedullary injection, direct intraventricular administration, intravenous administration, intranasal administration, intraocular administration, intramuscular administration, intraarterial administration, intrathecal administration, intraperitoneal administration, rectal administration, and intravaginal administration are exemplary and are in no way limited thereto. Two or more routes can be used to administer the therapeutic agent, and in some cases, a particular route may provide more immediate and effective response than another route. Depending on a particular disorder to be treated, such agents can be formulated and administeredsystemically orlocally. The formulation and administration methods can be found in Remington's Pharmaceutical Sciences, 18th ed., Mack Publishing Co., Easton, Pa. (1990).

Formulations suitable for oral administration can contain: (a) an effective amount of an inhibitor dissolved in a diluent such as a liquid solution, for example, water, saline, or orange juice; (b) a capsules, a sachet, a tablet, a lozenge, and a troche, each containing a predetermined amount of the active ingredient as solid or granule; (c) a powder; (d) an appropriate liquid suspension; and (e) a suitable emulsion. Liquid formulations can contain a diluent such as water and alcohols, for example, ethanol, benzyl alcohol, and polyethylene alcohols, either with or without the addition of a pharmaceutically acceptable surfactant. Capsule forms can be of ordinary hard or soft shelled gelatin type containing, for example, a surfactant, a lubricant, and an inert filler such as lactose, sucrose, calcium phosphate, and corn starch. Tablet forms can contain one or more of lactose, sucrose, mannitol, corn starch, potato starch, alginic acid, microcrystalline cellulose, acacia, gelatin, guar gum, colloidal silicon dioxide, croscarmellose sodium, talc, magnesium stearate, calcium stearate, zinc stearate, stearic acid, and other excipients, colorants, diluents,buffers, disintegrators, wetting agents, preservatives, flavoring agents, and other pharmacologicallycompatible excipients. Lozenge forms can contain an inhibitor in a flavor, usually sucrose and acacia or tragacanth, as well as a pastille containing the inhibitor in an inert base, for example, gelatin and glycerin, or sucrose and acacia, an emulsion, a gel, or the like, and can further contain such excipients as known in the technical field.

Examples of pharmaceutical preparations that can be used orally include push-fit capsules made of gelatin, and soft sealed capsules made of gelatin and a plasticizer such as glycerol or sorbitol. The push-fit capsules can contain the active ingredients in admixture with a filler such as lactose, a binder such as starch, and/or a lubricant such as talc or magnesium stearate, and optionally a stabilizer. In the soft capsules, the active compound may be dissolved or suspended in a suitable liquid such as a fatty oil, a liquid paraffin, or a liquid polyethylene glycol. In addition, a stabilizer may also be added thereto.

The therapeutic agent, alone or in combination with other suitable constituents, can be made into aerosol formulations for inhalation administration. These aerosol formulations can be placed into acceptable pressurized gases such as dichlorodifluoromethane, propane, and nitrogen. These aerosol formulations can also be formulated as pharmaceutical drugs for non-pressurized preparations, such as in a nebulizer or an atomizer. Such spray formulations can also be used to spray mucosa. Topical formulations are known to those skilled in the art. Such formulations are particularly suitable in the present invention for application to the skin.

Injectable formulations are in accordance with the present invention. The parameters for effective pharmaceutical carriers for injectable compositions are known to those skilled in the art (see, for example, Pharmaceutics and Pharmacy Practice, J. B. Lippincott Company,Philadelphia,PA,Banker and Chalmers,eds.,pages 238250 (1982), and ASHP Handbook on Injectable Drugs, Toissel, 4th ed., pages 622 630 (1986)). For injection, the agents of the present invention can be formulated in an aqueous solution, preferably in a physiologically compatible buffer such as Hanks's solution, Ringer's solution, or physiological saline buffer. For such transmucosal administration, a penetrant appropriate to the barrier to be permeated is used in the formulation. Such penetrants are generally known in the technical field.

Formulations suitable for parenteral administration can include: aqueous and non-aqueous, isotonic sterile injection solutions, which can contain anti-oxidants, buffers, bacteriostats, and solutes that render the formulation isotonic with the blood of the intended recipient; and aqueous and non-aqueous sterile suspensions which can contain suspending agents, solubilizers, thickers, stabilizers, and preservatives. The therapeutic agent can be administered in a physiologically acceptable diluent in a pharmaceutical carrier, such as a sterile liquid or mixture of liquids, including, for example, water, saline, aqueous dextrose and related sugar solutions, an alcohol such as ethanol or hexadecyl alcohol, a glycol such as propylene glycol or polyethylene glycol, poly(ethylene glycol) 400, glycerol, dimethylsulfoxide, ketals such as 2,2-dimethyl-1,3-dioxolane-4-methanol, ethers, oils, fattyacids, fatty acid esters or glycerides, or acetylated fatty acid glycerides, with or without the addition of a pharmaceutically acceptable surfactant, for example, a soap or a detergent, a suspending agent such as pectins, carbomers, methylcellulose, hydroxypropylmethylcellulose, or carboxymethyl cellulose, or emulsifiers and other pharmaceutical adjuvants.

Examples of the oils, which can be used in parenteral formulations, include petroleum, animal oils, vegetable oils or synthetic oils. Specific examples of the oils include peanut oils, soybean oil, sesame oil, cottonseed oil, corn oil, olive oil, petrolatum, and mineral oil. Examples of suitable fatty acids for use in parenteral formulations include oleic acid, stearic acid, and isostearic acid. Ethyl oleate and isopropyl myristate are examples of suitable fatty acid esters.

Examples of suitable soaps for use in parenteral formulations include fatty alkali metal, ammonium, and triethanolamine salts. Examples of suitable detergents include: (a) cationic detergents such as, for example, dimethyl dialkyl ammonium halides and alkyl pyridinium halides; (b) anionic detergents such as, for example, alkyl, aryl, and olefin sulfonates, alkyl, olefin, ether, and monoglyceridesulfates, and sulfosuccinates; (c) nonionic detergents such as, for example, fatty amine oxides, fatty acid alkanolamides, and polyoxyethylenepolypropylene copolymers; (d) amphoteric detergents such as, for example, alkyl-β-aminopropionates and 2-alkyl-imidazoline quaternary ammonium salts; and (e) mixtures thereof.

The parenteral formulations can contain from approximately 0.5% to approximately 25% by weight of the drug in the solution. Preservatives and buffers can be used. In order to minimize or eliminate inflammation at the injection site, such compositions may contain one or more nonionic surfactants having a hydrophilic-lipophilic balance (HLB) of from approximately 12 to approximately 17. The amount of a surfactant in such formulations is normally within a range from approximately 5% to approximately 15% by weight. Examples of suitable surfactants include polyethylene glycol sorbitan fatty acid esters, such as sorbitan monooleate and high molecular weight adducts of ethylene oxide with a hydrophobic base, formed by the condensation of propylene oxide with propylene glycol. The parenteral formulations can be presented in unit-dose or multi -dose sealed containers such as ampoules and vials, and can be stored in a freeze-dried (lyophilized) condition requiring only the addition of a sterile liquid excipient, for example, water, for injections immediately prior to use. Extemporaneous injection solutions and suspensions can be prepared from sterile powders, granules, and tablets of the kind previously described.

The therapeutic agent can be made into suppositories by mixing with a variety of bases such as emulsifying bases or water-soluble bases. Formulations suitable for intravaginal administration can be presented as pessaries , tampons, creams, gels, pastes, foams, or spray formulations containing, in addition to the active ingredient, such carriers as are known to be appropriate in the technical field.

Agents intended to be administered intracellularly can be administered using techniques known to those skilled in the art. For example, such agents can be encapsulated into liposomes. Liposomes are spherical lipid bilayers with aqueous interiors. Molecules present in an aqueous solution at the time of liposome formation are incorporated into the aqueous interior. The liposomal contents are both protected from the external microenvironment and efficiently delivered into the cell cytoplasm because liposomes fuse with cell membranes. Further, due to their hydrophobicity, small organic molecules may be directly administered intracellularly.

Furthermore, when the antibody of the present invention is used in the detection of a group 1 influenza A virus, the antibody of the present invention may be labeled. As the label, it is possible to use, for example, detectable markers as described above. When the antibody of the present invention is to be prepared as a testing agent, it can be obtained in any dosage form by adopting any means suitable for the purpose. For example, a purified antibody is measured for the antibody titer and diluted as appropriate with PBS or the like; thereafter, 0.1% sodium azide or the like can be added as a preservative. Alternatively, for example, the antibody of the present invention adsorbed to latex or the like is measured for the antibody titer and diluted as appropriate, and a preservative may be added thereto for use.

In addition, the present invention also encompasses a kit for detecting a group 1 influenza A virus, the kit comprising the testing agent of the present invention as a constituent. The kit may comprise, for example, for carrying out an antigen-antibody reaction (such as an ELISA method, an immunohistochemical staining method, flow cytometry),various reagents (such as a secondary antibody, a chromogenic reagent, a staining reagent, a buffer, a standard preparation), a reaction vessel, an operation tool, and/or an instruction, in addition to the testing agent of the present invention.

### <Aptamer>

The influenza antagonist can comprise an aptamer capable of binding to an HA protein of a group 1 influenza A virus. The aptamer is capable of binding to HA in the same location/epitope as the antibody of the present invention, and/or in other locations/epitopes. The aptamer can contain one or more of a nucleic acid, a RNA, a DNA, and an amino acid. Aptamers can be selected and prepared using any suitable technique or protocol. For example, oligonucleotide libraries having variable regions ranging from 18 to 50 nucleotides in length can be used as templates for run-off transcription reactions to prepare random pools of RNA aptamers. Then, the aptamer pools can be exposed to nonconjugated matrix to remove non-specific interacting species. Subsequently, the remaining pool is incubated together with an immobilized target. The majority of aptamer species in this pool have low affinity, and the target can be washed away, leaving a smaller, more specific pool bound to the matrix. Thereafter, this pool can be eluted, precipitated, and reverse transcribed for use as a template of run-off transcription. After five rounds of selection, aliquots can be removed that are cloned and sequenced. The selection can be continued until similar sequences are reproducibly recovered.

Aptamers can be prepared using a bead-based selection system. In this process, a library of beads is prepared in which each bead is coated with a population of aptamers having the same sequence composed of natural and modified nucleotides. This bead library, which may contain more than 100,000,000 unique sequences, can be incubated together with a peptide corresponding to a hemagglutinin (HA) protein or a portion thereof, for example, an extracellular domain, which forms a conjugate with a tag such as a fluorescent dye. After washing, beads exhibiting the highest binding affinity can be isolated, and aptamer sequences can be determined for subsequent synthesis.

### [Examples]

Hereinafter, the present invention will be described more specifically on the basis of Examples. However, the present invention is not limited to the following Examples.

Moreover, the materials and methods for obtaining the antibody of the present invention and the methods for evaluating the obtained antibody will be described below.

### <Preparation of Human Monoclonal Antibodies (HuMAbs)>

Hybridomas for producing anti-influenza virus antibodies, HuMAbs, were prepared using fusion partner cells, SPYMEG (manufactured by MEDICAL & BIOLOGICAL LABORATORIES CO., LTD.) (see Yasugi M, Kubota-Koketsu R, Yamashita A, Kawashita N, Du A, et al. (2013) Human monoclonal antibodies broadly neutralizing against influenza B virus. PLoS Pathog 9: e1003150., and Yasugi M, Kubota-Koketsu R, Yamashita A, Kawashita N, Du A, et al. (2013) Emerging antigenic variants at the antigenic site Sb in pandemic (H1N1) 2009 influenza virus in Japan detected by a human monoclonal antibody. PLoS One 8: e77892.).

To be more specific, first, blood was obtained from a patient infected with H1N1pdm in October 2009. Note that the patient was a patient who was rapid-diagnosed with the Prime Check Flu (H1N1) 2009 (manufactured by Alfresa Pharma Corporation) for the rapid detection of the NP protein derived from H1N1pdm (see Mizuike R, Sasaki T, Baba K, Iwamoto H, Shiba Y, et al. (2011) Development of two types of rapid diagnostic test kits to detect the hemagglutinin or nucleoprotein of the swine-origin pandemic influenza A virus H1N1. Clin Vaccine Immunol 18 : 494-499., and Sasaki T, Kubota-Koketsu R, Takei M, Hagihara T, Iwamoto S, et al. (2012)Reliability of a newly-developed immunochromatography diagnostic kit for pandemic influenza A/H1N1pdm virus: Implications for drug administration. PLoS One 7 : e50670.). Then, from the blood drawn at 3, 9, 16, and 23 days after the onset of symptoms, peripheral blood mononuclear cells (PBMCs) were collected by density gradient centrifugation using Ficoll-Paque Plus (manufactured by GE Healthcare). The obtained PBMCs were fused with the SPYMEG cells using polyethylene glycol #1500 (manufactured by Roche). Subsequently, the obtained fused cells were selectively cultured in Dulbecco's modified Eagle medium (DMEM, manufactured by Invitrogen) supplemented with 15% fetal bovine serum and hypoxanthine-aminopterin-thymidine.

Next, the first screening for antibody specific to influenza virus was performed by immunofluorescence assay (IFA). Then, the obtained specific MAb-positive cells were cloned by limiting dilution, followed by the second screening by IFA. Subsequently, hybridoma cells taken from IFA-positive wells that had a single colony per well were cultured and expanded in Hybridoma-SFM (manufactured by Invitrogen).

Thereafter, MAbs were purified from 100 ml of the hybridoma culture supernatant by affinity chromatography using HiTrap Protein G HP Columns (manufactured by GE Healthcare) . After that, the MAbs were dialyzed into PBS using Slide-A-Lyzer Dialysis Cassettes (manufactured by Thermo Scientific). Note that, as a control IgG, D23-1B3B9 HuMAb, derived from PBMCs of a patient infected with dengue virus serotype 2, was used.

### <Viruses>

In the present Examples, used were: six influenza A virus H1N1pdm isolates (A/Suita/1/2009, A/Osaka/168/2009, A/California/07/2009, A/Suita/117/2011, A/Suita/104/2011, A/Suita/105/2011), two influenza A virus H1N1 sea isolates (A/Brisbane/59/2007, A/ PR8/ 1934), one influenza A virus H2N2 isolate (A/Izumi/5/1965), two influenza A virus H3N2 isolates (A/Aichi/2/1968, A/Uruguay/716/2007), two influenza A virus H5N1 isolates (A/Duck/Egypt/DIBr12/2007, A/Chicken/Egypt/RIMD12-3/2008), one influenza A virus H7N7 isolate (A/Tufted dunk/Shimane/124R/1980), one influenza A virus H9N2 isolate (A/Turkey/Wisconsin/1/1966), and two influenza B virus isolates (B/Florida/4/2006, B/Malaysia/2506/04).

Additionally, among the virus strains used in the present Examples, strains isolated in Suita City, Osaka, Japan (Suita) were strains isolated and stored in the Research Institute for Microbial Diseases, Osaka University. Moreover, A/Osaka/168/2009 was a strain received from Osaka Prefectural Institute of Public Health. The others were strains obtained from the National Institute of Infectious diseases (Japan) and ATCC. In addition, those skilled in the art can obtain any of the strains by inquiring of the institutes.

The virus was propagated either in MDCK cells or 9-day-old embryonated chicken eggs. Moreover, the infectivity was titrated by focus-forming assay. To be more specific, the viruses serially 10-fold diluted were cultured together with the MDCK cells in a 96-well plate at 37°C for 1 hour. Then, the cells were washed with PBS and incubated at 37°C for 12 hours. Subsequently, the cells were fixed and subjected to IFA, probed by an antibody C43 against influenza A strains (see Okuno Y, Isegawa Y, Sasao F, Ueda S (1993) A common neutralizing epitope conserved between the hemagglutinins of influenza A virus H1 and H2 strains. J Virol 67: 2552-2558) and an antibody 5A7 against influenza A strains (see Yasugi M, Kubota-Koketsu R, Yamashita A, Kawashita N, Du A, et al. (2013) Human monoclonal antibodies broadly neutralizing against influenzaBvirus. PLoS Pathog 9: e1003150.). The infected cells were fixed with PBS containing 4% formaldehyde for 20 minutes at room temperature. Thereafter, the cells were treated and reacted with appropriately diluted antibody or hybridoma culture supernatant at 37°C for 1 hour, followed by incubation together with FITC-conjugated anti-human IgG at 37°C for 45 minutes.

### <Western Blotting>

The infected cells or transfected cells were collected in a loading buffer in the presence or absence of β-mercaptoethanol. Then, the cells were subjected to electrophoresis and blotted to a PVDF membrane. Next, the cells were probed with a hybridoma culture supernatant and cultured together with the PVDF membrane at 37 °C for 1 hour, followed by incubation together with HRP-conjugated anti-human IgG at 37°C for 1 hour.

### <Focus-Forming Assay>

The virus serially 10-fold diluted was cultured together with MDCK cells in a 96-well plate at 37°C for 1 hour. Then, the cells were washed with PBS and incubated at 37°C for 12 hours. Subsequently, the cells were fixed and subjected to IFA. Thereafter, the obtained focus-formingunit (FFU) was designatedas the infectivity.

### <VN Assay>

The VN test was carried out according to the method described in Okuno Y, Tanaka K, Baba K, Maeda A, Kunita N, et al. (1990) Rapid focus reduction neutralization test of influenza A and B viruses in microtiter system. J Clin Microbiol 28: 1308-1313, with minor modifications. To be more specific, 100 µg/ml of HuMAb was serially two-fold diluted with minimum essential medium (MEM, manufactured by Life Technologies) and cultured together with 200 FFU of the virus at 37°C for 1 hour. Then, the virus-antibody mixture prepared in this manner was adsorbed to MDCK cells by incubation at 37°C for 1 hour. After the incubation for 16 hours, the cells were fixed and subjected to IFA. The antibody concentration to suppress viral infection to 50% was estimated as VN50 and used as the representative VN titer.

### <HI Assay>

First, viral titers were determined by a hemagglutination assay. To be more specific, the virus was serially diluted two-fold with PBS and mixed with 0.7% (v/v) human O-type red blood cells. After incubation at room temperature for 1 hour, hemagglutination units (HAUs) were calculated. Next, HI titration was performed as follows: 100 µg/ml of HuMAb was serially two-fold diluted and mixed with 8 HAUs per 50 µl of viral sample. After incubation at 37°C for 1 hour, the mixture was further incubated together with 0.7% (v/v) human red blood cells at room temperature for 1 hour. Then, the lowest concentration of HuMAb to completely inhibit hemagglutination was designated as the HI titer.

### <Fusion Inhibition Assay>

An assay for cell-cell fusion was carried out according to the method described in Whittle JR, Zhang R, Khurana S, King LR, Manischewitz J, et al. (2011) Broadly neutralizing human antibody that recognizes the receptor-binding pocket of influenza virus hemagglutinin. Proc Natl Acad Sci USA 108: 14216-14221.

To be more specific, monkey kidney cell line CV-1 cells were infected with A/Suita/1/2009 at an MOI of 1.6. Then, after cultured for 24 hours in MEM supplemented with 4% bovine serum albumin (BSA) and 2.5 µg/ml of acetylated trypsin (manufactured by Sigma), the cells were washed with MEM. After the washing, the cells were incubated for 30 minutes together with diluted HuMAb. Thereafter, the cells were treated with PBS (pH 5.5) at 37°C for 5 minutes. After the medium was completely removed by washing, the cells were incubated for 3 hours. Then, the cells were fixed with absolute methanol and stained with Giemsa (manufactured by Wako).

### <Selection of Escape Mutation>

Escape mutations were selected by culturing A/Suita/1/2009 in the presence of HuMAb according to the method described in Yasugi M, Kubota-Koketsu R, Yamashita A, Kawashita N, Du A, et al. (2013) Human monoclonal antibodies broadly neutralizing against influenza B virus. PLoS Pathog 9: e1003150, with minor modifications.

To be more specific, MDCK cells were seeded in 96-well plates in an amount of 30000 cells per well and cultured for 16 hours. Next, the virus (100 FFU/ml) and HuMAb serially 10-fold diluted (0.0025, 0.025, 0.25, and 2.5 µg/ml) were mixed and incubated at 37°C for 1 hour. Then, the mixture was reacted with the MDCK cells. After the reaction at 37°C for 1 hour, the cells were washed with PBS and replenished with 200 µl of DMEM/F-12+GlutaMAX-I supplemented with 0.4% bovine serum albumin (BSA), antibiotics, and 2 µg/ml of acetylated trypsin. After 72 hours post-infection in this manner, the supernatant was collected and stocked. Moreover, all the viral stocks were titrated and used to infect new cell preparations. After the subsequent ten passages, the entire HA gene was directly sequenced from the mixed population in the culture supernatant.

### <Direct Sequencing Analysis>

Viral RNA was extracted using QIAamp viral RNA Mini kit (manufactured by Qiagen) and subjected to one step RT-PCR (Superscript III one-step RT-PCR System with Platinum Taq-High Fidelity, manufactured by Invitrogen) with the following HA primer set:
forward: 5'-TATTCGTCTCAGGGAGCAAAAGCAGGGG-3' (SEQ ID NO: 43) and
reverse: 5'-ATATCGTCTCGTATTAGTAGAAACAAGGGTGTTTT-3' (SEQ ID NO: 44).

The obtained PCR products were purified using Qiaquick PCR Purification kit (manufactured by Qiagen). After electrophoresis, the discrete band was collected using the Qiaquick Gel Extraction kit (manufactured by Qiagen) and subjected to sequencing analysis.

### <Sequencing of HuMAb Variable Regions>

Total RNA was collected from the hybridoma using an RNase Mini kit (manufactured by Qiagen) and subjected to RT-PCR using a PrimeScript RT reagent kit (manufactured by Takara) with an oligo (dt) primer. Then, the coding regions of the H- and L-chains of HuMAb were amplified by PCR with the following primers:
5'-ATGGAGTTTGGGCTGAGCTGGGTT-3' (H-chain-forward, SEQ ID NO: 45) and 5'-CTCCCGCGGCTTTGTCTTGGCATTA-3' (H-chain-reverse, SEQ ID NO: 46); and
5'-ATGGCCTGGRYCYCMYTCYWCCTM-3' (L-chain-forward, SEQ ID NO: 47) and 5'-TGGCAGCTGTAGCTTCTGTGGGACT-3' (L-chain-reverse, SEQ ID NO: 48).

The obtained PCR products were purified using Qiaquick PCR Purification kit (manufactured by Qiagen). After electrophoresis, the discrete band was collected using the Qiaquick Gel Extraction kit (manufactured by Qiagen). Then, their sequences were analyzed using a BigDye Terminator v3. 1 Cycle Sequencing kit and an ABI Prism 3100GeneticAnalyzer (manufactured by Applied Biosystems). Subsequently, the obtained sequences were analyzed and used to search the NCBI database with IgBLAST software (http://www.ncbi.nlm.nih.gov/igblast/).

### <Construction of HA Plasmids>

The HA gene of the A/Suita/1/2009 strain was amplified by one step RT-PCR and inserted into the pGEM-T Easy Vector (manufactured by Promega).

The HA genes of the wild type and an amino-acid substituted mutant were prepared by conventional PCR (Expand High Fidelity PLUS PCR System, manufactured by Roche) and site-directed mutagenic PCR (GeneTailor Site-Directed Mutagenesis System, manufactured by Invitrogen), respectively, using the HA-gene inserted pGEM-T easy as a template.

Each gene was subcloned into an expression vector pCAG PM2. Then, the obtained expression plasmids were transfected into human kidney-derived 293T cells with lipofectamine2000 (manufactured by Invitrogen) according to the attached instructions.

### <Genetic Analysis>

All the influenza A virus sequences (full-length, non-redundant, non-lab) were downloaded from the NCBI influenza virus resource (http://www.ncbi.nlm.nih.gov/genomes/FLU/FLU.html).

At the time of the downloading (June 5, 2013), the database included 296197 sequences encompassing all 16 influenza A viruses. Moreover, 8898 sequences from pandemic H1N1, 4378 sequences from seasonal H1N1, 2993 sequences from H5, and 995 sequences from H9 were aligned using the BioEdit program.

### <IgG Isotyping>

ELISA microplates (MaxiSorp, manufactured by Nunc) were coated with goat-derived anti-human IgG (manufactured by Jackson ImmunoResearch Laboratories) by adding 0.05 M sodium bicarbonate buffer (pH 8.6) containing the antibody to the plates, followed by incubation overnight at 4°C. Then, after washing with PBS containing 0.1% Tween 20, PBS containing 0.5% BSA was added followed by incubation at 37°C for 1 hour to block the wells. After washing again, the wells were incubated at 37°C for 2 hours with a hybridoma culture supernatant or control serum added thereto. After further washing, the wells were incubated at 37°C for 1 hour with HRP-conjugated anti-IgG1, IgG2, IgG3, or IgG4 (manufactured by SouthernBiotech) added thereto. Subsequently, the wells were washed five times, followed by incubation in the dark at room temperature with 3,3',5,5'-tetramethylbenzidine peroxidase substrate (KPL) added thereto. After 20 minutes, the reaction was stopped by adding a 2 N solution of sulfuric acid. Thereafter, the color development was evaluated as absorbance at a wavelength of 450 nm in an ELISA photometer (Biotek ELISA Reader, manufactured by Biotek). Note that all samples were analyzed in triplicate.

### <Molecular Modeling>

The HA protein structure was constructed using Cn3D and PyMol based on the crystal structures of A/Suita/1/2009 and A/California/07/2009.

### <Protease Susceptibility Assay>

To prevent aggregation of the post-fusion HA, 0.6 µg of baculovirus-expressed insoluble H1 HA (derived from A/Suita/1/2009) was incubated in 5 ml of 250 mM imidazole. Then, the resultant was transferred into four tubes in an amount of 100 µl per tube. To these tubes, 900 µl of a protease buffer (250 mM imidazole, 20 µg/ml trypsin-EDTA, pH 5.3) was added and incubated at 37°C for 1 hour. After the incubation, the mixtures were centrifuged, and the collected pellet was subjected to western blotting. In this western blotting, HuMAbs 1H11, 2H5, and 5G2 were used as primary antibodies, and a HuMAb 5E4 capable of binding to head regions of H1N1pdm was used as a control. Moreover, HRP-conjugated anti-human IgG was used as a secondary antibody.

### <Trypsin Cleavage Inhibition Assay>

The baculovirus-expressed insoluble H1 HA (derived from A/Suita/1/2009), 0.6 µg, was incubated in 5 ml of 250 mM imidazole. Then, the resultant was transferred into four tubes in an amount of 100 µl per tube. One of the tubes was used as a control to which 6 µg of 5E4 and 900 µl of a protease buffer (250 mM imidazole, 20 µg/ml trypsin-EDTA, pH 5.3) were further added.

To the other three tubes, HuMAb (1H11, 2H5, or 5G2) was added together with 900 ml of the protease buffer, and mixed. Then, the reaction was allowed at 37°C for 1 hour. After incubation, the reaction products were centrifuged, and the collected pellet was subj ected to a western blotting test. In this western blotting, 5E4 was used as a primary antibody. Moreover, HRP-conjugated anti-human IgG was used as a secondary antibody.

### <Epitope Analysis>

The epitope region of each HuMAb was analyzed by mass spectrometry following to the protease treatment, according to the Przybylski method (Macht M, Marquardt A, Deininger SO, Damoc E, Koh; mann M, et al. (2004) "Affinity-proteomics":direct protein identification from biological material using mass spectrometric epitope mapping. Anal Bioanal Chem 378: 1102-1111.).

To be more specific, HuMAb-immobilized columns were prepared by adding HuMAb onto HiTrap NHS-activated HP columns. Then, 2 µg/ml of recombinant HA (derived from A/California/07/2009) (manufactured by Protein Sciences Corp.) was applied to the columns. After washing with PBS, 0.1 mg/ml of modified trypsin (manufactured by Promega) was applied and incubated at 37°C for 2 hours. After washing with PBS followed by ultrapure water, peptide fragments bound onto HuMAb were eluted with 0.1% trifluoroacetic acid. The eluate was concentrated, and the peptide fragments were analyzed by MALDI ToF MS.

### <Preparation of Epitope Sequences>

Influenza sequences and sequence information files (influenza.faa and influenza_aa.dat, respectively) were downloaded from Influenza Virus Resource (ftp://ftp.ncbi.nih.gov/genomes/INFLUENZA) on August24, 2013. The complete hemagglutinin (HA) sequences were searched for epitope regions using blastp search with E-value cutoff value 1e⁻³ (see Altschul SF, Madden TL, Schaffer AA, Zhang J, Zhang Z, et al. (1997) Gapped BLAST and PSI-BLAST: a new generation of protein database search programs. Nucleic Acids Res 25: 3389-3402). Homologous regions that did not show similarities on C or N terminal for 5 amino acids or more were discarded for the following analysis. Moreover, the sequences of H1N1, H2N2, H3N2, H5N1, H7N9, and H9N2 from Avian, Human, and Swine were selected based on the information written in influenza_aa.dat.

### <H1N1pdm (2009) and Seasonal H1N1 Sequences>

To select H1N1pdm (2009) and seasonal H1N1 sequences from the database, a phylogenetic tree was constructed using the full-length amino acid sequence of H1N1 HA and by utilizing the Maximum Likelihood method based on the JTT matrix-based model (Jones DT, Taylor WR, Thornton JM (1992) The rapid generation of mutation data matrices from protein sequences. ComAppl Biosci 8 : 275-282.) . Note that all positions containing gaps and missing data were eliminated. Moreover, evolutionary analyses were conducted in MEGA5 (see Tamura K, Peterson D, Peterson N, Stecher G, Nei M, et al. (2011) MEGA5: Molecular evolutionary genetics analysis using maximum likelihood, evolutionary distance, and maximum parsimony methods. Mol Biolo Evol 28: 2731-2739.).

The sequence set for the phylogenetic analysis was prepared by replacing redundant sequences which had an identity of 99% or more into one representative sequence using uclust program. From the constructed phylogenetic tree, one branch which contained the largest number of human H1N1 HA sequences sampled in 2009 and thereafter was regarded as H1N1pdm (2009) strain. Moreover, human hosted sequences from the H1N1pdm (2009) strains and their 99% identical sequences were used as H1N1pdm (2009) HA sequences. Similarly, seasonal HA sequence set was selected from a branch which contained the largest number of human H1N1 sequences before 2009.

### <Sequence Population Analysis>

The number of unique sequences was counted for the epitopes of: H1N1pdm collected in 2009, 2010, 2011, 2012, and 2013 from human; H1N1sea, H3N2, H2N2, H5N1, H7N9, and H9N2 collected from human; H9N2 collected from avian; and H3N2 collected from swine.

The results obtained by the above-described methods will be described below.

### (Example 1)

### <Preparation of HuMAbs (Human Monoclonal Antibodies) against Influenza A Viruses>

The peripheral blood mononuclear cells (PBMCs) were prepared from the blood obtained from an influenza patient at 2 , 9 , 16 , and 23 days after the onset of inf luenza symptoms. Note that the patient had been diagnosed for infection with HIN1pdm. Then, the PBMCs were fused with fusion partner cells, SPYMEG, for hybridoma preparation.

Next, from the prepared hybridomas, cells for producing antibody specific to influenza virus were selected by immunofluorescence assay (IFA). Then, hybridomas on wells determined to have such specific antibody were each cloned by limiting dilution.

From the above result, hybridomas producing three anti-influenza A virus HuMAbs in total were successfully established: 1H11 and 5G2 from the blood at 9 days after the onset of symptoms, and 2H5 from the blood at 23 days after the onset of symptoms.

Next, the three HuMAbs were tested for the cross-reactivity with influenza A viruses by using Madin-Darby-canine kidney (MDCK) cells infected with the H1N1pdm 2009 isolates, H1N1pdm 2011 isolates, H1N1sea isolates, H5N1 isolates, H9N2 isolate, H2N2 isolate, or H3N2 isolates. Table 1 shows the obtained result. Note that the murine monoclonal antibody C43 against influenza A virus NP (see Okuno Y, Isegawa Y, Sasao F, Ueda S (1993) A common neutralizing epitope conserved between the hemagglutinins of influenza A virus H1 and H2 strains. J Virol 67: 2552-2558) and PBS were used as a positive control and a negative control, respectively. Moreover, the antibody 5A7 capable of exhibiting a broad reactivity with the hemagglutinin (HA) proteins of influenza B viruses was used as a control.

**[Table 1]**

| **Influenza virus** | | | | **Reactivity (IFA)** | | | | | | **Neutralization activity (VN50 value µg/ml)** | | | |
|---|---|---|---|---|---|---|---|---|---|---|---|---|---|
| | | | | HuMA bs | | | | Mouse MAbs | | HuMAbs | | | Mouse MAb |
| **Type** | **Lineage** | **Subtype** | **Strain** | 1H11 | 2H5 | 5G2 | 5A7 | C179 | C43 | 1H11 | 2H5 | 5G2 | C179 |
| A | **Group 1** | H1N1 pdm | A/Suita/1/2009 | + | + | + | - | + | + | +(1.58) | +(0.76) | +(1.39) | +(5.65) |
| | | | A/Osaka/168/2009 | + | + | + | - | + | + | +(0.78) | +(1.51) | +(2.00) | +(5.68) |
| | | | A/California/07/2009 | + | + | + | - | + | + | +(1.52) | +(2.82) | +(3.02) | +(3.01) |
| | | | A/Suita/117/2011 | + | + | + | - | + | + | +(11.60) | +(6.03) | +(11.61) | +(5.73) |
| | | | A/Suita/104/2011 | + | + | + | - | + | + | +(11.35) | +(6.00) | +(2232) | +(5.98) |
| | | | A/Suita/105/2011 | + | + | + | - | + | + | +(11.38) | +(621) | +(12.05) | +(3.11) |
| | | Seasonal H1N1 | A/Brisbane/59/2007 | + | + | + | - | + | + | +(2.89) | +(5.53) | +(5.69) | +(5.57) |
| | | | A/PR8/1934 | + | + | + | - | + | + | +(2.87) | +(5.83) | +(5.78) | +(3.10) |
| | | H5N1 | A/Duck/Egypt/D1Br12/2007 | + | + | + | - | + | + | +(6.08) | +(6.05) | +(6.03) | nd |
| | | | A/Chicken/Egypt/RIMD12- | + | + | + | - | + | + | +(6.12) | +(11.66) | +(11.27) | nd |
| | | | 3/2008 | | | | | | | | | | |
| | | H9N2 | A/Turkey/Wisconsin/1/1966 | + | + | + | - | + | + | +(11.82) | -(> 100) | +(11.08) | +(5.53) |
| | | H2N2 | A/Izumi/5/1965 | - | - | - | - | + | + | nd | nd | nd | nd |
| | **Group 2** | H3N2 | A/Aichi/2/1968 | - | - | - | - | - | + | nd | nd | nd | nd |
| | | | A/Uruguay/716/2007 | - | - | - | - | - | + | nd | nd | nd | nd |
| | | H7N7 | A/Tufted | - | - | - | - | - | + | nd | nd | nd | nd |
| | | | duck/Shimane/ 124R/ 1980 | | | | | | | | | | |
| B | | | B/Florida/4/2006 | - | - | - | + | - | - | nd | nd | nd | nd |
| | | | B/Malaysia/2506/2004 | - | - | - | + | - | - | nd | nd | nd | nd |

Note that "nd" in the table indicates not determined (the same shall apply to Table 3).

As apparent from the result shown in Table 1, all the three HuMAbs reacted with H1N1pdm, H1N1sea, H5N1, and H9N2. However, the HuMAbs did not react with H2N2, H3N2, H7N7, and influenza B viruses. Moreover, the staining pattern by IFA indicated that the three HuMAbs recognized the HA proteins of influenza viruses.

Hence, to confirm the reactivity of each HuMAb with the H1N1pdm-type HA protein, IFA was performed against 293T cells transfected with a plasmid encoding the A/Suita/1/2009-derived HA protein. As shown in Fig. 1, the result revealed that all the three HuMAbs reacted with the H1N1pdm HA in the 293T cells.

Moreover, the result of determining the isotype of the three HuMAbs revealed that all of these HuMAbs were IgG1 (see Table 2). Further, the result of the sequencing analysis of the VH and VL regions of the three HuMabs revealed that the HuMabs had similar sequences to those of germ-line heavy chains: IGVH1-69, IGDH3-10IGJH, and IGJH6 (see Figs. 2 to 4 and Table 2).

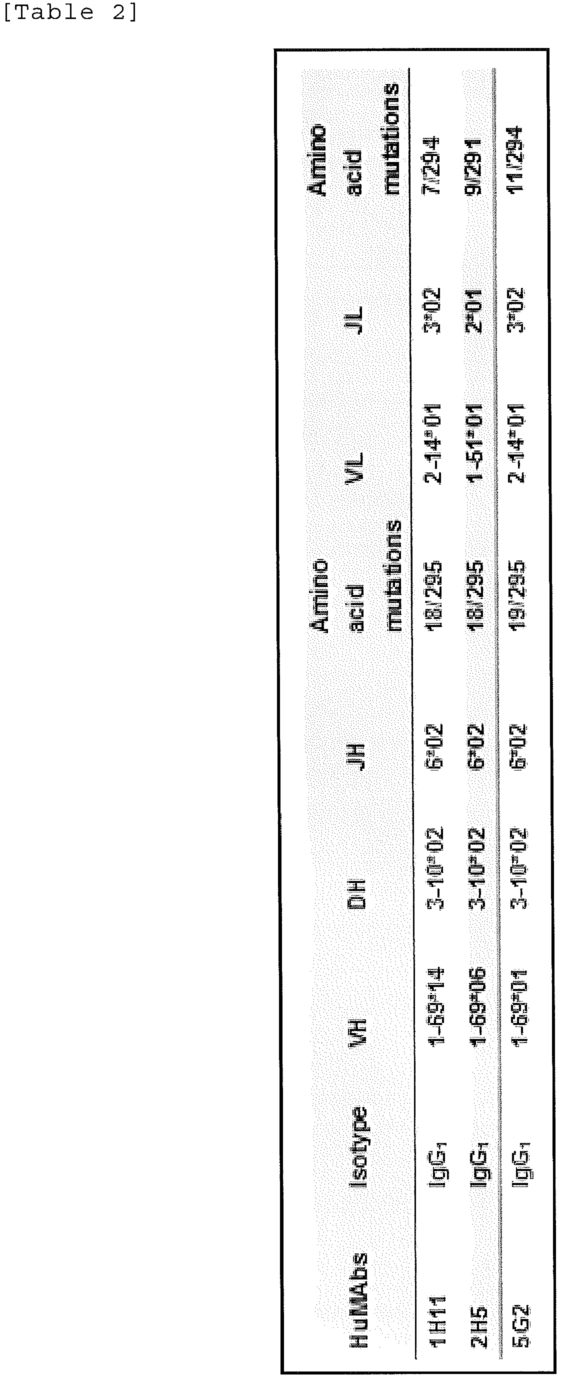

### (Example 2)

### <Neutralization Activities of HuMAbs>

Next, the three HuMAbs were evaluated for the ability to neutralize influenza viruses by virus neutralization (VN) assay in *vitro.* To be more specific, first, MDCK cells were pre treated with the antibody in various concentrations , and infected with H1N1pdm (2009 or 2011 isolates), H1N1sea, or H9N2. Then, the number of the infected cells was counted by immunofluorescent staining to evaluate the neutralization ability of the three HuMAbs. Fig. 5 and Table 1 show the obtained result. Note that, in this VN assay, D23-1G7C2, which was recently prepared as a HuMAb capable of exhibiting a global neutralization activity against dengue virus, was used as a negative control.

As apparent from the result shown in Fig. 5 and Table 1, all the three HuMAbs exhibited a neutralization activity against H1N1pdm, H1N1sea, and H5N1.

Further, the HuMAbs 1H11 and 5G2 were able to neutralize H9N2, too, while the HuMAb 2H5 was not able to neutralizeH9N2. Inaddition, the neutralization activity against H9N2 was exhibited only when the concentrations of the HuMAbs 1H11 and 5G2 were high (Fig. 5).

Moreover, the neutralization profiles with the dilutions of all the three HuMAbs were similar against H1N1pdm. Nevertheless, the neutralization activity, particularly at lower concentrations, of these HuMAbs, was lesser against the 2011 isolates than the neutralization activity against the 2009 isolates (Fig. 5 and Table 1).

Meanwhile, the VN50 estimates of the HuMabs against H1N1 and H5N1 were similar to those against the H1N1pdm 2011 isolates (see Table 1). However, in a range of lower concentrations, any of the three HuMAbs exhibited quite different neutralization profiles among the H1N1pdm 2011 isolates, H1N1sea, and H5N1.

Next, to clarify the mechanism of neutralization by the three HuMAbs, hemagglutinin inhibition (HI) and fusion inhibition assays were performed. Table 3 shows the obtained result. Moreover, Fig. 6 also shows the result of the fusion inhibition assay. Note that, as a control, a mouse MAb C179 capable of exhibiting a broad reactivity with HAs belonging to group 1 (see Okuno Y, Isegawa Y, Sasao F, Ueda S (1993) A common neutralizing epitope conserved between the hemagglutinins of influenza A virus H1 and H2 strains. J Virol 67: 2552-2558) was used. Moreover, the aforementioned D23-1G7C2 was used as a negative control.

**[Table 3]**

| Target | | Clone | Reactivity (WB) | | | | Reactivity (IFA) | | | Inhibition activity for: | | |
|---|---|---|---|---|---|---|---|---|---|---|---|---|
| | | | With β-ME | Without β-ME | Wt | 145F | E47K | E47G | 145F/E47G | Hemagglutination (HI assay) | Cell-to-cell fusion (Fusion inhibition assay) | HA conformational change (Protease susceptibility assay) |
| Anti-influenza | HuMAb | 1H11 | - | + | + | + | + | + | - | - | + | + |
| | | 2H5 | | + | + | + | + | + | | - | + | + |
| | | 5G2 | | + | + | + | + | + | | - | + | + |
| | | 5E4 | + | + | + | + | + | + | | + | nd | - |
| | Mouse MAb | C179 | nd | nd | nd | nd | nd | nd | nd | - | + | nd |
| Anti-dengue | HuMAb | D23-1G7C2 | nd | nd | - | - | - | - | - | - | - | - |

Note that "Wt" in Table 3 indicates wild-type HA protein.

As a result, as shown in Table 3, no HI activity was detected from all the three HuMAbs.

On the other hand, as apparent from the results shown in Fig. 6 and Table 3, the fusion inhibition assay showed that all the antibodies had the ability to inhibit cell-cell fusion. Nevertheless, the antibody concentration necessary for complete inhibition between cells was lower for 1H11, 2H5, and C179 than 5G2. Note that the negative control D23-1G7C2 did not exhibit any fusion inhibition activity even at 200 µg/ml.

### (Example 3)

### <Epitope Mapping of HuMAbs>

As described above, all the three HuMAbs were reactive with the 293T cells expressing the H1N1pdm-derived HA (see Fig. 1). Hence, the epitope regions recognized by these HuMAbs were mapped. First, the reactivity of the HuMAbs with the HA (A/Suita/1/2009) wastestedbywesternblotting. Fig. 7 shows the obtained result. Note that, in this western blotting, the HuMAb 5E4 capable of recognizing head regions of the HA protein (see Yasugi M, Kubota-Koketsu R, Yamashita A, Kawashita N, Du A, et al. (2013) Emerging antigenic variants at the antigenic site Sb in pandemic (H1N1) 2009 influenza virus in Japan detected by a human monoclonal antibody. PLoS One 8: e77892.) was used as a control.

As shown in Fig. 7, when the control 5E4 was used, 75 kDa band corresponding to HA monomer was detected in bothreducing and non-reducingconditions. Meanwhile, all the three HuMAbs reacted with HA only in the non-reducing condition. However, neither 75 kDa nor sub-75 kDa band corresponding to HA1 or HA2 was detected in the reducing condition. This suggested that the reaction of the three HuMAbs with the epitopes was conformation-dependent.

In fact, western blotting was performed using the three HuMAbs and several HA protein fragments of H1N1pdm to examine the reactivity of the three HuMAbs with the HA fragments. As a result, any of the HuMAbs reacted with full-length HA0, but not with any of the prepared fragments (see Fig. 8).

Next, H1N1pdm HA0 protein was treated with trypsin at low pH. Then, the resultant was subjected to western blotting using the three HuMAbs. The result revealed that all the three HuMAbs reacted with trimer forms of HA2 (tHA2) (see A in Fig. 9).

Next, the HA0 protein was first treated with any one of the three HuMAbs or a control 5E4 capable of recognizing HA head regions, then treated with trypsin at low pH similarly as described above, and subjected to western blotting using 5E4. The result revealed that, in the HA0 pretreated with any one of the three HuMAbs, the HA1/HA2 cleavage in the stalk region due to the low pH trypsin treatment was inhibited (see B in Fig. 9).

Thus, it was revealed that, unlike 5E4, the three HuMAbs were antibodies capable of recognizing not the head of HA1 but the stalk region of HA2.

To further identify the epitope region (s) recognized by the three HuMAbs, these HuMAbs and HA protein were subjected to protease susceptibility assay and protease treatment, followed by mass spectrometric epitope mapping. Fig. 10 shows the obtained result.

As a result, the Mascot search showed similar results regarding the three epitopes, except a control. To be more specific, on the basis of all virus protein sequence database, their sequences matched with the sequence of the HA protein of A/California/07/2009. Further, based on the calculated threshold score, the scores greater than 67 were determined to be significant (p<0.05). In addition, the score result revealed that the three HuMAbs had the same epitope of a region having amino acids at residues 40 to 58 in HA2, that is, a polypeptide having amino acids at residues 384 to 402 in A/Suita/1/200/2009 HA0. Note that the HA0 protein was a protein in which 18 amino acids including a signal sequence is added to the N terminal of a protein having an amino acid sequence of SEQ ID NO: 26. Moreover, the amino acid numbers in HA1 and HA2 are as described in (Nobusawa E, Aoyama T, Kato H, Suzuki Y, Tateno Y, et al. (1991) Comparison of complete amino acid sequences and receptor-binding properties among 13 serotypes of hemagglutinins of influenza A viruses. Virology 182: 475-485).

Thus, as shown in Fig. 10, it was revealed that all of the epitopes for the three HuMAbs were located at the α-helix having 40 to 58 amino acids in HA2 and constituting the stalk region of the HA protein.

### (Example 4)

### <Suppressive Effect of HuMAbs against Escape Mutation>

Escape mutations were analyzed which occurred by serial ten passages of H1N1pdm (H1N1pdm-SU1/09) -infected MDCK in the presence of the HuMAb in various concentrations (0.0025 to 2.5 µg/ml). Note that the HuMAb 5E4 against the HA antigenic site Sb of H1N1pdm was used as a control.

As shown in Fig. 11, with both 1H11 and 2H5, no escape mutations appeared even after the serial ten passages (Fig. 11). Particularly, for 1H11, the cytopathic effect by H1N1pdm was disappearred in the sixth passage.

On the other hand, when 5G2 and the control 5E4 were used, the cytopathic effects by H1N1pdm were continuously observed in several wells.

Moreover, RNAs were sampled from the wells in the tenth passages. These RNAs were analyzed by RT-PCR followed by sequencing. The result revealed that when the three HuMAbs were used, most of the tenth passages had the same sequences as the original virus, except for several wells cultured in the presence of 5G2. To be more specific, as shown in Fig. 12, an escape mutation with one amino acid substitution (Y86C) was identified from the influenza virus in the wells of the tenth passage cultured in the presence of 5G2.

Meanwhile, when 5E4 was used as the control, amutation at a similar site to the escape mutation previously reported with this HuMAb (see supra Yasugi M et al. , PLoS One, 2013, 8: e77892.) was detected this time, too.

### (Example 5)

### <Sequence Variation at Epitope Region Recognized by HuMAbs>

Next, the sequences corresponding to the epitope region of the three HuMAbs were compared among several subtypes of influenza A viruses and influenza B virus. The compared full-length sequences are shown in Figs. 13 and 14 separately. Moreover, Table 4 shows the summary of the compared result.

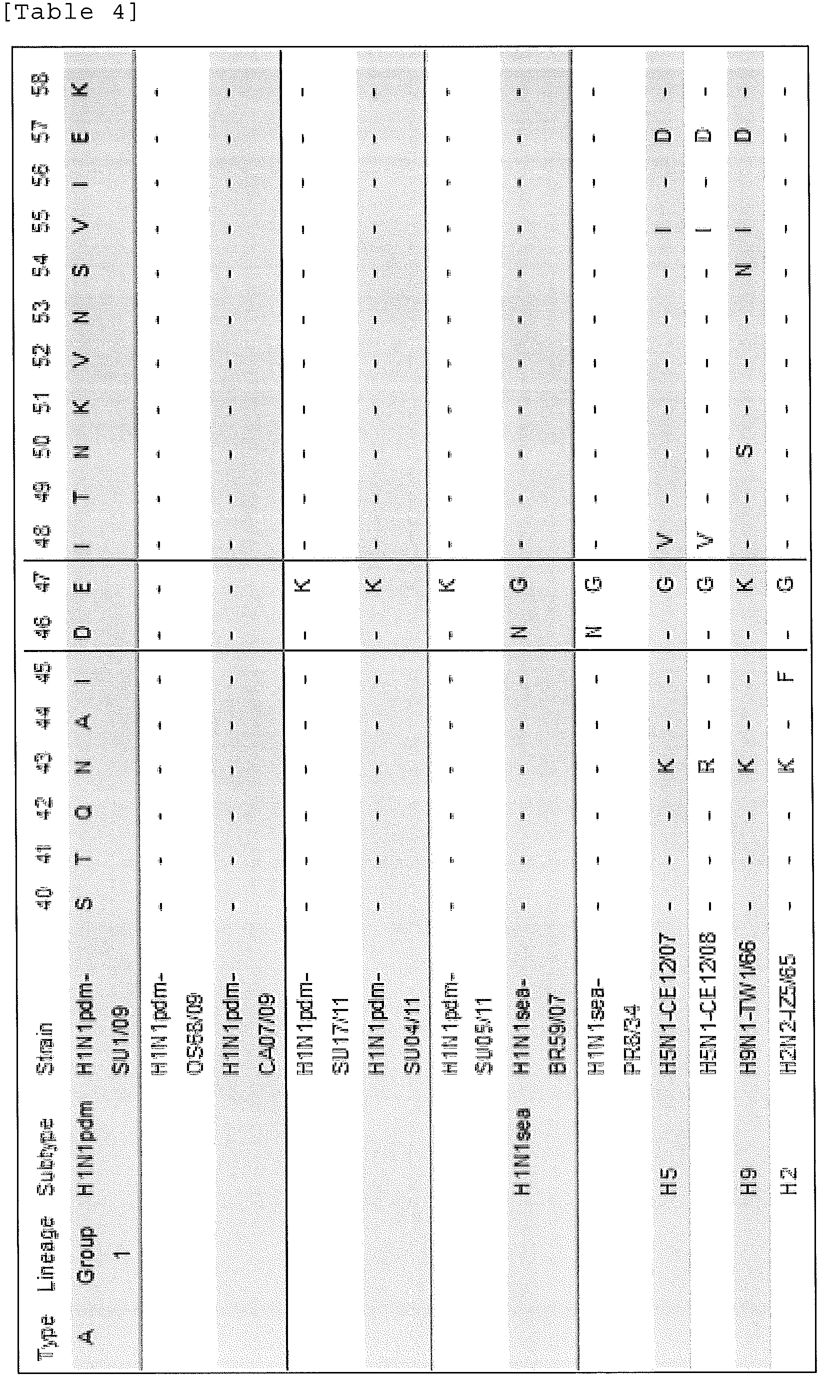

As shown in Table 4, it may be noteworthy that the amino acid at residue 47 of the H1N1pdm 2009 isolates was glutamic acid. On the other hand, that of the H1N1pdm 2011 isolates was lysine. This indicates that the substitution of the amino acid at residue 47 effects on the neutralization activity of HuMAb in consideration of the difference among the HuMAbs in the neutralization activity against against these isolates described above.

On the other hand, the amino acid substitutions of Gly47 together with Asn46 were also commonly detected in H1N1sea based on the H1N1pdm 2009 isolates. Moreover, in H5N1 and H9N2, Gly47 and Lys47 were respectively detected together with amino acid substitutions at several other sites. Further, Glu47 was most commonly detected in H3N2 together with amino acid substitutions at several other sites. In addition, in H2N2, Gly47 and another substitution of Phe45 were identified, indicating that the amino acid at residue 45 is crucial for the reactivity with the three HuMAbs. Note that, as shown in Table 4, the sequence of influenza B virus is quite different at most of the epitope region.

There have been reports of conserved epitopes at the HA stalk region for HuMAbs capable of exhibiting a global neutralization activity against influenza A viruses (NPLs 1 to 4, 6, and 25). Moreover, antibodies capable of recognizing HA head have also been identified as cross-reactive antibody (NPLs 2, 18, 20 to 24, and 26).

Hence, to analyze where or not these existing antibodies and the three HuMAbs had the epitope in common, two HA escape mutants (a mutant from H1N1pdmHA with amino acid substitutions of Q42G, D46T, T49N, and N52D, and a mutant from H1N1pdmHA with amino acid substitutions of D19N, W21F, and I45V) were prepared, which the existing antibodies capable of recognizing the HA stalk region described in NPLs 1 to 4, 6, and 25 were not able to recognize. Then, the reactivity of the three HuMAbs with these mutants was evaluated by western blotting. Fig. 15 shows the obtained result.

As apparent from the result shown in Fig. 15, there were no decreases in the reactivity of the three HuMAbs with the two HA escape mutations of the existing antibodies. Thus, it was revealed that these three HuMAbs recognized the epitope different from those reported in the past.

Meanwhile, the epitope for the global murine antibody (MAb) C179 against HA1 of group 1 has also been identified in the HA stalk region (Dreyfus C, Laursen NS, Kwaks T, Zuijdgeest D, Khayat R, et al. (2012) highly conserved protective epitopes on influenza B viruses. Science 337: 1343-1348). Moreover, existing HuMAbs capable of exhibiting a broad neutralization activity (CR6261, F10, CR9114, and FI6) also similarly recognize this HA stalk region. Additionally, the epitope has the N- and C-terminal regions of HA1 (amino acids at residues 38, 40, 42, 291 to 293, and 318), and the N-terminal portion of HA2 (amino acids at residues 18 to 21, 38, 41 to 43, 45, 46, 52, and 56), including α-helix.

Hence, next, using an escape mutant H1-SU1/89R with G189R, G225D, and T318K, the binding ability thereto was evaluated by IFA. Note that C179, which has been revealed to be incapable of reacting with this mutant strain, was used as a negative control. Moreover, the murine monoclonal antibody C43 against influenza A virus NP was used as a positive control. Table 5 shows the obtained result.

**[Table 5]**

| **Reaction by IFA with a concentration of serial two fold diltuions (µg/ml)** | | | | | | | | | |
|---|---|---|---|---|---|---|---|---|---|
| **MAb** | **Mock-infected MDCK** | **H1-SU 1/89-infected MDCK/H1-SU 1/89R-infected MDCK** | | | | | | | |
| | **12.5** | **12.5** | **6.3** | **3.1** | **1.6** | **0.8** | **0.4** | **0.2** | **0.1** |
| **1H11** | - | +/+ | +/+ | +/+ | +/+ | +/+ | +/+ | +/+ | +/+ |
| **2H5** | - | +/+ | +/+ | +/+ | +/+ | +/+ | +/+ | +/+ | +/+ |
| **5G2** | - | +/+ | +/+ | +/+ | +/+ | +/+ | +/+ | +/+ | +/+ |
| **C179** | - | +/- | +/- | +/- | +/- | +/- | +/- | +/- | +/- |
| **C43** | - | +/+ | +/+ | +/+ | +/+ | +/+ | +/+ | +/+ | +/+ |

As apparent from the result shown in Table 5, all of the three HuMAbs also reacted with the escape mutant (H1-SU1/89R) as in the case with the original wild-type (H1-SU1/89). Thus, it was revealed that all of the three HuMAbs bound to the epitope different also from that for C179 which is a global murine antibody against HA1 of group 1, and whose epitope has been identified in the HA stalk region. Moreover, it was demonstrated that the three HuMAbs were capable of binding also to the H1-SU1/89R strain, which the existing broad neutralizing antibody C179 was not able to suppress, and consequently exhibiting a neutralization activity against the strain.

### (Example 6)

### <Identification of Epitope Using Amino-Acid Substituted Mutants>

Since it was revealed that the amino acid substitutions at residues 45 and 47 were crucial for the reactivity with the three HuMAbs as described above, mutants with amino acid substitutions at the sites were prepared to examine the reactivity with the three HuMAbs. To be more specific, the mutants were prepared by substituting the amino acids in the HA2 region of H1N1pdm: the amino acid at residue 45 was substituted from isoleucine to phenylalanine; the amino acid at residue 47 was substituted from glutamic acid to lysine or glycine; or the amino acid at residue 45 and the amino acid at residue 47 were substituted from isoleucine to phenylalanine and from glutamic acid to glycine, respectively. The binding ability of the three HuMAbs to these mutants was evaluated by IFA. Table 3 shows the obtained result.

As a result, the negative control 5E4 reacted with all the mutants. In contrast, the three HuMAbs reacted with the mutants having the amino acid substitution introduced at residue 45 or 47 individually, but did not react in the case where the mutations were introduced to residues 45 and 47 at the same time.

Thus, it was confirmed that the amino acids at residues 45 and 47 in the HA2 region of the HA protein were crucial for binding between the three HuMAbs and the protein.

### (Example 7)

### <Comparison of Epitope Regions from Influenza Virus Database>

The nucleotide sequences at the epitope region recognized by the three HuMAbs were downloaded as many as possible from the Influenza Virus Resource at the NCBI for: group 1 influenza A viruses such as H1N1pdm, H1N1sea, H5N1, H9N2, and H2N2, group 2 influenza A viruses such as H3N2, H7N1, and H7N7, as well as H7N9.

As a result, the available sequence numbers for these viruses from human infection cases were : n=6174 during 2009, n=1139 during 2010, n=763 during 2011, n=232 during 2012, and n=48 during 2013 for H1N1pdm; n=1936 for H1N1sea; n=223 for H5N1; n=3 for H9N2; n=81 for human H2N2; n=5821 for H3N2; n=1 for H7N2; n=2 for H7N3; n=4 for H7N7; and n=15 for H7N9. Note that Fig. 16 shows sequences that account for ≥% among the various database-derived sequences within each influenza virus subtype, together with the percentages.

As shown in Fig. 16, the result of comparing the amino acid sequences of H1N1pdm showed that the sequence with Lys47 was gradually increased year after year and became a dominant population in 2010 (i.e., 13.7% in 2009, 63.2% in 2010, 86.1% in 2011, 96.5% in 2012, and 100% in 2013, see Table 6). Thus, this Lys47 amino acid mutation could be escape mutation. Nevertheless, it might be noteworthy that the three HuMAbs obtained this time exhibit a neutralization activity and can exert an effective treatment effect also against influenza viruses having this amino acid substitution as described above.

**[Table 6]**

| | | **H1N1pdm** | | **H1N1sea** | **H5N1** | **H9N2** |
|---|---|---|---|---|---|---|
| 2009 | Number | n=6512 | | n=627 | n=232 | n=706 |
| | Amino acid | E (85.53%) | K (13.67%) | G (94.90%) | G (100%) | K (99.58) |
| 2010 | Number | n=1295 | | n=290 | n=216 | n=121 |
| | Amino acid | E (33.44%) | K (63.17%) | G(98.28%) | G (100%) | K (100%) |
| 2011 | Number | n=846 | | n=376 | n=275 | n=168 |
| | Amino acid | E(10.44%) | K(86.05) | G (99.73.%) | G (100%) | K (100%) |
| 2012 | Number | n=227 | | n=387 | n=46 | n=36 |
| | Amino acid | E (3.31%) | K (96.48%) | G (99.22%) | G (100%) | K(100%) |
| 2013 | Number | n=18 | | n=111 | - | n=1 |
| | Amino acid | | K(100%) | G (100%) | - | K(100%) |

### [Industrial Applicability]

As has been described above, the present invention makes it possible to provide an antibody having a neutralization activity against subtype H1 and subtype H5 of group 1 influenza A viruses. To be more specific, the present invention makes it possible to provide an antibody capable of exhibiting a strong neutralization activity against not only seasonal influenza H1N1sea but also H1N1pdm having induced pandemic and H5N1 expected to induce pandemic in the future. Further, the present invention makes it possible to provide an antibody capable of exhibiting a neutralization activity also against a recent predominant H1N1pdm population of mutants having lysine as the amino acid at residue 47 of the HA2 protein.

Thus, the antibody of the present invention is useful in the treatment and prevention of infectious diseases related to group 1 influenza A viruses.

## Claims

1. An antibody having a neutralization activity against subtype H1 and subtype H5 of group 1 influenza A viruses and having the following features (a) and (b):
(a) capable of binding to an epitope present at residues 40 to 58 in an HA2 region of a hemagglutinin protein derived from an A/Suita/1/200/2009 strain; and
(b) not binding to the hemagglutinin protein derived from the A/Suita/1/200/2009 strain in the HA2 region of which an amino acid at residue 45 is substituted with phenylalanine and an amino acid at residue 47 is substituted with glycine.

2. The antibody according to claim 1, which further has the following features (c) to (e):
(c) capable of binding to the hemagglutinin protein derived from the A/Suita/1/200/2009 strain in the HA2 region of which an amino acid at residue 42 is substituted with glycine, an amino acid at residue 46 is substituted with threonine, an amino acid at residue 49 is substituted with asparagine, and an amino acid at residue 52 is substituted with aspartic acid;
(d) capable of binding to the hemagglutinin protein derived from the A/Suita/1/200/2009 strain in the HA2 region of which an amino acid at residue 19 is substituted with asparagine, an amino acid at residue 21 is substituted with phenylalanine, and the amino acid at residue 45 is substituted with valine; and
(e) capable of binding to the hemagglutinin protein derived from the A/Suita/1/200/2009 strain in an HA1 region of which an amino acid at residue 189 is substituted with arginine, an amino acid at residue 225 is substituted with aspartic acid, and an amino acid at residue 318 is substituted with lysine.

3. The antibody according to claim 1 or 2, which has any one of the following features (i) to (iii):
(i) comprising
a light chain variable region including amino acid sequences of SEQ ID NOs: 3 to 5 or the amino acid sequences in at least any one of which one or more amino acids are substituted, deleted, added, and/or inserted, and
a heavy chain variable region including amino acid sequences of SEQ ID NOs: 8 to 10 or the amino acid sequences in at least any one of which one or more amino acids are substituted, deleted, added, and/or inserted;
(ii) comprising
a light chain variable region including amino acid sequences of SEQ ID NOs: 13 to 15 or the amino acid sequences in at least any one of which one or more amino acids are substituted, deleted, added, and/or inserted, and
a heavy chain variable region including amino acid sequences of SEQ ID NOs: 18 to 20 or the amino acid sequences in at least any one of which one or more amino acids are substituted, deleted, added, and/or inserted; and
(iii) comprising
a light chain variable region including amino acid sequences of SEQ ID NOs: 23 to 25 or the amino acid sequences in at least any one of which one or more amino acids are substituted, deleted, added, and/or inserted, and
a heavy chain variable region including amino acid sequences of SEQ ID NOs: 28 to 30 or the amino acid sequences in at least any one of which one or more amino acids are substituted, deleted, added, and/or inserted.

4. The antibody according to claim 1 or 2, which has any one of the following features (i) to (iii):
(i) comprising
a light chain variable region including the amino acid sequence of SEQ ID NO : 2 or the amino acid sequence in which one or more amino acids are substituted, deleted, added, and/or inserted, and
a heavy chain variable region including an amino acid sequence of SEQ ID NO: 7 or the amino acid sequence in which one or more amino acids are substituted, deleted, added, and/or inserted;
(ii) comprising
a light chain variable region including the amino acid sequence of SEQ ID NO: 12 or the amino acid sequence in which one or more amino acids are substituted, deleted, added, and/or inserted, and
a heavy chain variable region including the amino acid sequence of SEQ ID NO: 17 or the amino acid sequence in which one or more amino acids are substituted, deleted, added, and/or inserted; and
(iii) comprising
a light chain variable region including the amino acid sequence of SEQ ID NO: 22 or the amino acid sequence in which one or more amino acids are substituted, deleted, added, and/or inserted, and
a heavy chain variable region including the amino acid sequence of SEQ ID NO: 27 or the amino acid sequence in which one or more amino acids are substituted, deleted, added, and/or inserted.

5. The antibody according to any one of claims 1 to 4, which is an antibody further having a neutralization activity against subtype H9 of group 1 influenza A viruses.

6. The antibody according to any one of claims 1 to 5, wherein a concentration of the antibody required to neutralize the group 1 influenza A viruses to 50% is 30 µg/ml or less.

7. The antibody according to any one of claims 1 to 6, which is a human antibody.

8. A DNA encoding the antibody according to any one of claims 1 to 7.

9. A cell which produces the antibody according to any one of claims 1 to 7, or comprises the DNA according to claim 8.

10. A method for producing an antibody, comprising the steps of:
culturing the cell according to claim 9; and
collecting the antibody according to any one of claims 1 to 7 produced in the cell or from a culture fluid thus obtained.

11. Apharmaceutical composition comprising the antibody according to any one of claims 1 to 7 as an active ingredient.
